(19) Europäisches Patentamt
European Patent Office
Office européen des brevets

(11) **EP 2 479 160 B1**

(12) **EUROPEAN PATENT SPECIFICATION**

(45) Date of publication and mention of the grant of the patent:
**09.09.2015 Bulletin 2015/37**

(21) Application number: **10817184.4**

(22) Date of filing: **15.09.2010**

(51) Int Cl.:
*C07C 37/62* (2006.01) *C07C 39/27* (2006.01)
*C07C 41/22* (2006.01) *C07C 43/225* (2006.01)
*C07C 45/36* (2006.01) *C07C 47/565* (2006.01)
*C07C 47/575* (2006.01) *C07C 209/80* (2006.01)
*C07C 211/52* (2006.01) *C07C 211/56* (2006.01)
*C07D 215/06* (2006.01) *C07D 219/02* (2006.01)
*C07D 333/02* (2006.01) *C07B 61/00* (2006.01)

(86) International application number:
**PCT/JP2010/065898**

(87) International publication number:
**WO 2011/034071 (24.03.2011 Gazette 2011/12)**

(54) **PROCESS FOR PRODUCTION OF OXIDATION REACTION PRODUCT OF AROMATIC COMPOUND**

VERFAHREN ZUR HERSTELLUNG EINES OXIDATIONSREAKTIONSPRODUKTS AUS EINER AROMATISCHEN VERBINDUNG

PROCÉDÉ POUR LA PRODUCTION D’UN PRODUIT DE RÉACTION D’OXYDATION D’UN COMPOSÉ AROMATIQUE

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **15.09.2009 JP 2009213811**

(43) Date of publication of application:
**25.07.2012 Bulletin 2012/30**

(73) Proprietor: **Osaka University**
**Suita-shi**
**Osaka 565-0871 (JP)**

(72) Inventors:
- **OHKUBO Kei**
  **Osaka 565-0871 (JP)**
- **FUKUZUMI Shunichi**
  **Osaka 565-0871 (JP)**
- **MIZUSHIMA Kentaro**
  **Osaka 565-0871 (JP)**

(74) Representative: **Duckworth, Timothy John et al**
**J A Kemp**
**14 South Square**
**Gray’s Inn**
**London WC1R 5JJ (GB)**

(56) References cited:
**WO-A1-2009/041519 JP-A- 2008 222 471**
**US-A1- 2010 004 454**

- **MOLINARI ET AL: "Photocatalytic and catalytic activity of heterogenized W10O32<4-> in the bromide-assisted bromination of arenes and alkenes in the presence of oxygen", JOURNAL OF MOLECULAR CATALYSIS A: CHEMICAL, ELSEVIER, AMSTERDAM, NL, vol. 262, no. 1-2, 14 January 2007 (2007-01-14), pages 156-163, XP005830744, ISSN: 1381-1169, DOI: 10.1016/J.MOLCATA.2006.08.056**
- **OHKUBO, K. ET AL.: "Rational design and functions of electron donor-acceptor dyads with much longer charge-separated lifetimes than natural photosynthetic reaction centers", BULLETIN OF THE CHEMICAL SOCIETY OF JAPAN, vol. 82, no. 3, March 2009 (2009-03), pages 303-315, XP002692289,**
- **OHKUBO, K. ET AL.: ’Rational design and functions of electron donor-acceptor dyads with much longer charge-separated lifetimes than natural photosynthetic reaction centers’ BULLETIN OF THE CHEMICAL SOCIETY OF JAPAN vol. 82, no. 3, 2009, pages 303 - 315, XP002692289**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 479 160 B1

• 'The 42nd The Symposium on Chemical and Biological Oxidation Koen Yoshishu', 14 November 2009 deel KENTARO MIZUSHIMA ET AL.: 'Acridinium Ion Yudotai o Denshi Idogata Hikari Shokubai to suru Bunshijo Sanso ni yoru Hokozoku Tankasuiso no Koritsuteki Kosansoka Oyobi Koshusoka Hanno', pages 114 - 116, XP008160469

## Description

Technical Field

The present invention relates to a process for producing an oxidation reaction product of an aromatic compound.

Background Art

Aromatic compounds show a variety of reactivities. Thus, they are used widely as various materials, raw materials for synthesizing reaction intermediates, etc. Among reactions of aromatic compounds, for example, reactions of oxidatively substituting hydrogen that is on an aromatic ring or at the benzylic position with another atom or atomic group are of great variety, and are highly versatile and useful.

For example, bromobenzenes (aromatic bromides) are used widely as raw materials for preparing Grignard reagents and the like, and are used as precursors in preparation of many drugs (pharmaceuticals). These bromobenzenes can be generated in the presence of an iron catalyst through an aromatic electrophilic substitution reaction caused by reacting bromine with benzenes, for example (Non-Patent Document 1 etc.). Also, bromination of aromatic hydrocarbons using N-bromosuccinimide is well known. In any of these reactions, anhydrous carbon tetrachloride, halogenated hydrocarbon, or the like is used as a solvent, for example.

Citation List

Non-Patent Document(s)

[Non-Patent Document 1] "Kagaku Daijiten (chemical encyclopedia)", vol. 4, 656 (Kyoritsu Shuppan Co., Ltd., edited by Kagaku Daijiten Henshu Iinkai (editorial committee of chemical encyclopedia), the first impression of the first edition was published on December 30, 1960; the 39th impression of the compact edition was published on September 15, 2006)

Molinari et al., Journal of Molecular Catalysis A, 2007, vol. 262, pp. 156-163 discloses photocatalytic and catalytic activity of heterogenized $W_{10}O_{32}^{4-}$ in the bromide assisted bromination of arenes and alkenes in the presence of oxygen.

Ohkubo, K et al., Bulletin of the Chemical Society of Japan, 2009, vol. 82, pp. 303-315 discloses rational design and functions of electron donor-acceptor dyads with much longer charge-separated lifetimes than natural photosynthetic reaction centres.

Brief Summary of the Invention

In organic synthesis reactions, it is important to carry out reactions and aftertreatments while suppressing the environmental load due to the toxicity of reactants, a solvent, etc., and besides, reducing the cost as much as possible. This is particularly important when organic synthesis reactions are used for industrial (manufacturing-industrial) purposes. From such a viewpoint, organic synthesis reactions are required to be improved constantly. This applies to the above-described oxidation reaction of aromatic compounds.

With the foregoing in mind, it is an object of the present invention to provide a process for producing an oxidation reaction product of an aromatic compound, having excellent environmental load reduction performance, cost reduction performance, etc.

In order to achieve the above object, the present invention provides a process for producing an oxidation reaction product of a raw material aromatic compound comprising a benzene, naphthalene or thiophene aromatic ring having at least one substituent selected from the group consisting of $-OR^{100}$, $-NR^{200}{}_2$, and $-AR^{100}$ which is covalently bound to the benzene, naphthalene or thiophene ring,

$R^{100}$ is a hydrogen atom or an arbitrary substituent, and when a plurality of $R^{100}$s are present, they may be identical to or different from each other,

$R^{200}$s are each a hydrogen atom or an arbitrary substituent, and they may be identical to or different from each other, and $AR^{100}$ is an aryl group, and when a plurality of $AR^{100}$s are present, they may be identical to or different from each other, which process comprises reacting the raw material aromatic compound with an oxidizing agent which is oxygen as an elementary substance, wherein

the process further uses an electron donor-acceptor linked molecule, and a Brønsted acid selected from hydrogen halides, sulfuric acid, nitric acid, sulfonic acid, carboxylic acids and phosphoric acid and anions thereof,

and the process comprises the steps of

generating an electron-transfer state of the electron donor-acceptor linked molecule by photoexcitation;

reacting the electron donor-acceptor linked molecule in the electron-transfer state, the oxidizing agent, the raw material

aromatic compound, and the Brønsted acid or anion thereof thereby generating an oxidation reaction product, wherein; the oxidation reaction product comprises a substitution product obtained by substitution of at least one hydrogen atom on an aromatic ring of the raw material aromatic compound with an atom or atomic group of the Brønsted acid anion, and the electron donor-acceptor linked molecule is at least one selected from the group consisting of: nitrogen-containing aromatic cation derivatives represented by the following formulae (A-1) to (A-8); 9-substituted acridinium ions represented by the following formula (A-9); quinolinium ion derivatives represented by the following formula (I); stereoisomers and tautomers thereof and salts thereof

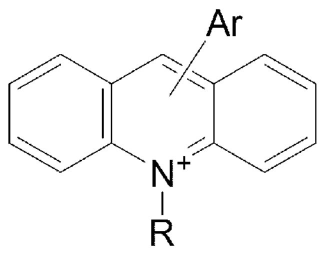

(A－1)

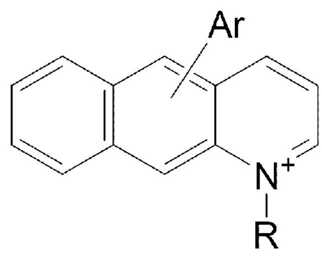

(A－2)

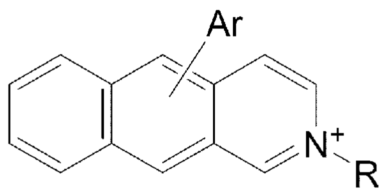

(A－3)

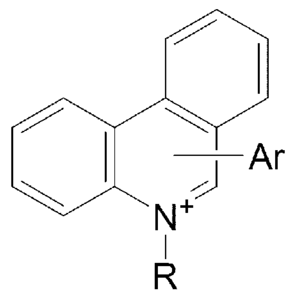

(A－4)

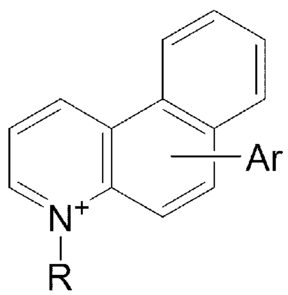

(A－5)

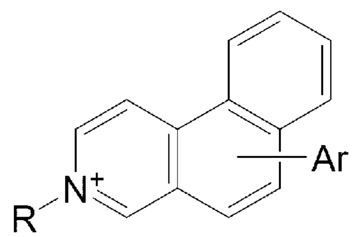

(A－6)

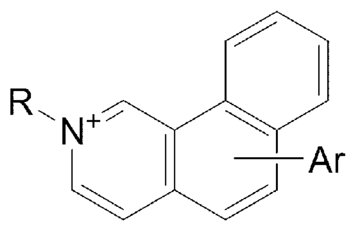

(A－7)

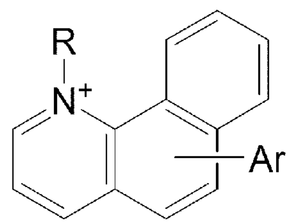

(A－8)

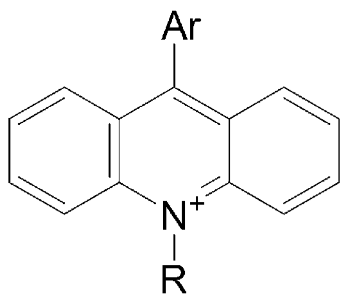

(A－9)

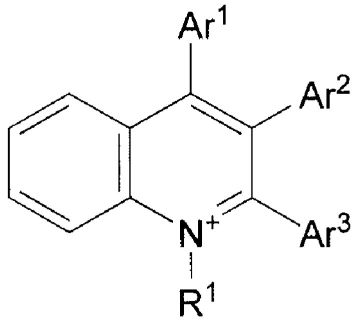

(I)

where in the formulae (A-1) to (A-8) and (A-9),
R is a hydrogen atom or an arbitrary substituent,
Ar is the electron donor group, and the number of Ars may be one or more, and when a plurality of Ars are present, they may be identical to or different from each other,
a nitrogen-containing aromatic ring that forms a nitrogen-containing aromatic cation may or may not contain at least one arbitrary substituent other than R and Ar, and
in the formula (I),
$R^1$ is a hydrogen atom or an arbitrary substituent,
$Ar^1$ to $Ar^3$ are each a hydrogen atom or the electron donor group and may be identical to or different from each other, and at least one of $Ar^1$ to $Ar^3$ is the electron donor group.

The present invention also provides a process for producing hydrogen peroxide, the process comprising the step of
producing an oxidation reaction product of a raw material aromatic compound by the process of the invention,
wherein the oxidizing agent comprises oxygen ($O_2$),
a supply source of a hydrogen atom further is used, and
in the step of generating the oxidation reaction product, the oxygen binds to the hydrogen atom, thereby generating hydrogen peroxide.

According to the present invention, it is possible to provide a process for producing an oxidation reaction product of an aromatic compound, having excellent environmental load reduction performance, cost reduction performance, etc.

Brief Description of Drawings

[FIG. 1] FIG. 1 is a schematic view showing the production scheme of an inorganic-organic composite material of Reference Example 1.
[FIG. 2] FIG. 2 is a graph showing the amount of 9-mesityl-10-methylacridinium ions inserted to AlMCM-41 zeolite in Reference Example 1.
[FIG. 3] FIG. 3 is a spectral diagram showing an XRD pattern of AlMCM-41 produced for use in Reference Example 1.

[FIG. 4] FIG. 4 is a spectral diagram showing XRD patterns of AlMCM-41 (literature data).

[FIG. 5] FIG. 5 is a graph showing an ultraviolet-visible absorption spectrum of an acetonitrile solution of 9-mesityl-10-methylacridinium perchlorate (9-mesityl-10-methylacridinium ion, $Acr^+$-Mes), together with a diffuse reflectance spectrum of a suspension of the inorganic-organic composite material of Reference Example 1 ($Acr^+$-Mes@AlMCM-41).

[FIG. 6] FIG. 6 shows an ESR spectrum of the inorganic-organic composite material of Reference Example 1 ($Acr^+$-Mes@AlMCM-41) after light irradiation under high vacuum at 25°C (298 K) using a high-pressure mercury lamp (wavelength: $\lambda$ > 390 nm).

[FIG. 7] FIG. 7 shows an ESR spectrum of the inorganic-organic composite material of Reference Example 1 ($Acr^+$-Mes@AlMCM-41) after light irradiation under high vacuum at 100°C (373 K) using a high-pressure mercury lamp (wavelength: $\lambda$ > 390 nm).

[FIG. 8] FIG. 8 shows decay of an ESR signal shown in FIG. 7.

[FIG. 9] FIG. 9 shows an ESR signal intensity when light irradiation using a high-pressure mercury lamp (wavelength: $\lambda$ > 390nm) under the same conditions as in FIG. 7 and interruption of the light irradiation were repeated.

[FIG. 10] FIG. 10 shows an ultraviolet-visible absorption spectrum (diffuse reflectance spectrum) of the inorganic-organic composite material of Reference Example 1 ($Acr^+$-Mes@AlMCM-41) itself as a solid before and after the light irradiation under high vacuum at 25°C (298 K) using a high-pressure mercury lamp (wavelength: $\lambda$ > 390nm).

[FIG. 11] FIG. 11 shows a decay curve of a diffuse reflectance spectrum when the inorganic-organic composite material of Reference Example 1 ($Acr^+$-Mes@AlMCM-41) was irradiated with visible light at 100°C (373 K), together with a decay curve of an ESR signal.

[FIG. 12] FIG. 12 is a schematic view showing the production scheme of an inorganic-organic composite material of Reference Example 2 ($Acr^+$-Mes@AlSBA-15).

[FIG. 13] FIG. 13 is a spectrum showing an XRD pattern of AlSBA-15 produced for use in Reference Example 2.

[FIG. 14] FIG. 14 is a graph showing an ultraviolet-visible absorption spectrum of an acetonitrile solution of 9-mesityl-10-methylacridinium perchlorate (9-mesityl-10-methylacridinium ion, $Acr^+$-Mes) before a reaction in the production of the inorganic-organic composite material of Reference Example 2 ($Acr^+$-Mes@AlSBA-15), together with an ultraviolet-visible absorption spectrum of a supernatant obtained by centrifuging a suspension after the reaction.

[FIG. 15] FIG. 15 shows a diffuse reflectance spectrum of the suspension of the inorganic-organic composite material ($Acr^+$-Mes@AlSBA-15) in FIG. 14 after the reaction.

[FIG. 16] FIG. 16 shows a diffuse reflectance transient absorption spectrum of the inorganic-organic composite material of Reference Example 2 ($Acr^+$-Mes@AlSBA-15).

[FIG. 17] FIG. 17 shows the results of measuring ultraviolet-visible absorption spectra of a solution of electron donor-acceptor linked molecules of Reference Example 3-1 before and after the addition of zeolite.

[FIG. 18] FIG. 18 is a graph showing the number of the electron donor-acceptor linked molecules with respect to ten supercages of zeolite in Reference Example 3-1.

[FIG. 19] FIG. 19 is a graph showing ultraviolet-visible absorption spectrum of the inorganic-organic composite material of Reference Example 3-1 being suspended in acetonitrile, measured by diffuse reflectance spectroscopy.

[FIG. 20] FIG. 20 is a graph showing an ultraviolet-visible absorption spectrum of the inorganic-organic composite material of Reference Example 3-1 itself as a solid, measured by diffuse reflectance spectroscopy.

## Mode for Carrying out the Invention

Embodiments of the present invention will be described below. It is to be noted, however, that the present invention is by no means limited by the following description. Furthermore, in the present invention, when the scope of the invention is limited by numerical values, the present invention encompasses not only the case of the exact range defined by the numerical values but also the case of an approximate range defined the numerical values. For example, when the scope of the invention is limited by a temperature range from "0°C to 45°C", the scope may be exactly from 0°C to 45°C, or may be approximately from 0°C to 45°C.

As described above, the production process according to the present invention is a process for producing an oxidation reaction product of a raw material aromatic compound by reacting the raw material aromatic compound with an oxidizing agent. The process further uses an electron donor-acceptor linked molecule and a Brønsted acid or anion thereof. The process includes the step of: reacting the electron donor-acceptor linked molecule in an electron-transfer state, the oxidizing agent, the raw material aromatic compound and the Brønsted acid or anion thereof, thereby generating an oxidation reaction product resulting from oxidation of the raw material aromatic compound.

The production process according to the present invention is applicable to a broad range of reactions. For example, as shown in Scheme 1 below, by using a 9-mesityl-10-methylacridinium ion ($Acr^+$-Mes; J. Am. Chem. Soc. 2004, 126, 1600), which exhibits a strong oxidizing power when it is in the electron-transfer state, as a photocatalyst, it is possible to convert aromatic hydrocarbon to a monobrominated product highly selectively with visible light irradiation.

There have been no previous cases where a bromination reaction proceeds in a photocatalytic manner as described above. In this reaction, hydrogen bromide can be used as a supply source of bromine. Also, it is possible to cause similar reactions for halogens other than bromine. It should be noted, however, that the reactivities of these halogens may be different from that of bromine.

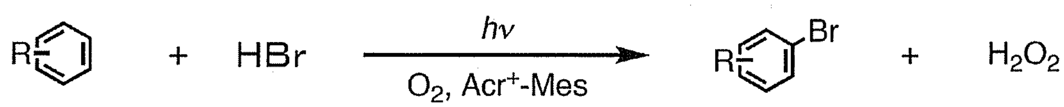


Scheme 1

The mechanism of the above reaction is not necessarily certain, but can be speculated to be as follows, for example. Specifically, a 9-mesityl-10-methylacridinium ion that is in the electron-transfer state generated through photoexcitation exhibits a strong oxidizing power, so that it causes one-electron oxidation of various aromatic hydrocarbons. By reacting a radical cationic species of the aromatic hydrocarbon obtained through this one-electron oxidation with a bromo anion, a monobrominated aromatic hydrocarbon can be obtained with highly selectivity. More specifically, this is as shown in Scheme 2 below. First, by intramolecular electron transfer in photoexcited $Acr^+$-Mes, an electron-transfer state ($Acr^\cdot$-$Mes^{\cdot+}$) is generated. The mesitylene radical cation site of this $Acr^\cdot$-$Mes^{\cdot+}$ in the electron-transfer state has a high oxidizing power. With this mesitylene radical cation site, one-electron oxidation of a substrate, i.e., a raw material aromatic compound (e.g., 1,2,4-trimethoxybenzene) is caused, whereby a radical cationic species (e.g., 1,2,4-trimethoxybenzene radical cation) of the substrate is generated. To this radical cation, a halide ion (bromide ion) is added. On the other hand, at the acridinyl radical site of $Acr^\cdot$-$Mes^{\cdot+}$, oxygen is reduced, thereby generating a superoxide. Furthermore, it is considered that the superoxide is protonated by water present in a reaction system, and $HO_2\cdot$ thus generated abstracts hydrogen from an adduct radical of the halide (bromide or the like), whereby an aromatic halide (e.g., monobrominated product) and hydrogen peroxide are generated as end products. It should be noted, however, that this reaction mechanism is an example of a presumable mechanism, and does not limit the present invention by any means. In the chemical formula, an unpaired electron in a radical is indicated with "·" (dot).

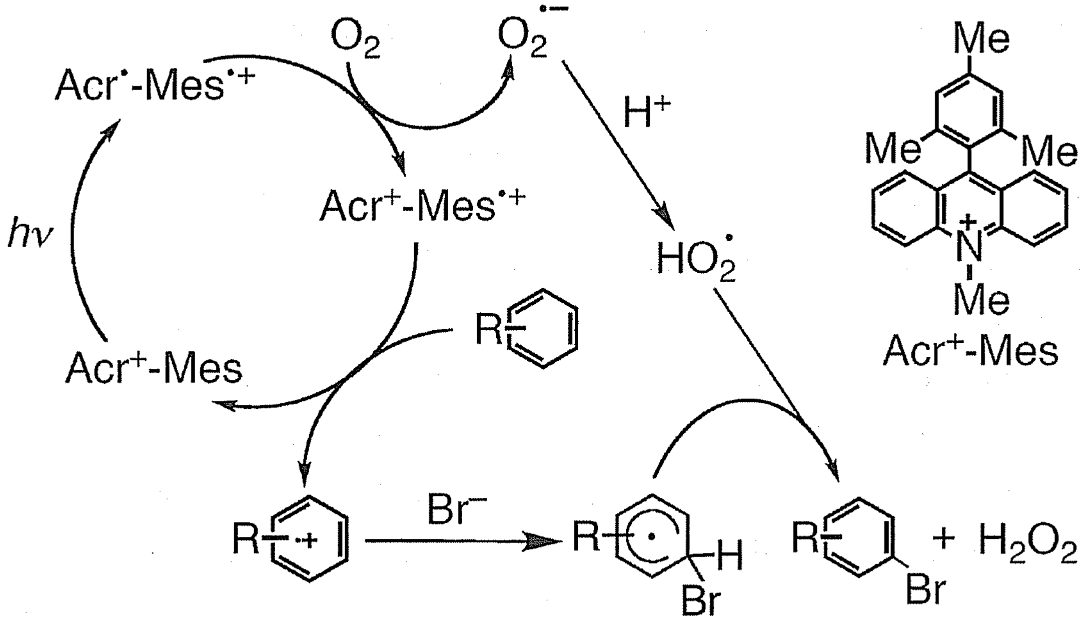


Scheme 2

In the above reaction, the 9-mesityl-10-methylacridinium ion ($Acr^+$-Mes) corresponds to an "electron donor-acceptor linked molecule" in the present invention. The oxygen molecule corresponds to an "oxidizing agent". On the other hand, seen from another point of view, the hydrogen bromide (bromide ion) corresponds to an "oxidizing agent", because it oxidatively substitutes a hydrogen atom on an aromatic ring. The aromatic bromide as the resultant product corresponds to an "oxidation reaction product" of the raw material aromatic compound. In the above reaction, although it is not necessary to use an oxygen molecule, the use of an oxygen molecule allows the reaction to proceed more

smoothly. Furthermore, in the above reaction, for example, a methylene group ($-CH_2-$) is bound covalently to the aromatic ring of the raw material aromatic compound. Thus, by using an oxygen molecule as an oxidizing agent, it is also possible to oxidize the methylene group ($-CH_2-$) to a carbonyl group (-CO-). In this case, if bromination (bromine substitution reaction) of the aromatic ring is not necessary, it is not necessary to use hydrogen bromide (bromide ion).

In the present invention, the electron donor-acceptor linked molecule is not limited only to 9-mesityl-10-methylacridinium ion ($Acr^+$-Mes). If a common electron donor-acceptor linked molecule is used instead of $Acr^+$-Mes in Scheme 2, the reaction would be as shown in Scheme 2' below. In Scheme 2', A-D represents an electron donor-acceptor linked molecule. D represents an electron donor site, and A represents an electron acceptor site. In Scheme 2', $X^-$ represents an anion of a Brønsted acid.

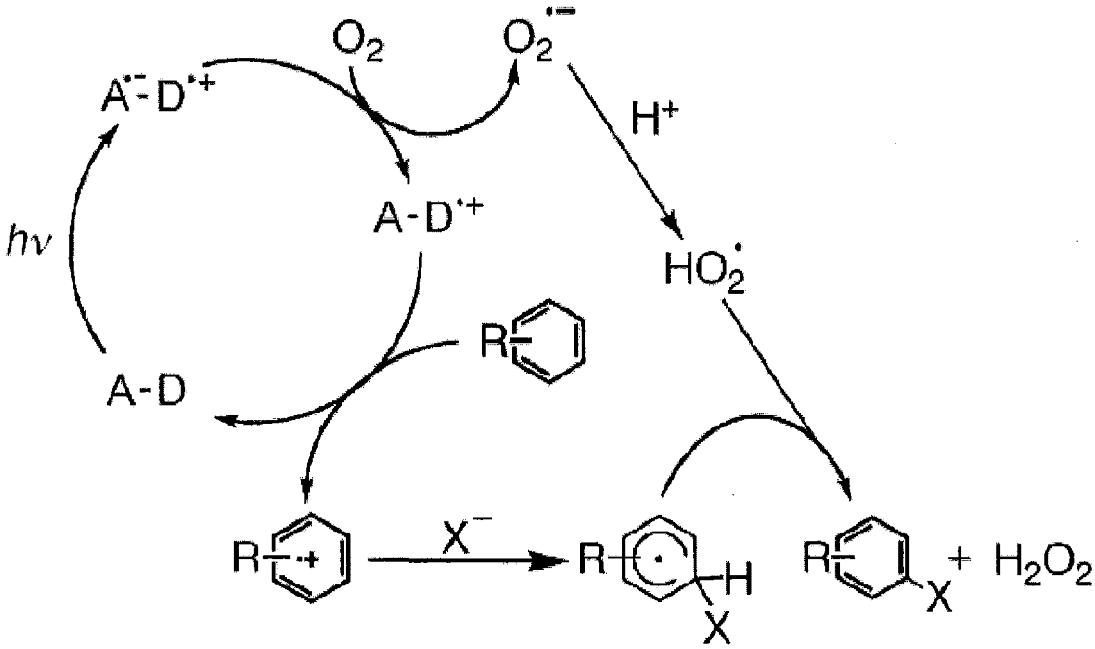


Scheme 2'

In the present invention, a substituent and the like in organic compounds such as the raw material aromatic compound and the electron donor-acceptor linked molecule are as follows, for example. An alkyl group is not particularly limited, and examples thereof include a methyl group, an ethyl group, an n-propyl group, an isopropyl group, an n-butyl group, an isobutyl group, a sec-butyl group, a tert-butyl group, an n-pentyl group, a neopentyl group, and an n-propyl group. The same applies to groups containing an alkyl group in its structure (an alkylamino group, an alkoxy group, etc.). Furthermore, a perfluoroalkyl group is not particularly limited, and examples thereof include perfluoroalkyl groups derived from a methyl group, an ethyl group, an n-propyl group, an isopropyl group, an n-butyl group, an isobutyl group, a sec-butyl group, a tert-butyl group, an n-pentyl group, a neopentyl group, and an n-propyl group. The same applies to groups containing a perfluoroalkyl group in their structures (a perfluoroalkylsulfonyl group, a perfluoroacyl group, etc.). In the present invention, an acyl group is not particularly limited, and examples thereof include a formyl group, an acetyl group, a propionyl group, an isobutyryl group, a valeryl group, an isovaleryl group, a pivaloyl group, a hexanoyl group, a cyclohexanoyl group, a benzoyl group, and an ethoxycarbonyl group. The same applies to groups containing an acyl group in their structures (an acyloxy group, an alkanoyloxy group, etc.). In the present invention, the number of carbon atoms in the acyl group includes a carbon atom of a carbonyl group. For example, an alkanoyl group (an acyl group) having one carbon atom indicates a formyl group. An aryl group not only includes groups derived from a benzene ring, but also includes a group derived from any aromatic rings such as a benzene ring, a naphthalene ring, an anthracene ring, a phenanthrene ring, a pyridine ring, a thiophene ring, and a pyrene ring. In the present invention, "halogen" refers to an arbitrary halogen element, and examples thereof include fluorine, chlorine, bromine, and iodine. When a substituent or the like has isomers, any isomer can be used. For example, in the case of a "naphthyl group", it may be a 1-naphthyl group or a 2-naphthyl group.

The present invention will be described more specifically below.

[<1> Electron donor-acceptor linked molecule]

In the present invention, the electron donor-acceptor linked molecule is selected from the formula (A-1) to (A-9) and the formula (I) as described above.

The electron donor group in the electron donor-acceptor linked molecule preferably is at least one selected from the group consisting of alkyl groups and aromatic rings. In this case, the aromatic ring further may have one or more substituents on the ring, and when a plurality of substituents are present, they may be identical to or different from each other. When a plurality of electron donor groups are present, they may be identical to or different from each other. Furthermore, in the electron donor group in this case, it is more preferable that the alkyl group is a straight-chain or branched alkyl group having 1 to 6 carbon atoms. Furthermore, in the electron donor group, it is more preferable that the aromatic ring is at least one selected from the group consisting of a benzene ring, a naphthalene ring, an anthracene ring, a phenanthrene ring, a pyridine ring, a thiophene ring, and a pyrene ring. In the electron donor group, it is more preferable that the substituent on the aromatic ring is at least one selected from the group consisting of alkyl groups, alkoxy groups, primary to tertiary amines, carboxylic acids, and carboxylate esters. In Ar, it is more preferable that the substituent on the aromatic ring is at least one selected from the group consisting of straight-chain or branched alkyl groups having 1 to 6 carbon atoms, straight-chain or branched alkoxy groups having 1 to 6 carbon atoms, primary to tertiary amines, carboxylic acids, and carboxylate esters. In the substituent on the aromatic ring, a "carboxylic acid" refers to a carboxyl group or a group having a carboxyl group added to its end (e.g., a carboxyalkyl group), and a "carboxylate ester" refers to a carboxylate ester group such as an alkoxycarbonyl group or a phenoxycarbonyl group, or an acyloxy group. An alkyl group in the carboxyalkyl group preferably is a straight-chain or branched alkyl group having 1 to 6 carbon atoms, for example. An alkoxy group in the alkoxycarbonyl group preferably is a straight-chain or branched alkoxy group having 1 to 6 carbon atoms, for example.

It is still more preferable that the electron donor group is at least one selected from the group consisting of a phenyl group, an o-tolyl group, an m-tolyl group, a p-tolyl group, a 2,3-dimethylphenyl group, a 2,4-dimethylphenyl group, a 2,5-dimethylphenyl group, a 2,6-dimethylphenyl group, a 3,4-dimethylphenyl group, a 3,5-dimethylphenyl group, a 2,3,4-trimethylphenyl group, a 2,3,5-trimethylphenyl group, a 2,3,6-trimethylphenyl group, a mesityl group (2,4,6-trimethylphenyl group), and a 3,4,5-trimethylphenyl group. Among these, a mesityl group is particularly preferable from the viewpoint of the lifetime of the electron-transfer state (charge-separated state) and the like. Although the reasons why a mesityl group can bring about a particularly excellent effect is not clear, they are speculated to be as follows, for example: two methyl groups are present in the ortho position, so that the benzene rings of the mesityl groups easily cross at right angles to the aromatic ring of the aromatic cation; and hyperconjugation does not occur very often inside the mesityl group. This, however, merely is an example of a presumable mechanism, and does not limit the present invention by any means.

The electron donor-acceptor linked molecule is at least one selected from the group consisting of: nitrogen-containing aromatic cation derivatives represented by the following formulae (A-1) to (A-9); quinolinium ion derivatives represented by the following formula (I), stereoisomers and tautomers thereof; and salts thereof, from the viewpoints of the lifetime, oxidizing power, reducing power, and the like of the electron-transfer state (charge-separated state).

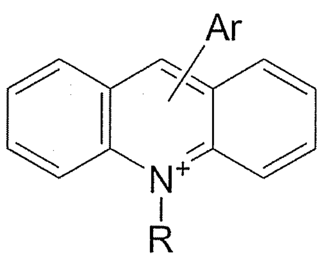

(A－1)

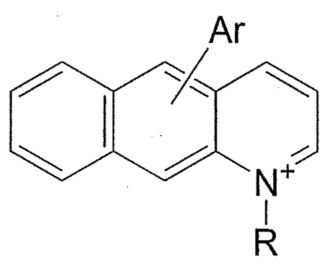

(A－2)

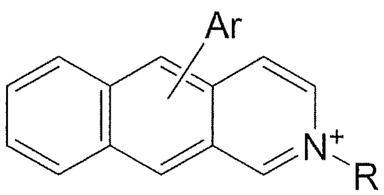

(A－3)

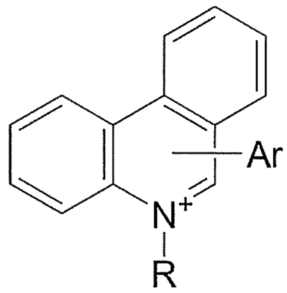

(A－4)

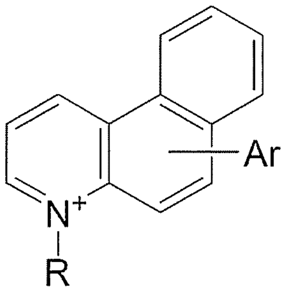

(A－5)

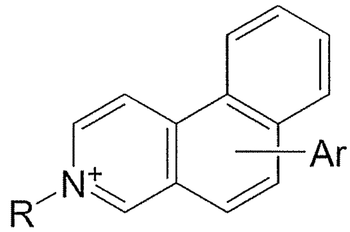

(A－6)

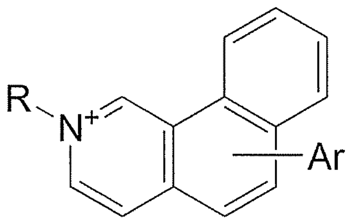

(A－7)

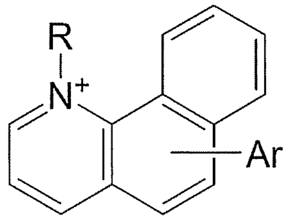

(A－8)

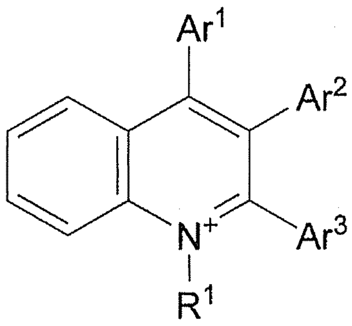

(I)

In the formulae (A-1) to (A-8),
R is a hydrogen atom or an arbitrary substituent,
Ar is the electron donor group, and the number of Ars may be one or more, and when a plurality of Ars are present, they may be identical to or different from each other, and

the nitrogen-containing aromatic ring that forms a nitrogen-containing aromatic cation may or may not contain at least one arbitrary substituent other than R and Ar.

In the formula (I),

$R^1$ is a hydrogen atom or an arbitrary substituent,

$Ar^1$ to $Ar^3$ are each a hydrogen atom or the electron donor group and may be identical to or different from each other, and at least one of $Ar^1$ to $Ar^3$ is the electron donor group.

In the formulae (A-1) to (A-8), R preferably is a hydrogen atom, an alkyl group, a benzyl group, a carboxyalkyl group (an alkyl group with a carboxyl group added to its end), an aminoalkyl group (an alkyl group having an amino group added to its end), or a polyether chain. More preferably, R is a hydrogen atom, a straight-chain or branched alkyl group having 1 to 6 carbon atoms, a benzyl group, a straight-chain or branched alkyl group having 1 to 6 carbon atoms with a carboxyl group added to its end, a straight-chain or branched alkyl group having 1 to 6 carbon atoms with an amino group added to its end, or a polyethylene glycol (PEG) chain. The PEG chain is an example of the polyether chain. The kind of the polyether chain is not limited thereto, and the polyether chain may be of any kind. In R, the degree of polymerization of the polyether chain is not particularly limited, and is, for example, 1 to 100, preferably 1 to 50, and more preferably 1 to 10. In the case where the polyether chain is a PEG chain, the degree of polymerization is not particularly limited, and is, for example, 1 to 100, preferably 1 to 50, and more preferably 1 to 10.

More preferably, the electron donor-acceptor linked molecule is at least one selected from the group consisting of 9-substituted acridinium ions represented by the following formula (A-9), tautomers thereof, and stereoisomers thereof.

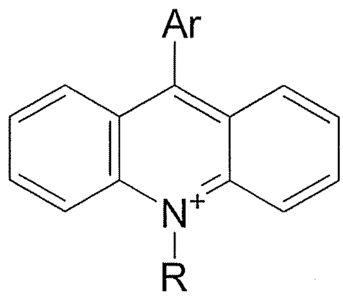

(A-9)

In the formula (A-9), R and Ar are the same as those in the formula (A-1).

Furthermore, it is particularly preferable that the electron donor-acceptor linked molecule is a 9-mesityl-10-methylacridinium ion represented by the following formula (A-10). By photoexcitation of this 9-mesityl-10-methylacridinium ion, it is possible to generate a long-lived electron-transfer state (charge-separated state) having a high oxidizing power and a high reducing power. As excitation light for the photoexcitation, it is possible to use visible light, for example.

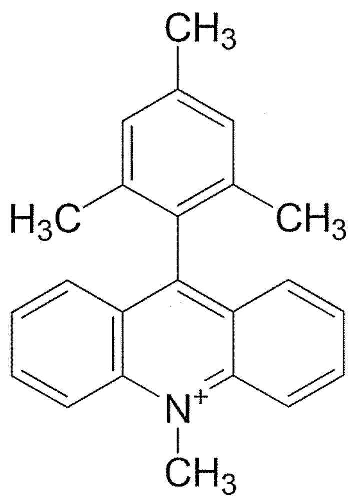

(A-10)

Examples of the 9-substituted acridinium ion represented by the formula (A-9) further include compounds (A-101) to (A-116) shown in the following table, in addition to the one represented by the above formula (A-10).

[Table 1]

| Substituent / Compound No. | R | Ar |
|---|---|---|
| (A-101) | methyl group | phenyl group |
| (A-102) | methyl group | o-tolyl group |
| (A-103) | methyl group | m-tolyl group |
| (A-104) | methyl group | p-tolyl group |
| (A-105) | methyl group | 2,3-dimethylphenyl group |
| (A-106) | methyl group | 2,4-dimethylphenyl group |
| (A-107) | methyl group | 2,5-dimethylphenyl group |
| (A-108) | methyl group | 2,6-dimethylphenyl group |
| (A-109) | methyl group | 3,4-dimethylphenyl group |
| (A-110) | methyl group | 3,5-dimethylphenyl group |
| (A-111) | methyl group | 2,3,4-trimethylphenyl group |
| (A-112) | methyl group | 2,3,5-trimethylphenyl group |
| (A-113) | methyl group | 2,3,6-trimethylphenyl group |
| (A-114) | methyl group | mesityl group (2,4,6-trimethylphenyl group) |
| (A-115) | methyl group | 3,4,5-trimethylphenyl group |
| (A-116) | methyl group | hydrogen atom |

In the quinolinium ion derivative represented by the formula (I), $R^1$ preferably is a hydrogen atom, an alkyl group, a benzyl group, a carboxyalkyl group (an alkyl group with a carboxyl group added to its end), an aminoalkyl group (an alkyl group having an amino group added to its end), or a polyether chain, for example. More preferably, $R^1$ is a hydrogen atom, a straight-chain or branched alkyl group having 1 to 6 carbon atoms, a benzyl group, a straight-chain or branched alkyl group having 1 to 6 carbon atoms with a carboxyl group added to its end, a straight-chain or branched alkyl group having 1 to 6 carbon atoms with an amino group added to its end, or a polyethylene glycol (PEG) chain, for example. The PEG chain is an example of the polyether chain. The kind of the polyether chain is not limited thereto, and the polyether chain may be of any kind. In $R^1$, the degree of polymerization of the polyether chain is not particularly limited, and is, for example, 1 to 100, preferably 1 to 50, and more preferably 1 to 10. In the case where the polyether chain is a PEG chain, the degree of polymerization is not particularly limited, and is, for example, 1 to 100, preferably 1 to 50, and more preferably 1 to 10. Furthermore, $Ar^1$ to $Ar^3$ preferably are each a hydrogen atom, an alkyl group, or an aromatic ring, for example, and the alkyl group more preferably is a straight-chain or branched alkyl group having 1 to 6 carbon atoms. In $Ar^1$ to $Ar^3$, the aromatic ring further may have one or more substituents on the ring, and when a plurality of substituents are present, they may be identical to or different from each other.

In $Ar^1$ to $Ar^3$ in the formula (I), the aromatic ring more preferably is a benzene ring, a naphthalene ring, an anthracene ring, a phenanthrene ring, a pyridine ring, a thiophene ring, or a pyrene ring, for example. Furthermore, in

$Ar^1$ to $Ar^3$, the substituent on the aromatic ring more preferably is an alkyl group, an alkoxy group, any one of primary to tertiary amines, a carboxylic acid, or a carboxylate ester. Still more preferably, the substituent on the aromatic ring is a straight-chain or branched alkyl group having 1 to 6 carbon atoms, a straight-chain or branched alkoxy group having 1 to 6 carbon atoms, any one of primary to tertiary amines, a carboxylic acid, or a carboxylate ester. The secondary amine is not particularly limited, and preferably is an alkylamino group, and more preferably is a straight-chain or branched alkylamino group having 1 to 6 carbon atoms, for example. The tertiary amine is not particularly limited, and preferably is a dialkylamino group, and more preferably is a dialkylamino group with a straight-chain or branched alkyl group having 1 to 6 carbon atoms, for example.

In the substituent on the aromatic ring in $Ar^1$ to $Ar^3$, a "carboxylic acid" refers to a carboxyl group or a group having a carboxyl group added to its end (e.g., a carboxyalkyl group), and a "carboxylate ester" refers to a carboxylate ester group such as an alkoxycarbonyl group or a phenoxycarbonyl group, or an acyloxy group. An alkyl group in the carboxyalkyl group preferably is a straight-chain or branched alkyl group having 1 to 6 carbon atoms, for example. An alkoxy group in the alkoxycarbonyl group preferably is a straight-chain or branched alkoxy group having 1 to 6 carbon atoms, for example.

Among quinolinium ion derivatives represented by the formula (I), for example, quinolinium ion derivatives represented by any one of the following formulae 1 to 5 are particularly preferable in terms of a long lifetime, a high oxidizing power, a high reducing power, and the like of the charge-separated state.

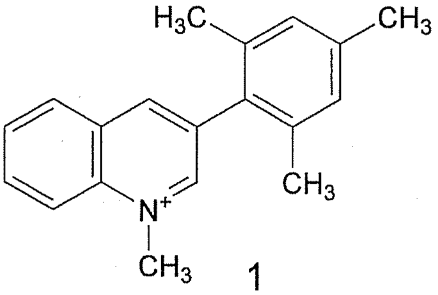
1

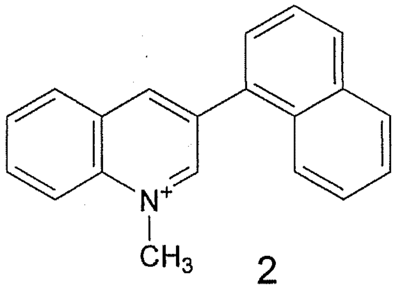
2

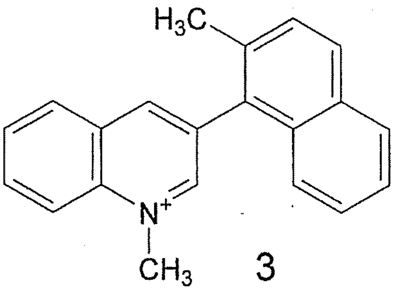
3

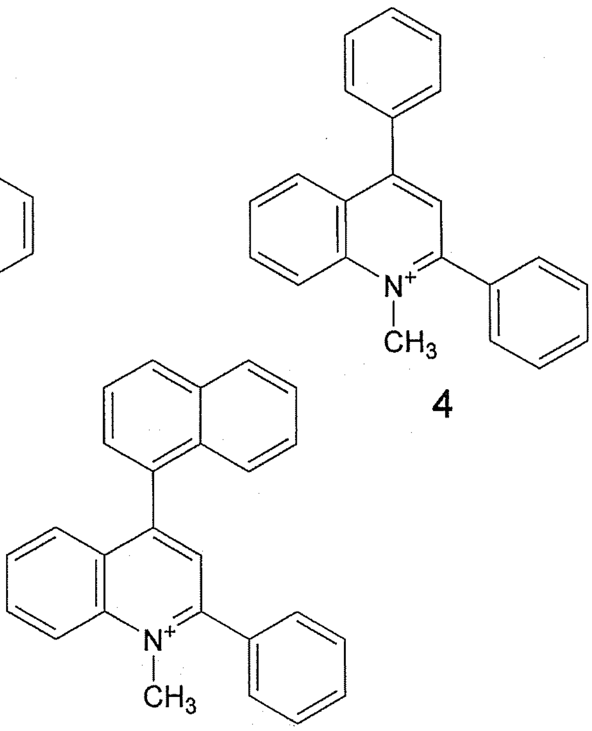
4

5

Besides the above compounds 1 to 5, compounds 6 to 36 shown in Tables 1 and 2 below are particularly preferable, for example. Tables 2 and 3 show the structures of the compounds 6 to 36 by indicating the combination of $R^1$ and $Ar^1$ to $Ar^3$ in the formula (I). Those skilled in the art can produce and use the compounds 6 to 36 easily according

to the production and use of the compounds 1 to 5 with reference to examples to be described below, without undue trial and error, complicated and advanced experiments, etc.

[Table 2]

| Substituent / Compound No. | $R^1$ | $Ar^1$ | $Ar^2$ | $Ar^3$ |
|---|---|---|---|---|
| 6 | methyl group | hydrogen atom | phenyl group | hydrogen atom |
| 7 | methyl group | hydrogen atom | tolyl group | hydrogen atom |
| 8 | methyl group | hydrogen atom | xylyl group | hydrogen atom |
| 9 | methyl group | hydrogen atom | durenyl group | hydrogen atom |
| 10 | methyl group | hydrogen atom | phenyl group | hydrogen atom |
| 11 | methyl group | hydrogen atom | aminophenyl group | hydrogen atom |
| 12 | methyl group | hydrogen atom | methoxynaphthyl group | hydrogen atom |
| 13 | methyl group | hydrogen atom | anthryl group | hydrogen atom |
| 14 | methyl group | hydrogen atom | pyrenyl group | hydrogen atom |
| 15 | ethoxycarbonyl group | hydrogen atom | phenyl group | hydrogen atom |

| | | | | |
|---|---|---|---|---|
| 16 | ethoxycarbonyl group | hydrogen atom | tolyl group | hydrogen atom |
| 17 | ethoxycarbonyl group | hydrogen atom | xylyl group | hydrogen atom |
| 18 | ethoxycarbonyl group | hydrogen atom | durenyl group | hydrogen atom |
| 19 | ethoxycarbonyl group | hydrogen atom | phenyl group | hydrogen atom |
| 20 | ethoxycarbonyl group | hydrogen atom | methoxynaphthyl group | hydrogen atom |
| 21 | ethoxycarbonyl group | hydrogen atom | anthryl group | hydrogen atom |
| 22 | ethoxycarbonyl group | hydrogen atom | pyrenyl group | hydrogen atom |

[Table 3]

| Substituent / Compound No. | $R^1$ | $Ar^1$ | $Ar^2$ | $Ar^3$ |
|---|---|---|---|---|
| 23 | ethoxycarbonyl group | hydrogen atom | mesityl group | hydrogen atom |
| 24 | ethoxycarbonyl group | hydrogen atom | naphthyl group | hydrogen atom |
| 25 | ethoxycarbonyl group | hydrogen atom | methylnaphthyl group | hydrogen atom |
| 26 | methyl group | aminophenyl group | hydrogen atom | phenyl group |
| 27 | methyl group | tolyl group | hydrogen atom | phenyl group |
| 28 | methyl group | xylyl group | hydrogen atom | phenyl group |
| 29 | methyl group | durenyl group | hydrogen atom | phenyl group |
| 30 | methyl group | phenyl group | hydrogen atom | phenyl group |
| 31 | methyl group | methoxy -naphthyl group | hydrogen atom | phenyl group |
| 32 | methyl group | anthryl group | hydrogen atom | phenyl group |
| 33 | methyl group | pyrenyl group | hydrogen atom | phenyl |

| | | | | |
|---|---|---|---|---|
| | | | | group |
| 34 | methyl group | mesityl group | hydrogen atom | phenyl group |
| 35 | methyl group | (N,N-dimethyl -amino) phenyl group | hydrogen atom | phenyl group |
| 36 | methyl group | phenyl group | phenyl group | phenyl group |

In the present invention, when the electron donor-acceptor linked molecule has isomers such as tautomers and stereoisomers (e.g., a geometric isomer, a conformer, and an optical isomer), any isomer can be used in the present invention. Furthermore, the salt of the electron donor-acceptor linked molecule may be an acid addition salt. However, when the electron donor-acceptor linked molecule can form a base addition salt, it may be a base addition salt. Moreover, an acid that forms the acid addition salt may be either an inorganic acid or an organic acid, and a base that forms the base addition salt may be either an inorganic base or an organic base. The inorganic acid is not particularly limited, and examples thereof include sulfuric acid, phosphoric acid, hydrofluoric acid, hydrochloric acid, hydrobromic acid, hydroiodic acid, hypofluorous acid, hypochlorous acid, hypobromous acid, hypoiodous acid, fluorous acid, chlorous acid, bromous acid, iodous acid, fluorine acid, chloric acid, bromic acid, iodic acid, perfluoric acid, perchloric acid, perbromic acid, and periodic acid. The organic acid also is not particularly limited, and examples thereof include p-toluenesulfonic acid, methanesulfonic acid, oxalic acid, p-bromobenzenesulfonic acid, carbonic acid, succinic acid, citric acid, benzoic acid, and acetic acid. The inorganic base is not particularly limited, and examples thereof include ammonium hydroxides, alkali metal hydroxides, alkaline-earth metal hydroxides, carbonates, and hydrogencarbonates. More specifically, the inorganic base may be, for example, sodium hydroxide, potassium hydroxide, potassium carbonate, sodium carbonate, sodium hydrogencarbonate, potassium hydrogencarbonate, calcium hydroxide, and calcium carbonate. The organic base also is not particularly limited, and examples thereof include ethanolamine, triethylamine, and tris(hydroxymethyl)aminomethane. A process for producing these salts also is not particularly limited. For example, they can be produced by adding an acid or a base such as described above to the electron donor-acceptor linked molecule as appropriate by a known method.

Furthermore, in the present invention, the absorption band of the electron donor-acceptor linked molecule is not particularly limited. Preferably, the electron donor-acceptor linked molecule has an absorption band in the visible region. This is because, if the electron donor-acceptor linked molecule has an absorption band in the visible region, it can be excited easily with visible light. This allows sunlight to be used as an energy source.

[<1-2> Process for producing electron donor-acceptor linked molecule]

In the present invention, a commercially available product may be used as the electron donor-acceptor linked molecule, or the electron donor-acceptor linked molecule may be produced (synthesized) as appropriate. When producing the electron donor-acceptor linked molecule, a process for producing it is not particularly limited, and it can be produced as appropriate by a known production process or with reference to a known production process, for example. As an illustrative example, the electron donor-acceptor linked molecule represented by the above formula (A-9) can be produced through a Grignard reaction of acridone or a derivative thereof (A-11) as described below, for example. In the formulae (A-11) and (A-12) below, R and Ar are the same as those in the formula (A-9). Reaction conditions (a reaction temperature, a reaction time, a solvent, etc.) also are not particularly limited, and may be set as appropriate with reference to a known Grignard reaction and the like.

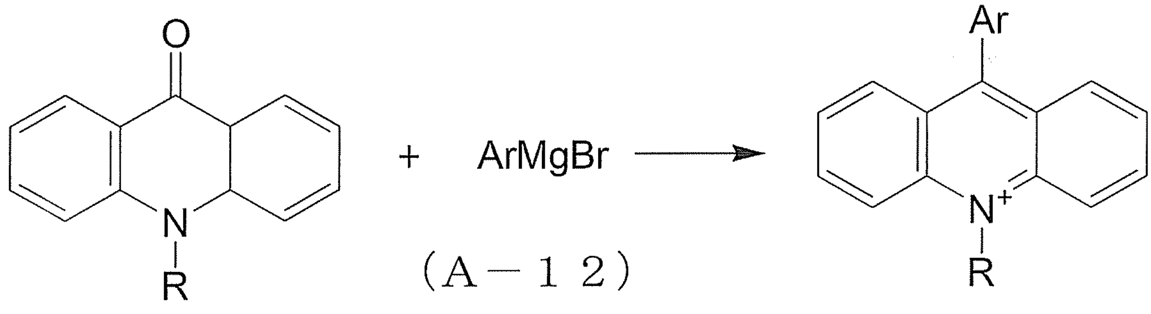

(A-11) (A-12) (A-9)

For example, the 9-mesityl-10-methylacridinium ion represented by the above formula (A-10) may be a commercially available product. Alternatively, it can be produced in the following manner. Specifically, first, the air in a sufficiently dried reaction vessel is substituted with argon, thereby providing an argon atmosphere at an ordinary temperature and pressure. In this atmosphere, a mixture of 2.0 g (10 mmol) of mesitylene bromide (commercially available reagent), 250 mg (10 mmol) of magnesium (commercially available reagent), and 5 ml of dehydrated tetrahydrofuran is stirred, thus preparing a Grignard reagent. 50 ml of dehydrated dichloromethane solution of 1.0 g (4.8 mmol) of 10-methylacridone (commercially available reagent) was added thereto, and the resultant mixture was stirred for 12 hours. The product was hydrolyzed with water, and perchloric acid aqueous solution was added thereto. The mixture was then subjected to extraction with dichloromethane. The extract obtained is recrystallized using methanol/dimethyl ether. Thus, 9-mesityl-10-methylacridinium perchlorate is obtained. This production process is described in Patent Document 1 (JP 2005-145853 A).

The quinolinium ion derivative (I) can be produced by a production process to be described below, for example.

Specifically, the quinolinium ion derivative (compound (I)) can be produced by a production process including the step of reacting a quinoline derivative represented by the following formula (II) and a compound represented by the following formula (III), for example.

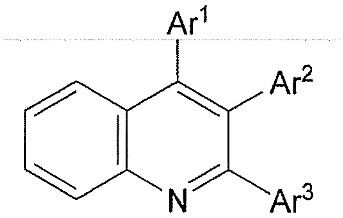

(II)

$R^1-Q$

(III)

In the formula (II), $Ar^1$ to $Ar^3$ are the same as those in the formula (I). In the formula (III), $R^1$ is the same as that in the formula (I), and Q is an electron-withdrawing group.

In the formula (III), Q is not particularly limited as long as it is an electron-withdrawing group. Examples of Q include halogens, a perfluoroalkyl group, a perfluoroalkylsulfonyl group, and a perfluoroacyl group. Among them, fluorine, chlorine, bromine, iodine, a trifluoromethyl group, a trifluoromethylsulfonyl group, a trifluoromethylcarbonyl group, and the like are more preferable.

Conditions for the reaction between the quinoline derivative represented by the formula (II) and the compound represented by the formula (III) are not particularly limited, and can be set as appropriate with reference to conditions for known similar reactions, for example. The amount ratio (molar ratio) between the quinoline derivative (II) and the compound (III) is not particularly limited, and is, for example, 1 : 1 to 1 : 10, preferably 1 : 1 to 1 : 4, and particularly preferably 1 : 1. Furthermore, for example, a reactant other than the quinoline derivative (II) and the compound (III) and a solvent may or may not be used appropriately as necessary. The solvent is not particularly limited, and may be, for example, water or an organic solvent. Examples of the organic solvent include: halogenated solvents such as methylene chloride, chloroform, and carbon tetrachloride; ketones such as acetone; and nitrile solvents such as acetonitrile. These solvents may be used alone, or two or more of them may be used in combination. In the case where a solvent is used, the concentration of the quinoline derivative (II) is not particularly limited, and is, for example, 0.01 to 0.2 mol/l, preferably 0.02 to 0.1 mol/l, and more preferably 0.03 to 0.05 mol/l. The reaction temperature is not particularly limited, and is, for

example. 0°C to 80°C, preferably 10°C to 40°C, and more preferably 20°C to 30°C. The reaction time also is not particularly limited, and is, for example, 10 to 40 hours, preferably 20 to 30 hours, and more preferably 25 to 30 hours.

Furthermore, after the production of the quinolinium ion, an anion-exchange treatment may be performed, as necessary. The method of the anion-exchange treatment is not particularly limited, and any method can be used as appropriate. Examples of substances that can be used in the anion-exchange treatment include: perhalogen acids such as perfluoric acid, perchloric acid, perbromic acid, and periodic acid; boron tetrafluoride; and phosphorus hexafluoride. They may be used alone, or two or more of them may be used in combination. Furthermore, for example, a reactant other than those described above, a solvent, and the like may or may not be used appropriately as necessary.

The process for producing the quinoline derivative represented by the formula (II) is not particularly limited. For example, as a first production process, it is preferable to produce the quinoline derivative by reacting a halogenated quinoline represented by the following formula (IV) with a boronate ester represented by the following formula (V), for example.

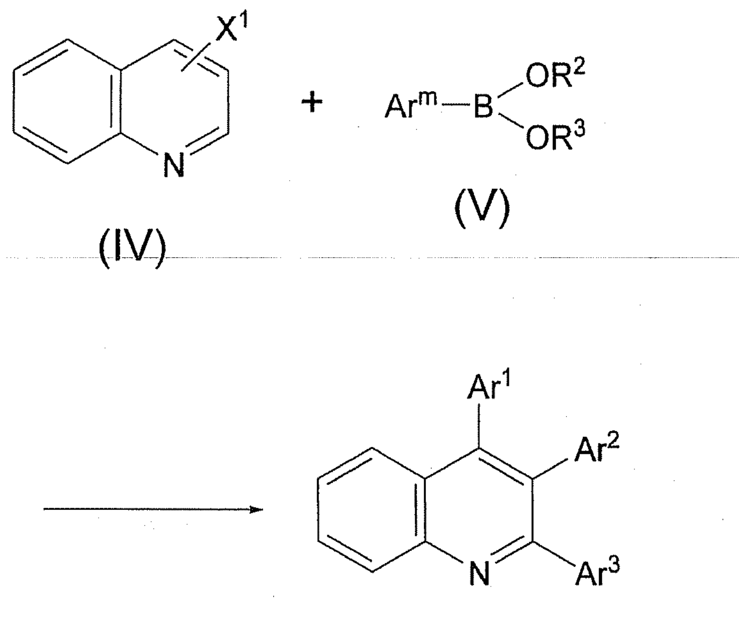

(IV) (V)

(II)

In the formula (IV),
$X^1$ is a halogen group on a pyridine ring, the number of $X^1$s may be one or more, and when a plurality of $X^1$s are present, they may be identical to or different from each other.
In the formula (V),
$R^2$ and $R^3$ are each a hydrogen atom or a hydrocarbon group, or $R^2$ and $R^3$ may have been unified.
m in $Ar^m$ is an integer from 1 to 3.

Only one kind or a plurality of kinds of the boronate esters (V) may be used.

Preferably, $R^2$ and $R^3$ are each a hydrogen atom or an alkyl group, or together form an alkylene group. In the case of an alkyl group, it is more preferable that the alkyl group is a straight-chain or branched alkyl group having 1 to 6 carbon atoms. In the case of an alkylene group, it is more preferable that the alkylene group is a straight-chain or branched alkylene group having 1 to 12 carbon atoms, and it is particularly preferable that the alkylene group is an ethylene group (dimethylene group) or a trimethylene group.

Conditions for the reaction between the halogenated quinoline represented by the formula (IV) and the boronate ester represented by the formula (V) are not particularly limited, and can be set as appropriate with reference to conditions for known similar reactions, for example. The amount ratio (molar ratio) between the halogenated quinoline (IV) and the boronate ester (V) is not particularly limited, and is, for example, 1 : 2 to 1 : 10, preferably 1 : 2 to 1 : 4, and particularly preferably 1 : 2. Furthermore, for example, a reactant other than the halogenated quinoline (IV) and the boronate ester (V) and a solvent may or may not be used appropriately as necessary. In the case where a solvent is used, the concentration of the halogenated quinoline (IV) is not particularly limited, and is, for example, 0.2 to 2.0 mol/l, preferably 0.3 to 1.5 mol/l, and more preferably 0.5 to 1.0 mol/l. As the reactant other than the halogenated quinoline (IV) and the boronate ester (V), a catalyst may be used, for example. Examples of the catalyst include a palladium catalyst. The palladium catalyst is not particularly limited, and $Pd(PPh_3)_4$, $Pd(PPh_3)_2Cl_2$, and the like are particularly preferable. These catalysts

may be used alone, or two or more of them may be used in combination as necessary. The amount of the catalyst to be used is not particularly limited, and may be such that, for example, the number of moles thereof is 0.002 to 0.1 times, preferably 0.005 to 0.04 times, and more preferably 0.01 to 0.02 times the number of moles of the halogenated quinoline (IV). The catalyst may or may not be used in combination with another substance as necessary. The above described another substance is not particularly limited, and examples thereof include basic substances such as alkali metal carbonates, alkaline-earth metal carbonates, and triethylamine. $K_2CO_3$ and triethylamine are particularly preferable. The amount of such a substance to be used is not particularly limited, and is such that, for example, the number of moles thereof is 50 to 500 times, preferably 100 to 400 times, and more preferably 200 to 300 times the number of moles of the halogenated quinoline (IV). Furthermore, the solvent to be used for the reaction between the compounds (IV) and (V) is not particularly limited, and may be water or an organic solvent, for example. Examples of the organic solvent include: ethers such as diethyl ether, tetrahydrofuran (THF), 1,3-dioxane, 1,4-dioxane, 1,3-dioxolane, thioxane, ethylene glycol dimethyl ether (1,2-dimethoxyethane), diethylene glycol dimethyl ether, and methyl-t-butyl ether; dichloroethane; and DMF. These solvents may be used alone, or two or more of them may be used in combination. The reaction temperature is not particularly limited, and is, for example, 50°C to 120°C, preferably 80°C to 100°C, and more preferably 90°C to 100°C. The reaction time also is not particularly limited, and is, for example, 10 to 50 hours, preferably 15 to 30 hours, and more preferably 20 to 25 hours.

As a second process for producing the quinoline derivative represented by the formula (II), it is preferable to produce the quinoline derivative by reacting 1-acyl-2-aminobenzene represented by the following formula (VI) with a ketone represented by the following formula (VII).

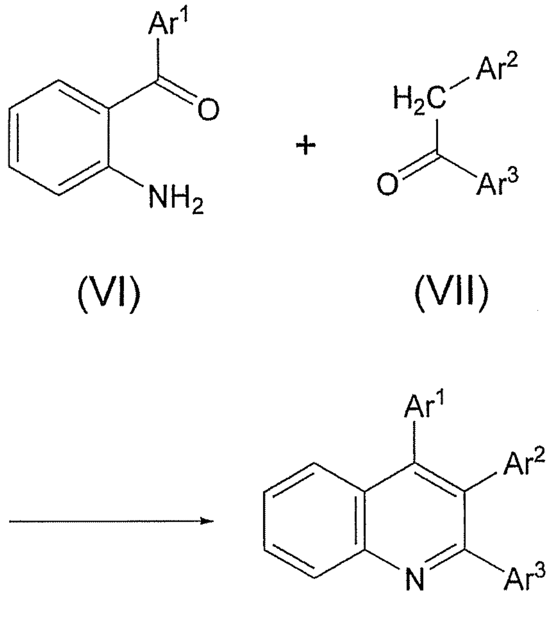

(VI) (VII)

(II)

In the formula (VI), $Ar^1$ is the same as that in the formula (I). In the formula (VII), $Ar^2$ and $Ar^3$ are the same as those in the formula (I).

Conditions for the reaction between the 1-acyl-2-aminobenzene represented by the formula (VI) and the ketone represented by the formula (VII) are not particularly limited, and can be set as appropriate with reference to conditions for known similar reactions, for example. The amount ratio (molar ratio) between the 1-acyl-2-aminobenzene (VI) and the ketone (VII) is not particularly limited, and is, for example, 1 : 3 to 1 : 10, preferably 1 : 3 to 1 : 5, and particularly preferably 1 : 3. Furthermore, for example, a reactant other than the 1-acyl-2-aminobenzene (VI) and the ketone (VII) and a solvent may or may not be used appropriately as necessary. In the case where a solvent is used, the concentration of the 1-acyl-2-aminobenzene (VI) is not particularly limited, and is, for example, 0.2 to 3.0 mol/l, preferably 0.5 to 2.0 mol/l, and more preferably 0.8 to 1.0 mol/l. Examples of the reactant other than the 1-acyl-2-aminobenzene (VI) and the ketone (VII) include: phosphites such as diphenylphosphite; potassium hydroxide; and sodium hydroxide. They may be used alone, or two or more of them may be used in combination as necessary. The amount of the reactant to be used is not particularly limited, and is such that, for example, the number of moles thereof is 2 to 10 times, preferably 3 to 8 times, and more preferably 5 to 6 times the number of moles of the 1-acyl-2-aminobenzene (VI). Furthermore, the solvent to be used for the reaction between the 1-acyl-2-aminobenzene (VI) and the ketone (VII) is not particularly limited, and

may be water or an organic solvent, for example. The organic solvent is not particularly limited, and preferably is a polar solvent. Examples thereof include: phenols; hydroxybenzenes such as orthocresol, metacresol, and paracresol; DMF; and DMSO. These solvents may be used alone, or two or more of them may be used in combination. The reaction temperature is not particularly limited, and is, for example, 100°C to 200°C, preferably 120°C to 160°C, and more preferably 130°C to 140°C. The reaction time also is not particularly limited, and is, for example, 5 to 30 hours, preferably 10 to 25 hours, and more preferably 20 to 25 hours.

The process for producing the compound represented by the formula (VI) also is not particularly limited. Preferably, it is produced by reacting a compound represented by the following formula (VIII) with a halide represented by the following formula (IX), for example.

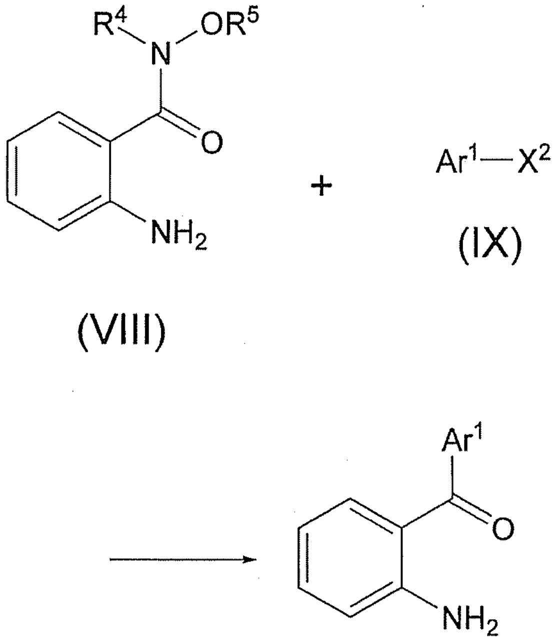

(IX)

(VIII)

(VI)

In the formula (VIII), $R^4$ and $R^5$ are each a hydrogen atom or an alkyl group, and may be identical to or different from each other. In the formula (IX), $Ar^1$ is the same as that in the formula (VI), and $X^2$ is a halogen. Preferably, $R^4$ and $R^5$ are each a hydrogen atom or a straight-chain or branched alkyl group having 1 to 6 carbon atoms. As the alkyl group, a methyl group or an ethyl group is particularly preferable.

Conditions for the reaction between the compound represented by the formula (VIII) and the halide represented by the formula (IX) are not particularly limited, and can be set as appropriate with reference to conditions for known similar reactions, for example. The amount ratio (molar ratio) between the compound (VIII) and the halide (IX) is not particularly limited, and is, for example, 1 : 1 to 1 : 2, preferably 1 :1 to 1 : 1.5, and particularly preferably 1 : 1. Furthermore, for example, a reactant other than the compound (VIII) and the halide (IX) and a solvent may or may not be used appropriately as necessary. In the case where a solvent is used, the concentration of the compound (VIII) is not particularly limited, and is, for example, 0.05 to 0.8 mol/l, preferably 0.1 to 0.5 mol/l, and more preferably 0.2 to 0.3 mol/l. Examples of the reactant other than the compound (VIII) and halide (IX) include organolithium reagents such as n-butyllithium. They may be used alone, or two or more of them may be used in combination as required. The amount of the organolithium reagent to be used is not particularly limited, and is such that, for example, the number of moles thereof is 1.5 to 2.5 times, preferably 1.6 to 2.3 times, and more preferably 1.9 to 2.1 times the number of moles of the compound (VIII). Furthermore, the solvent is not particularly limited, and may be water or an organic solvent, for example. Examples of the organic solvent include ethers such as diethyl ether, tetrahydrofuran (THF), 1,3-dioxane, 1,4-dioxane, 1,3-dioxolane, thioxane, ethylene glycol dimethyl ether (1,2-dimethoxyethane), diethylene glycol dimethyl ether, and methyl-t-butyl ether. These solvents may be used alone, or two or more of them may be used in combination. The reaction temperature is not particularly limited, and is, for example, -100 to -50°C, preferably -80 to -60°C, and more preferably -80 to -70°C. The reaction time also is not particularly limited, and is, for example, 1 to 5 hours, preferably 2 to 4 hours, and more preferably 2 to 3 hours.

As described above, the reactants and solvents to be used for the above-described reactions are not particularly

limited. However, it is preferable to use them in suitable combinations. For example, a substance such as n-butyllithium has a high reactivity with water, so that the reactivity thereof may be affected by water contained in the solvent. In such a case, it is preferable to remove as much water as possible from the solvent. Furthermore, as described above, the process for producing an electron donor-acceptor linked molecule in the present invention is not limited to those described above, and any production process may be employed.

[<1-3> Composite material of electron donor-acceptor linked molecules and porous material]

In the production process according to the present invention, a porous material further may be used, and the electron donor-acceptor linked molecules may form a composite material with the porous material. It is preferable that, in the composite material, the electron donor-acceptor linked molecules are present in pores of the porous material. The porous material is not particularly limited, and examples thereof include inorganic materials, organic materials, and other materials (e.g., active carbon and the like). Among them, inorganic materials are preferable. It is more preferable that the porous material is at least one of aluminum-substituted mesoporous silica and zeolite. In the present invention, "aluminum-substituted mesoporous silica" refers to a material obtained by substitution of at least one of silicon (Si) atoms in mesoporous silica with an aluminum (Al) atom.

If the porous material and the electron donor-acceptor linked molecules form a composite material, a long-lived electron-transfer state (charge-separated state) can be obtained more easily as compared with the case where electron and donor-acceptor linked molecules are used alone. The reason for this is not necessarily clear, but can be considered to be as follows, for example. That is, the electron-transfer state of electron donor-acceptor linked molecules is generated by exciting them through light irradiation or the like in a solution, for example. When two or more molecules in the electron-transfer state are present in proximity to each other in the solution, they are deactivated because they returns to their original state easily due to intermolecular electron transfer reactions. In contrast, the structure of the composite material is such that, for example, the electron donor-acceptor linked molecules are inserted into pores of the porous material. Thus, it is considered that the electron donor-acceptor linked molecules that are in the electron-transfer state are isolated from each other, so that the intermolecular electron transfer reactions can be inhibited. This allows high reactivity of the electron-transfer state to be utilized still more efficiently. It is to be noted, however, that this description merely relates to an example of a presumable mechanism, and does not limit the present invention by any means.

As the porous material, the one having a size suitable for the molecular size of the electron donor-acceptor linked molecules is preferable. For example, for the nitrogen-containing aromatic cation derivative represented by any one of the formulae (A-1) to (A-8), aluminum-substituted mesoporous silica is particularly preferable, and for the quinolinium ion derivative represented by the formula (I), zeolite is particularly preferable. This will be described more specifically below.

Mesoporous silica has a relatively large pore size. Thus, electron donor-acceptor linked molecules with a large molecular size, such as those shown in the formulae (A-1) to (A-8), also can be inserted into the pores of the mesoporous silica. This allows a long-lived electron-transfer state to be obtained at room temperature or temperatures higher than room temperature, for example. The lifetime of the electron-transfer state of an inorganic-organic composite material formed of aluminum-substituted mesoporous silica and the nitrogen-containing aromatic cation derivative represented by any one of the formulae (A-1) to (A-8) is not particularly limited. In a vacuum at 25°C (298 K), the lifetime of the electron-transfer state of the inorganic-organic composite material is, for example, 1 second or more, preferably $1 \times 10^2$ seconds or more, and more preferably $5 \times 10^2$ seconds or more, and the upper limit thereof is not particularly limited, and is, for example, $1 \times 10^4$ seconds or less. Furthermore, in a vacuum at 100°C (373 K), the lifetime of the electron-transfer state of the inorganic-organic composite material is, for example, $1 \times 10^{-1}$ seconds or more, preferably 10 seconds or more, and more preferably $1 \times 10^2$ seconds or more, and the upper limit thereof is not particularly limited, and is, for example, $5 \times 10^3$ seconds or less.

Furthermore, the quinolinium ion derivative represented by the formula (I) has a relatively small molecular size. Thus, it is considered that the quinolinium ion derivative can be inserted into zeolite with a relatively small pore size easily, thus forming a stable composite material. In particular, in the compound (I), the size of the quinolinium ion site (electron acceptor site) is as small as about 7Å according to theoretical calculation. Thus, it is considered that the quinolinium ion derivative can be inserted easily and stably into pores of a porous material with the diameter of an inlet opening being 1 nm or less. It is to be noted, however, that this description merely is an example of theoretical considerations, and the present invention is by no means limited by this consideration. 1 Å is equal to 0.1 nm, i.e., $10^{-10}$ m.

The electron donor-acceptor linked molecules that form a composite material with the aluminum-substituted mesoporous silica preferably are cationic molecules. The cationic molecule may or may not be a molecule represented by any of the formulae (A-1) to (A-8). That is, in the aluminum-substituted mesoporous silica, at least one of silicon (Si) atoms on the silicon dioxide skeleton is substituted with an aluminum (Al) atom. Thus, the aluminum atom becomes an acid site (negatively charged site). As a result, the aluminum-substituted mesoporous silica (inorganic material) has a structure such that the entire skeleton thereof is negatively charged, and cations (e.g., alkali metal ions such as Li, Na,

and K; any other metal ions; etc.) are incorporated into its pores (mesopores). By adding the cationic electron donor-acceptor linked molecules thereto, a cation-exchange reaction occurs between the cations in the pores and the electron donor-acceptor linked molecules. Thus, it is possible to form a composite material in which the electron donor-acceptor linked molecules are incorporated in the pores of the aluminum-substituted mesoporous silica. It is to be noted, however, that these descriptions merely are illustrative, and do not limit the present invention by any means.

Next, aluminum-substituted mesoporous silica and zeolite will be described.

[<1-3-2> Aluminum-substituted mesoporous silica]

Mesoporous silica is silica gel with mesopores, i.e., pores having a relatively large pore size. The aluminum-substituted mesoporous silica in the composite material may or may not contain an element other than Si, Al, and O. In the present invention, the element other than Si, Al, and O that may be contained in the aluminum-substituted mesoporous silica is not particularly limited, and examples thereof include hydrogen (H), alkali metals (Li, Na, K, Rb, Cs, and Fr), halogens (F, Cl, Br, I, and At), and transition metals (e.g., , Fe, Cu, and Mn).

The pore size of the pores, i.e., "mesopores" of the aluminum-substituted mesoporous silica in the composite material is not particularly limited. For example, the aluminum-substituted mesoporous silica may be as follows. That is, first, the magnification of TEM (transmission electron microscope, manufactured by Hitachi, Ltd., trade name "HITACHI Model H-800 transmission electron microscope") is set to $5 \times 10^5$, and a square region with a size of 1 micrometer square on a surface of the aluminum-substituted mesoporous silica is observed. The aluminum-substituted mesoporous silica may be such that, among all the mesopores observed in the square region, the number of mesopores having a long diameter (maximum diameter) $L_{max}$ (nm) and a short diameter (minimum diameter) $L_{min}$ (nm) both within a certain numerical range is 80% or more of the total number of the observed mesopores. The certain numerical range is, for example, 2 to 50 nm, preferably 2 to 10 nm, and more preferably 2 to 4 nm. It is to be noted, however, that the above measurement method merely is an example of a method for measuring the pore size of the mesopores of the aluminum-substituted mesoporous silica. In the present invention, the aluminum-substituted mesoporous silica in the composite material is not limited to the one measured by this measurement method.

The aluminum-substituted mesoporous silica in the composite material is a substance having a structure in which at least one of silicon (Si) atoms of mesoporous silica, i.e., a substance having silicon dioxide as a basic skeleton, is substituted with an aluminum (Al) atom. Thus, it also can be referred to as a kind of zeolite. The term "zeolite" may refer to, in a restricted sense, among various substances having a structure in which at least one of silicon (Si) atoms in a substance having silicon dioxide as a basic skeleton is substituted with an aluminum (Al) atom, those having a pore size of about 2.0 nm or less (e.g., 0.5 to 2.0 nm). However, in the present invention, the term "zeolite" refers generally to substances having a structure in which at least one of silicon (Si) atoms in a substance having silicon dioxide as a basic skeleton is substituted with an aluminum (Al) atom, and encompasses aluminum-substituted mesoporous silica.

The aluminum-substituted mesoporous silica in the composite material is not particularly limited, and a known aluminum-substituted mesoporous silica can be used as appropriate, for example. Furthermore, in the present invention, the aluminum-substituted mesoporous silica synthesized (produced) as appropriate may be used, or a commercially available product may be used. The production process when synthesizing (producing) the aluminum-substituted mesoporous silica also is not particularly limited. For example, it can be produced by a sol-gel method using a surfactant as a template. By changing the kind of the surfactant, it is possible to control the size and shape of the pores and the packing structure. This will be described more specifically below, for example. First, in an inert gas atmosphere, an aqueous solution of an aluminum compound (e.g., triisopropoxyaluminum) and alkali (e.g., sodium hydroxide) is prepared. In this aqueous solution, a surfactant is dissolved so that its concentration becomes equal to or greater than the critical micelle concentration, thereby causing micelle particles having a certain size and structure to be formed depending on the kind of the surfactant. The solution is allowed to stand still for a while, and the micelle particles form a packing structure and convert to colloidal crystals. Then, tetraethoxysilane or the like that serves as a silica source further is added to this solution, so that a sol-gel reaction is allowed to proceed in gaps between the colloidal crystals. Thus, an aluminum-substituted mesoporous silica skeleton is formed. After the completion of the reaction, the obtained aluminum-substituted mesoporous silica is subjected to calcination at a high temperature to degrade and remove the surfactant used as the template, whereby pure aluminum-substituted mesoporous silica is obtained. In this manner, aluminum-substituted mesoporous silica to be used in the present invention can be obtained.

In the reaction of synthesizing the aluminum-substituted mesoporous silica, the reaction temperature is not particularly limited. For example, the reaction may be carried out in water at room temperature (5°C to 35°C) or in hot water (water at 100°C). Also, the reaction may be carried out at an appropriate temperature between these temperatures. The reaction time also is not particularly limited, and may be set as appropriate. Furthermore, for example, by using a small-molecule cationic surfactant as the surfactant, aluminum-substituted mesoporous silica corresponding to MCM series (e.g., MCM-41) mesoporous silica can be produced. Still further, for example, by using a block copolymer as the surfactant, aluminum-substituted mesoporous silica corresponding to SBA series (e.g., SBA-15) mesoporous silica can

be produced. The reaction of synthesizing the aluminum-substituted mesoporous silica can be carried out as appropriate with reference to the following literatures (1) to (4), for example.

(1) Kresge, C. T.; Leonowicz, M. E.; Roth, W. J.; Vartuli, J. C.; Beck, J. S. Nature 1992, 359, 710-712
(2) Beck, J. S. et al., J. Am. Chem. Soc., 1992, 114, 10834
(3) Corma, A.; Kumar, D. Stud. Surf. Sci. Catal., 1998, 117, 201
(4) Janicke, M. T. et al., J. Am. Chem. Soc., 1998, 120, 6940-6951.

In the composite material, the aluminum-substituted mesoporous silica is not particularly limited, as described above. Preferably, the aluminum-substituted mesoporous silica is aluminum-substituted mesoporous silica having a structure in which at least one of silicon (Si) atoms in, for example, MCM-41, MCM-48, MCM-50, SBA-15, or FSM-16 mesoporous silica is substituted with an aluminum (Al) atom. They may be used alone, or two or more of them may be used in combination. Among them, aluminum-substituted mesoporous silica (AlMCM-41) having a structure in which at least one of silicon (Si) atoms in MCM-41 mesoporous silica is substituted with an aluminum (Al) atom is particularly preferable. In the present invention, the pore size of the pores (mesopores) of AlMCM-41 is not particularly limited, and is 2 to 10 nm, for example.

In the present invention, the structure of the composite material is not particularly limited. For example, as described above, it is preferable that the composite material has a structure in which the electron donor-acceptor linked molecules are inserted to the pores of the aluminum-substituted mesoporous silica. The reason for this is as follows. If the electron donor-acceptor linked molecules are inserted to and immobilized on the pores by ion exchange, they are not desorbed easily, thus providing a stable structure. Thus, it is considered that properties such as high reactivity in the electron-transfer state can be utilized sufficiently. It is to be noted, however, that this description merely relates to an example of a presumable mechanism, and does not limit the present invention by any means.

[<1-3-3> Zeolite]

In the composite material, zeolite is not particularly limited, and Y zeolite, A zeolite, X zeolite, L zeolite, beta zeolite, ferrierite-type zeolite, mordenite-type zeolite, ZSM-5 zeolite, TS-1 zeolite, or MCM-22 zeolite is preferable, for example. They may be used alone, or two or more of them may be used in combination. Among them, Y zeolite is particularly preferable because it has a relatively large pore size and is relatively readily available, for example. Y zeolite is not particularly limited, and SK40 (trade name) and the like available from GL Sciences Inc. can be used, for example.

In the present invention, the structure of the composite material is not particularly limited. For example, as described above, the structure preferably is such that electron donor-acceptor linked molecules of the quinolinium ion derivative (I) or the like are inserted to pores of the zeolite. It is more preferable that the pores are supercages. The reason for this is as follows. If the electron donor-acceptor linked molecules are inserted into supercages, they are not detached easily, thus providing a stable structure. Thus, properties such as high reactivity in the electron-transfer state can be utilized sufficiently. It is to be noted, however, that this description does not limit the present invention by any means.

[<1-3-4> Process for producing composite material of electron donor-acceptor linked molecule and porous material]

The process for producing the composite material is not particularly limited. For example, the composite material can be produced by a production process including the step of immersing the aluminum-substituted mesoporous silica or the zeolite in a solution of the electron donor-acceptor linked molecules or a salt thereof. This will be described more specifically below.

The production process can be carried out in the following manner, for example. First, the aluminum-substituted mesoporous silica or the zeolite is pretreated by calcination. The process for producing the composite material may or may not include this pretreatment step, but preferably includes this pretreatment step. The reason for this is as follows. By calcinating the aluminum-substituted mesoporous silica (zeolite), water molecules and the like in pores are removed. Thus, it is considered that the electron donor-acceptor linked molecules can be inserted more easily, thus allowing the activity and the like of the composite material to be improved. It is to be noted, however, that the present invention is by no means limited by this consideration.

The calcination temperature in the pretreatment step is not particularly limited, and is, for example, 100°C to 400°C, preferably 150°C to 350°C, and more preferably 200°C to 300°C. The calcination time also is not particularly limited, and is, for example, 2 to 24 hours, preferably 5 to 15 hours, and more preferably 8 to 12 hours. The atmosphere at the time of calcination also is not particularly limited. For example, the calcination may be carried out in the air, in an inert gas atmosphere, or under reduced pressure, for example. The calcination method also is not particularly limited, and the calcination can be carried out using a device generally used for calcination of zeolite, such as an electric furnace or a drying machine (dryer) as appropriate, for example. The kinds of the aluminum-substituted mesoporous silica and

zeolite that can be used are not particularly limited, and are as described above, for example.

Next, the step of immersing the aluminum-substituted mesoporous silica or the zeolite in the solution of the electron donor-acceptor linked molecules or a salt thereof is carried out. This step may be carried out by simply immersing the aluminum-substituted mesoporous silica or the zeolite in the solution of the electron donor-acceptor linked molecules or a salt thereof. However, it is preferable to carry out this step while stirring the solution in terms of the formation speed of the composite material, for example. In the solution, the solvent is not particularly limited. One kind of solvent may be used, or two or more kinds of solvents may be used in combination. From the viewpoint of the solubility and the like of the electron donor-acceptor linked molecules or a salt thereof, a solvent with high polarity is preferable. More specifically, for example, it is preferable that the solvent is at least one selected from the group consisting of nitriles, halogenated solvents, ethers, amides, sulfoxides, ketones, alcohols, and water. The nitriles are not particularly limited, and examples thereof include acetonitrile, benzonitrile, and butyronitrile. The halogenated solvents are not particularly limited, and examples thereof include chloroform and dichloromethane. The ethers are not particularly limited, and examples thereof include THF (tetrahydrofuran). The amides are not particularly limited, and examples thereof include DMF (dimethylformamide). The sulfoxides are not particularly limited, and examples thereof include DMSO (dimethylsulfoxide). The ketones are not particularly limited, and examples thereof include acetone. The alcohols are not particularly limited, and examples thereof include methanol. Among these solvents, acetonitrile is particularly preferable. In the solution, the concentration of the electron donor-acceptor linked molecules is not particularly limited, and is, for example, $1.25 \times 10^{-5}$ to $1.50 \times 10^{-4}$ mol/l, preferably $5.0 \times 10^{-5}$ to $1.50 \times 10^{-4}$ mol/l, and particularly preferably $1.0 \times 10^{-4}$ to $1.25 \times 10^{-4}$ mol/l. Furthermore, in the solution, the amount (the number of moles) of the electron donor-acceptor linked molecules or a salt thereof is not particularly limited, and is, for example, $2.5 \times 10^{-5}$ to $3.0 \times 10^{-4}$ mol, preferably $1.0 \times 10^{-4}$ to $2.9 \times 10^{-4}$ mol, and more preferably $2.0 \times 10^{-4}$ to $2.5 \times 10^{-4}$ mol per 1 g of the aluminum-substituted mesoporous silica. In this immersion step, the temperature of the solution also is not particularly limited, and is, for example, 0°C to 45°C, preferably 15°C to 40°C, more preferably 20°C to 30°C, and particularly preferably 20°C to 25°C. For example, it is preferable to perform the immersion step at room temperature without heating or cooling the solution for simplicity in operation, for example. The immersion time also is not particularly limited, and is, for example, 1 to 48 hours, preferably 3 to 48 hours, more preferably 8 to 36 hours, and particularly preferably 12 to 24 hours.

Furthermore, an isolation step of collecting the composite material by filtration, washing it, and then drying it is carried out. This isolation step also is not particularly limited, and may be omitted if it is not necessary. A solvent to be used for washing is not particularly limited, and the same solvent or the like as used in the immersion step can be used, for example. The drying temperature also is not particularly limited, and is, for example, 15°C to 60°C, preferably 20°C to 50°C, and more preferably 25°C to 40°C. The atmosphere at the time of drying also is not particularly limited. For example, the drying may be carried out in the air, an inert gas atmosphere, or the like. Furthermore, although the drying may be performed under ordinary pressure, it is preferable to perform the drying under reduced pressure from the viewpoint of drying rate.

[<2> Raw material aromatic compound]

In the present invention, the raw material aromatic compound comprises a benzene, naphthalene or thiophene aromatic ring having at least one substituent selected from the group consisting of $-OR^{100}$, $-NR^{200}_2$, and $AR^{100}$ which is covalently bound to the benzene, naphthalene or thiphene aromatic ring. $R^{100}$ is a hydrogen atom or an arbitrary substituent, and when a plurality of $R^{100}$s are present, they may be identical to or different from each other. $R^{200}$s are each a hydrogen atom or an arbitrary substituent, and they may be identical to or different from each other. $AR^{100}$ is an aryl group, and when a plurality of $AR^{100}$s are present, they may be identical to or different from each other.

$AR^{100}$ may be a group derived from any aromatic ring such as a benzene ring, a naphthalene ring, an anthracene ring, a phenanthrene ring, a pyridine ring, a thiophene ring, or a pyrene ring. The aromatic ring further may have one or more substituents thereon, and when a plurality of substituents are present, they may be identical to or different from each other. $AR^{100}$ may be a phenyl group, for example.

$R^{100}$ preferably is at least one selected from the group consisting of a hydrogen atom, alkyl groups, aryl groups, and acyl groups. The alkyl group preferably is a straight-chain or branched alkyl group having 1 to 6 carbon atoms, and a methyl group is particularly preferable. The acyl group preferably is a straight-chain or branched acyl group having 1 to 6 carbon atoms. The aryl group is the same as $AR^{100}$, for example, and is a phenyl group, for example.

$R^{200}$ preferably is at least one selected from the group consisting of a hydrogen atom, alkyl groups, aryl groups, and acyl groups. The alkyl group preferably is a straight-chain or branched alkyl group having 1 to 6 carbon atoms, and a methyl group is particularly preferable. The acyl group preferably is a straight-chain or branched acyl group having 1 to 6 carbon atoms. The aryl group is the same as $AR^{100}$, for example, and is a phenyl group, for example. As $-NR^{200}_2$, an amino group substituted with an electron donor substituent, such as a dimethylamino group or a diphenylamino group, is preferable because of its particularly high electron-donating property.

Furthermore, the raw material aromatic compound may be such that, for example, a substituent such as an

alkyl group is covalently bound to the aromatic ring, and the substituent may be oxidized in the step of generating the oxidation reaction product. For example, the raw material aromatic compound may contain a methylene group ($-CH_2-$) covalently bound to the aromatic ring, and in the step of generating the oxidation reaction product, the methylene group ($-CH_2-$) may be converted to a carbonyl group (-CO-) by oxidation. In this case, an atom or atomic group that is bound to the methylene group and the carbonyl group is not particularly limited, and examples thereof include a hydrogen atom, alkyl groups, and aryl groups. The alkyl group preferably is a straight-chain or branched alkyl group having 1 to 6 carbon atoms. The alkyl group and aryl group may further be substituted with one or more substituents. When they are substituted with a plurality of substituents, the substituents may be identical to or different from each other. For example, the methylene group becomes a methyl group ($-CH_3$) when hydrogen is bound thereto, and it becomes a formyl group (-CHO) after oxidation. The methylene group becomes an ethyl group ($-CH_2CH_3$) when a methyl group is bound thereto, and it becomes an acetyl group ($-COCH_3$) after oxidation. The methylene group becomes a benzyl group ($-CH_2Ph$) when a phenyl group is bound thereto, and it becomes a benzoyl group (-COPh) after oxidation.

[<3> Oxidizing agent]

The oxidizing agent is oxygen as an elementary substance. Examples of the oxygen as an elementary substance include an oxygen molecule ($O_2$), ozone ($O_3$), and ions thereof, and an oxygen molecule is particularly preferable. The oxygen molecule ($O_2$) may be provided in a reaction system prior to the step of generating the oxidation reaction product or the step of generating an electron-transfer state to be described below, for example. Alternatively, in the step of generating the oxidation reaction product, $O_2$ in the air may be taken in and utilized as appropriate. In either case, since oxygen molecules ($O_2$) are present universally in the air, using this as an oxidizing agent is preferable in terms of cost reduction.

When the oxidizing agent is an oxygen molecule as shown in Scheme 2 or 2', for example, hydrogen peroxide may be produced as a by-product in the step of generating the oxidation reaction product. In this case, collecting the by-product hydrogen peroxide and utilizing it is most preferable from the view point of industrial applicability. In this case, the production process according to the present invention also can be referred to as a process for producing hydrogen peroxide. Specifically, the process for producing hydrogen peroxide according to the present invention includes the step of: producing an oxidation reaction product of a raw material aromatic compound by the process for producing an oxidation reaction product of a raw material aromatic compound according to the present invention. In this process for producing hydrogen peroxide, the oxidizing agent contains oxygen ($O_2$), and a supply source of a hydrogen atom further is used. In the step of generating the oxidation reaction product, the oxygen binds to the hydrogen atom, whereby the hydrogen peroxide is produced. The supply source of the hydrogen atom is not particularly limited. For example, at least one of the electron donor-acceptor linked molecule, the oxidizing agent, and the raw material aromatic compound also may serve as the hydrogen atom supply source. Alternatively, the hydrogen atom supply source may be a substance other than the electron donor-acceptor linked molecule, the oxidizing agent, and the raw material aromatic compound. For example, in the case of Scheme 2 or 2', it is considered that water contained in a solvent serves as a supply source of a hydrogen atom (proton). It should be noted, however, that this merely is an example of a presumable reaction mechanism, and does not limit the present invention by any means. Furthermore, the method for collecting the generated hydrogen peroxide is not particularly limited. Furthermore, high-purity hydrogen peroxide or hydrogen peroxide solution suitable for practical use may be obtained by purifying the collected hydrogen peroxide as necessary. Specifically, for example, a high-concentration hydrogen peroxide solution is obtained by extracting the generated hydrogen peroxide with ion-exchange water or the like and then distilling it under reduced pressure.

Hydrogen peroxide is a compound that is highly useful for various applications such as industrial use, as well as experiments and research. Examples of the industrial use include various uses such as pulp bleaching at the time of paper manufacturing, wastewater treatment, and cleaning of semiconductors. Hydrogen peroxide can be degraded to water and oxygen after use. Thus, it is considered that, unlike chlorine-based bleaching agents etc., hydrogen peroxide does not produce any harmful substance and thus places little burden on the environment. Therefore, the demand for hydrogen peroxide is increasing in recent years. Furthermore, as is well known, a hydrogen peroxide solution is used for disinfection and sterilization under the trade names of Oxydol, Oxyfull, etc., and plays an important role for pharmaceutical and medical purposes, for example. However, hydrogen peroxide is produced by a method (anthraquinone method) utilizing an autoxidation reaction of an anthracene derivative with hydrogen ($H_2$) and oxygen ($O_2$) as raw materials, for example. Thus, there is a problem in that the production cost thereof is very high. Accordingly, collecting and utilizing the hydrogen peroxide that may be generated as a by-product in the production process according to the present invention can lead to a reduction in the production cost of hydrogen peroxide.

[<4>Brønsted acid or anion thereof]

The production process according to the present invention is configured so that,

a Bronsted acid or anion thereof further is used,
in the step of generating the oxidation reaction product, the electron donor-acceptor linked molecule in the electron-transfer state, the oxidizing agent, the raw material aromatic compound, and further the Bronsted acid or anion thereof are reacted, and
the oxidation reaction product contains a substitution product obtained by substitution of at least one hydrogen atom on an aromatic ring of the raw material aromatic compound with an atom or atomic group of the Bronsted acid anion.

In the present invention, it is preferable that the Bronsted acid is a hydrogen halide, and the substitution product is an aromatic halide obtained by substitution of at least one hydrogen atom on the aromatic ring of the raw material aromatic compound with a halogen. Using a hydrogen halide in a substitution reaction of the aromatic ring as described above leads to the reduction of environmental load, the cost for an aftertreatment, etc., because the toxicity thereof is much lower than that of a halogen (e.g., bromine) as an elementary substance. Examples of the hydrogen halide include hydrofluoric acid, hydrochloric acid, hydrobromic acid, and hydroiodic acid. Among them, hydrobromic acid or hydroiodic acid is preferable because of their high reactivity.
Furthermore, examples of the Brønsted acid further include sulfuric acid, nitric acid, sulfonic acid, carboxylic acid, and phosphoric acid, in addition to the hydrogen halides. Examples of the carboxylic acid include acetic acid.

[<5> Process for producing oxidation reaction product of raw material aromatic compound]

As described above, the production process according to the present invention is a process for producing an oxidation reaction product of a raw material aromatic compound by reacting the raw material aromatic compound with an oxidizing agent. The process further uses an electron donor-acceptor linked molecule and a Brønsted acid or anion thereof. The process includes the step of: reacting the electron donor-acceptor linked molecule in an electron-transfer state, the oxidizing agent, the raw material aromatic compound and the Brønsted acid or anion thereof, thereby generating an oxidation reaction product resulting from oxidation of the raw material aromatic compound.

[<5-2> Reaction system]

In the production process according to the present invention, the reaction system is not particularly limited as long as it contains the electron donor-acceptor linked molecules, the oxidizing agent, and the raw material aromatic compound and further a Brønsted acid or anion thereof. Furthermore, the production process according to the present invention optionally may include the step of providing the reaction system. For example, a solution or a suspension obtained by adding the electron donor-acceptor linked molecules, the oxidizing agent, and the raw material aromatic compound to a solvent may be provided as the reaction system. For example, when oxygen ($O_2$) is used as the oxidizing agent as shown in Schemes 1, 2, and 2', it is preferable that the reaction system is saturated with oxygen ($O_2$) beforehand. The concentration of the electron donor-acceptor linked molecules in the reaction system is not particularly limited, and is, for example, $10^{-5}$ to 1 mol/l, preferably $10^{-3}$ to 1 mol/l, and still more preferably $10^{-3}$ to $10^{-2}$ mol/l. The electron donor-acceptor linked molecules may form a composite material with the porous material. In this case, for example, the electron donor-acceptor linked molecules may be added to a solvent in the form of the composite material. Alternatively, the electron donor-acceptor linked molecules and the porous material may be added to a solvent separately, and the composite material may be formed in the solvent.

The reaction system may further contain a substance other than the electron donor-acceptor linked molecules, the oxidizing agent, the raw material aromatic compound and the Brønsted acid or anion thereof. When an oxidative substitution reaction by an atom or atomic group of the Bronsted acid anion is to be caused, the amount ratio between the raw material aromatic compound and the Brønsted acid or salt thereof is not particularly limited. They may be at the stoichiometric ratio, or either of them may be present excessively.

The reaction system may be such that, for example, the raw material aromatic compound is liquid and also serves as a solvent. Furthermore, from the viewpoint of the reaction efficiency, reaction selectivity, cost, and the like, any suitable substance other than the raw material aromatic compound may be used as a solvent. The solvent may be either water or an organic solvent, or may be a mixed solvent of water and an organic solvent. For example, when the Bronsted acid or anion thereof is used, an organic solution may be provided by dissolving the raw material aromatic compound in an organic solvent, and an aqueous solution may be provided separately by dissolving the Brønsted acid or salt thereof in water. Then, both the solutions may be mixed together. Examples of the organic solvent include: nitriles such as benzonitrile, acetonitrile, and butyronitrile; halogenated solvents such as chloroform and dichloromethane; ethers such as THF (tetrahydrofuran); amides such as DMF (dimethylformamide); sulfoxides such as DMSO (dimethylsulfoxide); ketones such as acetone; and alcohols such as methanol. These organic solvents may be used alone, or two or more of them may be used in combination. Furthermore, it is preferable to use the organic solvent with low toxicity and small environmental load. As the solvent, the one with high polarity is preferable from the viewpoint of the solubility of the electron donor-acceptor linked molecules, the stability of the excited state, etc., and acetonitrile is particularly

preferable. Acetonitrile is preferable also from the viewpoint of the solubility in the raw material aromatic compound, and the affinity to water. Furthermore, acetonitrile also is advantageous in that the toxicity and the environmental load thereof are relatively small, and an aftertreatment and the like can be carried out easily. Furthermore, the reaction system further may contain, for example, a pH adjuster, from the viewpoint of reactivity to be described below. Examples of the pH adjuster include: basic substances such as sodium hydroxide and potassium hydroxide; and acidic substances such as hydrochloric acid, sulfuric acid, nitric acid, phosphoric acid, and acetic acid. Furthermore, for example, the water may be in the form of a pH buffer solution with a pH buffer agent being dissolved therein. Examples of the pH buffer solution include phosphate-buffered aqueous solution. The amounts of the pH adjuster and the pH buffer agent to be added are not particularly limited, and can be set as appropriate. When an oxidative substitution reaction by an atom or atomic group of the Bronsted acid anion is to be caused, it is preferable to use a pH adjuster that does not interfere with the oxidative substitution reaction or not to use a pH adjuster.

[<5-3> Electron-transfer state of electron donor-acceptor linked molecules]

In the production process according to the present invention, the electron donor-acceptor linked molecules that already are in the electron-transfer state may be used, or the production process further may include the step of generating an electron-transfer state of the electron donor-acceptor linked molecules. The electron-transfer state generation step is not particularly limited. The electron-transfer state generation step may be carried out either inside or outside of the reaction system containing the electron donor-acceptor linked molecules, the oxidizing agent, and the raw material aromatic compound. Preferably, the electron-transfer state generation step is carried out in the reaction system. The electron-transfer state generation step may be carried out prior to, at the same time with, or after the step of providing the reaction system, for example. As described above, the step of providing the reaction system is optional. In the electron-transfer state generation step, the method for generating the electron-transfer state (charge-separated state) of the electron donor-acceptor linked molecules is not particularly limited, and photoexcitation preferably is employed. Furthermore, excitation light also is not particularly limited. For example, visible light is more preferable. In order to perform excitation with visible light, it is preferable that the electron donor-acceptor linked molecules have an absorption band in the visible region. This allows a long-lived charge-separated state having both a high oxidizing power and a high reducing power to be generated easily. Among the wavelengths of the visible light used for irradiation, a more preferred wavelength is, for example, 300 to 450 nm, still more preferably 350 to 450 nm, although it may vary depending on the absorption band of the electron donor-acceptor linked molecules. In the case where the electron donor-acceptor linked molecule is the quinolinium ion derivative represented by any one of the above formulae 1 to 5, it is still more preferable that the wavelength of the excitation light is 300 to 360 nm. In the case where the electron donor-acceptor linked molecule is the mesityl acridinium derivative represented by the above formula (A-10), it is still more preferable that the wavelength of the excitation light is 350 to 430 nm. The temperature at the time of visible light irradiation also is not particularly limited. For example, it is also possible to allow the reaction (excitation) to proceed at a room temperature of about 10°C to 30°C.

In the photoexcitation, excitation can be performed easily by using visible light contained in natural light such as sunlight, for example. Also, for example, instead of or in addition to the natural light, a light source such as a xenon lamp, a halogen lamp, a fluorescent lamp, or a mercury lamp may or may not be used as appropriate. Furthermore, a filter that cuts wavelengths other than a necessary wavelength may or may not be used as appropriate.

[<5-4> Step of generating oxidation reaction product]

The step of generating the oxidation reaction product is not particularly limited. For example, the oxidation reaction product resulting from oxidation of the raw material aromatic compound may be generated by allowing the reaction system containing the electron donor-acceptor linked molecules in the electron-transfer state, the oxidizing agent, and the raw material aromatic compound to stand still, thereby causing the reaction of the electron donor-acceptor linked molecule in the electron-transfer state, the oxidizing agent, and the raw material aromatic compound. The reaction system may be subjected to heating or the like as necessary. However, causing the reaction only by photoexcitation without heating or the like is preferable in terms of simplicity in operation. Furthermore, it is more preferable to irradiate the reaction system with light continuously so as to carry out that the electron-transfer state generation step and the oxidation reaction product generation step continuously at the same time. The time period for light irradiation, the light intensity, and the like are not particularly limited, and can be set as appropriate.

In the oxidation reaction product generation step, an oxidation reaction of the raw material aromatic compound occurs as described above. This oxidation reaction is not particularly limited, and examples thereof include, as described above, an oxidative substitution reaction of the aromatic ring and an oxidation reaction of a substituent on the aromatic ring.

The reaction mechanism of the oxidation reaction product generation step can be represented by Scheme 2 or 2' shown above, for example. According to this reaction mechanism, the electron donor-acceptor linked molecule can

be used as a substance playing a catalytic role. Thus, theoretically, it is possible to reuse the electron donor-acceptor linked molecule infinitely, thereby realizing low cost. It is to be noted, however, that this reaction mechanism merely is in theory. In general, similarly to common catalytic reactions, the number of molecular rotations is limited owing to deterioration of a catalyst, for example. Furthermore, as described above, Schemes 2 and 2' merely illustrate a presumable reaction mechanism, and do not limit the present invention. In the oxidation reaction product generation step, the pH of the reaction system is not particularly limited, and is, for example, 1 to 6, preferably 1 to 5, and more preferably 1 to 4. Reduction of the electron donor-acceptor linked molecules by the oxidizing agent often can be carried out more efficiently under an acidic condition, although it may vary depending on other reaction conditions. On the other hand, oxidation (electron abstraction) of the raw material aromatic compound by the electron donor-acceptor linked molecules often can be carried out more efficiency under a basic condition. Therefore, it is preferable to set the pH of the reaction system appropriately taking them into account.

Furthermore, in the oxidation reaction product generation step, when the electron donor-acceptor linked molecules play a catalytic role, TON (turnover number) and TOF (turnover frequency, the number of rotations of the catalyst per hour) preferably are as high as possible, although they are not particularly limited. TON means the number of moles of hydrogen peroxide generated per 1 mole of the catalyst throughout the entire oxidation reaction product generation step, and TOF means a numerical value determined by dividing the TON by the time (h) of the hydrogen peroxide reaction step.

The production process according to the present invention can be carried out in the above-described manner. The method for isolating and generating an oxidation reaction product of the raw material aromatic compound as a desired compound is not particularly limited, and may be carried out as appropriate by a conventional method such as distillation or chromatography. Furthermore, as described above, when a highly useful substance such as hydrogen peroxide is produced as a by-product, it is preferable to isolate and utilize this by-product.

Examples

Next, examples of the present invention will be described. It is to be noted, however, that the present invention is by no means limited only to the following examples. Furthermore, theoretical considerations regarding reaction mechanisms and the like to be described below merely are examples of presumable mechanisms and the like, and do not limit the present invention by any means.

In the present example, according to the production process of the present invention, an oxidative substitution reaction of substituting hydrogen on an aromatic ring with a halogen, or a conversion (oxidation) reaction of converting an alkyl substituent to an acyl substituent was carried out in the manner described below. As electron donor-acceptor linked molecules, 9-mesityl-10-methylacridinium perchlorate (purchased from Tokyo Chemical Industry Co., Ltd., reagent grade) was used.

First, an oxygen saturated acetonitrile/water mixed solution (reaction system) containing $Acr^+$-Mes perchlorate (0.2 mM), 1,2,4-trimethoxybenzene (4.0 mM), and hydrogen bromide (20 mM) was provided. Next, this mixed solution was irradiated with light for 30 minutes using a xenon lamp ($\lambda$ > 320 nm). As a result, 1-bromo-2,4,5-trimethoxybenzene was generated with a yield and a selectivity of 100%. As the hydrogen bromide, a commercially available 47% hydrogen bromide aqueous solution was added to the mixed solution so that the concentration of the hydrogen bromide therein became 20 mM. Furthermore, deuterated acetonitrile was used as the acetonitrile. After the completion of the reaction, the mixed solution was subjected to $^1$H NMR measurement without further being treated, and the generation of 1-bromo-2,4,5-trimethoxybenzene as a desired compound was confirmed. Furthermore, the desired compound was isolated and weighed, and the yield was calculated. Furthermore, regarding hydrogen peroxide, confirmation of the generation and quantification thereof were carried out by iodometry. The stoichiometric formula was as shown in Scheme 1. Scheme 1 will be shown again below. From the fact that $Acr^+$-Mes showed little change after the completion of the reaction, it is considered that $Acr^+$-Mes plays a catalytic role as described above.

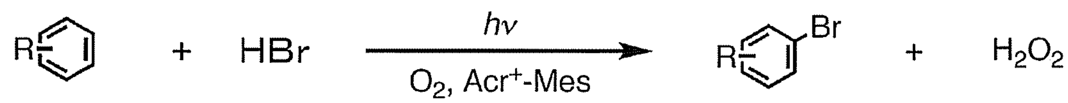


Scheme 1

Next, a reaction was carried out under the same conditions as described above, except that each of various substrates (raw material aromatic compounds) was used instead of 1,2,4-trimethoxybenzene. After the completion of the reaction, the mixed solution was subjected to $^1$H NMR measurement without further being treated as described above. By the $^1$H NMR measurement, the obtained products were identified, the presence or absence of residual raw

materials was checked, and the abundance ratio between each product and the raw material was calculated. Furthermore, substances other than the respective products and the raw material were removed from the reaction mixture. The products and the raw material then were weighed, and the yield of each product was calculated. The results thereof are all shown in Tables 4 and 5. The result obtained when 1,2,4-trimethoxybenzene was used is also shown in these tables.

[Table 4]

| substrate (raw material aromatic compound) | main product | conversion, % | yield, % bromide | light irradiation time, h | by-product | yield, % |
|---|---|---|---|---|---|---|
| 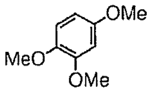 | 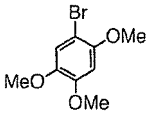 | 100 | 100 | 0.5 | | |
| 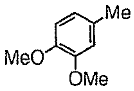 | 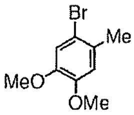 | 100 | 71 | 2 | 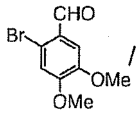 / 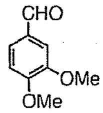 | 11/18 |
| 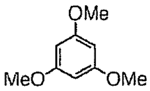 | 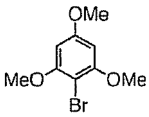 | 100 | 100 | 3 | | |
| 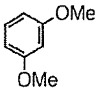 | 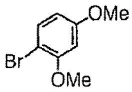 | 14 | 14 | 2 | | |
| 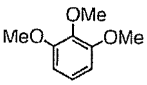 | 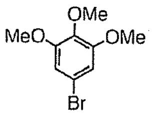 | 100 | 100 | 4 | | |
| 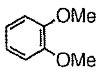 | 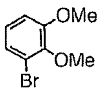 | 55 | 55 | 3 | | |
| 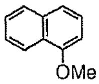 | 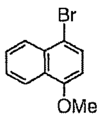 | 100 | 100 | 1.5 | | |

Reaction conditions

| | |
|---|---|
| Substrate (raw material aromatic compound) | 4.0 mM |
| $Acr^+$-Mes | 0.2 mM |
| HBr (aq) | 20 mM |
| $CD_3CN$ | 0.6 ml |

[Table 5]

| substrate (raw material aromatic compound) | main product | conversion, % | yield, % bromide | light irradiation time, h | by-product | yield % |
|---|---|---|---|---|---|---|
| 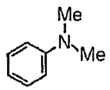 | 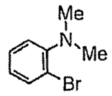 | 100 | 100 | 1 | | |
| 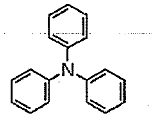 | 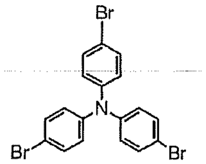 | 100 | 100 | 1 | | |
| 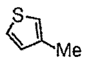 | 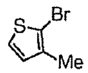 | 100 | 81 | 2 | 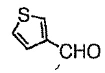 | 19 |
| 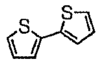 | 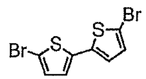 | 100 | 100 | 1.5 | | |
| 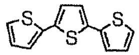 | 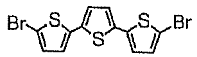 | 100 | 50 | 1.5 | 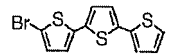 | 50 |
| 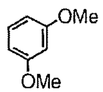 | 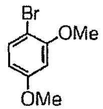 | 55 | 55 | 3 | | |
| 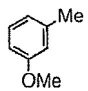 | 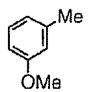 | 50 | 35 | 3 | 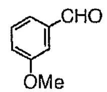 | 15 |
| 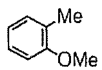 | 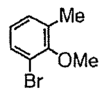 | 75 | 60 | 2 | 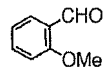 | 15 |
| 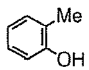 | 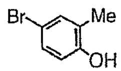 | 75 | 55 | 2 | 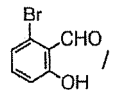 / 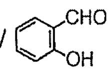 | 10/10 |

Reaction conditions

| | |
|---|---|
| Substrate (raw material aromatic compound) | 4.0 mM |
| $Acr^+$-Mes | 0.2 mM |
| HBr (aq) | 20 mM |
| $CD_3CN$ | 0.6 ml |

As can be seen from Tables 4 and 5, according to the present example, not only in the case where the aromatic ring is a benzene ring but also the case where the aromatic ring is a thiophene ring (heterocyclic ring), a bromine substitution reaction (bromination reaction) of the aromatic ring occurred with a favorable yield, and a substitution product of the raw material aromatic compound was obtained. Furthermore, it was confirmed that, even when the raw material aromatic compound contained amine or thiophene, in other words, even when the raw material aromatic compound contained an element other than carbon, hydrogen, and oxygen (e.g., nitrogen or sulfur), a bromine substitution reaction occurred with high yield and high selectivity.

In the case where triphenylamine was used as the substrate (raw material aromatic compound), hydrochloric acid was used instead of the hydrogen bromide aqueous solution, and light irradiation was performed for 2 hours, tris(4-chlorophenyl)amine was obtained with a conversion ratio and a yield of 31%. That is, it was confirmed that, according to the present invention, not only a bromine substitution reaction of an aromatic ring but also a chlorine substitution reaction was possible.

Aromatic halides are raw materials used widely in order to modify an aromatic ring by a coupling reaction or electrophilic substitution. In order to obtain an aromatic halide, an aromatic halogenation reaction of substituting a hydrogen atom on an aromatic ring with a halogen is important. However, there have been only a few reports about a case where an aromatic halogenation reaction proceeds photocatalytically. According to the present example, it was possible to carry out a photohalogenation reaction of an aromatic hydrocarbon by using, as a photocatalyst, 9-mesityl-10-methylacridinium ion ($Acr^+$-Mes) whose charge-separated state has a long lifetime as well as a strong oxidizing power and a strong reducing power, and also using, as a halogen source, a hydrogen halide aqueous solution (hydrogen bromide aqueous solution or the like) that can be handled easily. This allows a desired aromatic halide to be obtained with high yield and high selectivity. Furthermore, the use of a hydrogen halide aqueous solution (hydrogen bromide aqueous solution or the like) as in the present example is desirable from the viewpoint of toxicity and environmental load, because it has lower toxicity than halogen sources such as bromine or N-bromosuccinimide. The same applies to solvents. More specifically, the toxicity of acetonitrile used as a solvent in the present example is much lower than that of carbon tetrachloride, halogenated hydrocarbon, or the like generally used in a halogenation reaction caused using bromine or N-bromosuccinimide. Photocatalyst reactions that can be carried out under such clean and mild conditions are enormously valuable in terms of industrial applicability.

Furthermore, for example, in a reaction using a halogen source such as bromine or N-bromosuccinimide, bromination may proceed too much, which makes it difficult to obtain only a monobromide with high selectively. However, under the conditions of the present example, a monobromide with only one hydrogen atom on an aromatic ring being substituted with bromine could be obtained with high yield and high selectivity The fact that a desired product can be obtained with high selectivity as described above is advantageous in terms of costs for separating and generating the product.

As described above, according to the present example, an aromatic halide could be obtained with high yield and high selectivity by carrying out a photocatalytic halogenation reaction of an aromatic hydrocarbon by a hydrogen halide (hydrogen bromide aqueous solution or the like) using $Acr^+$-Mes in the electron-transfer state. Furthermore, as can be seen from Tables 4 and 5, in the case where a compound having a methyl group as a substituent on the aromatic ring was used, an oxidation reaction product having a formyl group converted from the methyl group could be obtained. This demonstrates that the present invention is applicable not only to an oxidative substitution reaction of an aromatic ring but also to an oxidation reaction of a substituent on an aromatic ring. Moreover, under the above-described conditions of the present example, the generation of carboxylic acid or the like due to overoxidation of the methyl group did not occur, and only aldehyde could be obtained with high selectivity.

Furthermore, in order to examine the reaction mechanism of a bromine substitution reaction (bromination reaction) according to the present example, nanosecond laser transient absorption measurement was performed. Specifically, a deoxygenated acetonitrile solution containing $Acr^+$-Mes and 1,2,4-trimethoxybenzene was subjected to nanosecond laser flash at 430 nm. As a result, an absorption band derived from 1,2,4-trimethoxybenzene radical cation was observed. From the rate of decay of this absorption band, the first-order dependence on the concentration of the hydrogen bromide aqueous solution was confirmed. From this, it is considered that the reaction proceeds by a reaction

of a radical cationic species generated through one-electron oxidation by $Acr^+$-Mes with a bromide ion. More specifically, this reaction is as shown in Scheme 2 above and as explained by the description regarding Scheme 2. Scheme 2 will be shown again below. As described above, Scheme 2 and the description regarding Scheme 2 merely relate to an example of a presumable reaction mechanism, and do not limit the present invention by any means.

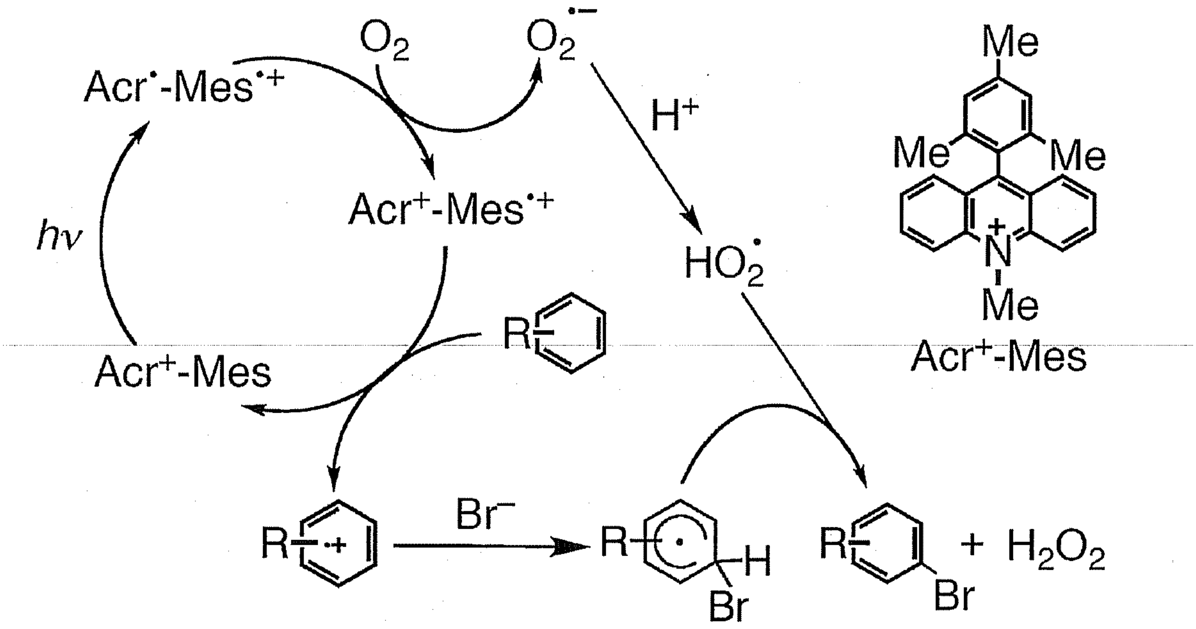


Scheme 2

In Tables 4 and 5, although the mechanism of the reaction in which a methyl group is converted to a formyl group by oxidation is not clear, it is considered that, for example, $O_2^{\cdot -}$ or $HO_2^{\cdot}$ shown in Scheme 2 acts directly on the methyl group independently of $Br^-$. This, however, also merely is an example of a presumable mechanism, and does not limit the present invention by any means.

Furthermore, in the case where any of the above-described other acridinium ion derivatives, quinolinium ion derivatives, and the like was used as electron donor-acceptor linked molecules instead of the 9-mesityl-10-methylacridinium ion, an oxidation reaction product of a raw material aromatic compound also could be produced as in the above described example.

Also, an oxidation reaction product of a raw material aromatic compound could be produced in the same manner as in the above described example using a composite material of electron donor-acceptor linked molecules with an aluminum-substituted mesoporous silica or zeolite. The following reference examples are directed to production examples of such composite materials (inorganic-organic composite materials).

In Reference Examples 1 and 2 to be described below, an inorganic-organic composite material was produced by inserting 9-mesityl-10-methylacridinium ions as cationic electron donor-acceptor (D-A) linked molecules to Na-substituted AlMCM-41 mesoporous silica by ion exchange. The absorbance of a solution (ultraviolet-visible absorption spectrum) was measured using a device named "8453 photodiode array spectrophotometer (trade name)" manufactured by Hewlett-Packard. An ultraviolet-visible absorption spectrum obtained by diffuse reflectance spectroscopy was measured using a Shimadzu UV-3300PC (trade name) manufactured by Shimadzu Corporation and an ISR-3100 (trade name) as an accessory device thereof manufactured by the same. XRD was measured by graphite-monochromatized Cu Ka radiation at room temperature using a RINT-1100 (trade name) manufactured by Rigaku Corporation. An ESR spectrum was measured inside a quartz ESR tube (inner diameter: 4.5 mm) using an X-band spectrometer (trade name "JES-RE1XE") manufactured by JEOL. As a high-pressure mercury lamp, a USH-1005 D (trade name, wavelength: $\lambda > 390$ nm, output: 1000W) manufactured by Ushio Inc. was used. As a xenon lamp, an Ushio Optical Model X SX-UID 500XAMQ (trade name, wavelength: $\lambda > 390$ nm, output: 500 W) manufactured by Ushio Inc. was used. All the chemicals were of reagent grade and were purchased from Tokyo Chemical Industry Co., Ltd., Wako Pure Chemical Industries, Ltd., and Aldrich.

[Synthesis of Na substituted AlMCM-41 mesoporous silica]

Na substituted AlMCM-41 mesoporous silica was synthesized in the following manner with reference to the description in Janicke, M. T. et al., J. Am. Chem. Soc. 1998, 120, 6940-6951. Specifically, first, 0.472 g of $Al(OiPr)_3$, 39.2 g of deionized water, and 5.00 g of 2M NaOHaq were stirred for 1 hour in a recovery flask in an inert gas ($N_2$ or Ar) atmosphere at 25°C (298 K). Next, 0.80 g of cetyltrimethylammonium bromide (CTAB) and 3.85 g of tetraethoxysilane (TEOS) were added thereto, and the resultant mixture was stirred further at 25°C (298 K) for 16 hours. The solid obtained was collected by filtration, washed with water, and heated to 500°C at 2°C/min in an inert gas ($N_2$ or Ar) stream. This was further kept being heated at 500°C for 6 hours. Thereafter, the solid was cooled to room temperature at 2°C/min. As a result, black powder was obtained. This black powder was heated to 500°C at 2°C/min in an inert gas ($N_2$ or Ar) stream, and was further kept being heated at 500°C for 6 hours. Thereafter, the solid was cooled to room temperature at 2°C/min. As a result, a desired compound AlMCM-41 (Na substituted) was obtained in the form of white powder. Furthermore, the reaction was carried out in the same manner, except that hot water at 100°C (373 K) was used instead of the water at 25°C (298 K). As a result, similarly to the above, the desired compound AlMCM-41 (Na substituted) was obtained in the form of white powder.

It was confirmed by XRD that the white powder was a desired compound. The results thereof are shown in the spectral diagram of FIG. 3. In FIG. 3, the horizontal axis indicates 2θ. FIG. 3 shows the XRD pattern of the white powder synthesized in the water at 25°C (298 K). FIG. 4 shows the XRD patterns of AlMCM-41 shown in the literature (Tuel, A. et al. Chem. Mater. 2004, 16, 2969-2974.). The left graph in FIG. 4 shows the XRD patterns before calcination, and the right graph in FIG. 4 shows the XRD patterns after calcination. These XRD patterns are those of [Ca], [Cs], [K], and [Na] AlMCM-41, respectively, from the top. As can be seen from FIGs. 3 and 4, the peak values of the XRD pattern were in excellent agreement with the literature data, whereby it was confirmed that the white powder was the desired AlMCM-41.

[Synthesis of AlSBA-15 mesoporous silica]

AlSBA-15 mesoporous silica having a Brønsted acid site was synthesized in the following manner with reference to the description in Martin, H. et al., J. Phys. Chem. B 2004, 108, 11496-11505. Specifically, first, 4 g of poly(ethylene glycol)-block-poly(propylene glycol)-block-poly(ethylene glycol) (trade name "Pluronic P123"), which is a surfactant with a triblock copolymer structure, and 30 ml of deionized water were stirred in a recovery flask at 25°C (298 K) for 2 hours. Next, 70 ml of 0.29 M hydrochloric acid was added thereto, and the mixture was stirred in the recovery flask at 25°C (298 K) for 2 hours. Then, 9 g of tetraethoxysilane (TEOS) and 1.245 g of $Al(OiPr)_3$ were added thereto, and the resultant mixture was stirred in a recovery flask at 40°C (313 K) for 24 hours. Thereafter, this was further stirred at 110°C (383 K) for 6 hours. The solid obtained was collected by filtration, washed with water, and dried. Thereafter, the solid was heated to 500°C at 1°C/min in an oxygen ($O_2$) stream, and was further kept being heated at 500°C for 6 hours. Thereafter, the solid was cooled to room temperature at 2°C/min. As a result, a desired compound A1SBA-15 was obtained in the form of white powder.

FIG. 13 shows an XRD spectrum of the white powder. By comparing the XRD of FIG. 13 with the XRD data shown in the literature (Martin, H. et al., J. Phys. Chem. B 2004, 108, 11496-11505.), it was confirmed that the white powder was a desired compound.

[Reference Example 1]

As shown in FIG. 1, an inorganic-organic composite material formed of AlMCM-41 aluminum-substituted mesoporous silica (zeolite) and 9-mesityl-10-methylacridinium ions was produced. FIG. 1 is a schematic view. The terms, numerical values, etc. in FIG. 1 merely are illustrative examples of a presumable molecular structure, reaction mechanism, etc., and do not limit the AlMCM-41 zeolite, the 9-mesityl-10-methylacridinium ions, the inorganic-organic composite material, etc. in the present reference example by any means. 1Å is equal to 0.1 nm, i.e., $10^{-10}$ m.

Specifically, to 0.25 g of Na substituted AlMCM-41 zeolite synthesized in water at 25°C (298 K) and pretreated by calcination in the above described manner, 25 ml of $4 \times 10^{-3}$ mol/l acetonitrile solution of 9-mesityl-10-methylacridinium perchlorate (Tokyo Chemical Industry Co., Ltd.) was added. The resultant mixture was stirred at room temperature for 12 hours. Thus, a yellow-green suspension was obtained. This suspension is acetonitrile in which an inorganic-organic composite material formed of the AlMCM-41 zeolite and the 9-mesityl-10-methylacridinium ions is suspended. FIG. 5 shows an ultraviolet-visible absorption spectrum of the acetonitrile solution of the 9-mesityl-10-methylacridinium perchlorate (9-mesityl-10-methylacridinium ions, $Acr^+$-Mes) before the reaction, together with a diffuse reflectance spectrum of the suspension of the inorganic-organic composite material ($Acr^+$-Mes@AlMCM-41) after the reaction. In FIG. 5, the vertical axis indicates an absorbance, and the horizontal axis indicates a wavelength (nm). The dashed line indicates the spectrum of the acetonitrile solution of the 9-mesityl-10-methylacridinium perchlorate (9-mesityl-10-methylacridinium ions, $Acr^+$-Mes) before the reaction, and the solid line indicates the spectrum of the suspension of the inorganic-organic

composite material ($Acr^{+}$-Mes@AlMCM-41) after the reaction. As can be seen from FIG. 5, both before and after the reaction, the maximum absorption wavelength $\lambda_{max}$ was about 360 nm, and no change was seen before and after the reaction. However, the diffuse reflectance spectrum of the suspension (yellow-green) after the reaction shows a different pattern from the acetonitrile solution of the 9-mesityl-10-methylacridinium perchlorate before the reaction and from the Na substituted AlMCM-41 zeolite (colorless, showing no absorption in the visible region). This confirms the generation of the inorganic-organic composite material formed of the AlMCM-41 zeolite and the 9-mesityl-10-methylacridinium ions. It is speculated that, since the pores (mesopores) of AlMCM-41 zeolite have a relatively large pore size of about 2 to 4 nm, the 9-mesityl-10-methylacridinium ions had been inserted into the pores of the AlMCM-41 zeolite by cation exchange with $Na^{+}$ ions. In the present invention, an inorganic-organic composite material may be indicated with "organic material name@inorganic material name" for the sake of convenience. In this case, the organic material name and the inorganic material name include abbreviations of their full names. For example, the "$Acr^{+}$-Mes@AlMCM-41" means a composite material of an inorganic material represented by the abbreviation "AlMCM-41" and an organic material represented by the abbreviation "$Acr^{+}$-Mes".

Furthermore, an inorganic-organic composite material was produced in the same manner as in the above, except that the concentration of the 9-mesityl-10-methylacridinium perchlorate in the acetonitrile solution was varied. Then, based on the amount of the 9-mesityl-10-methylacridinium ions remaining in the acetonitrile solution, the amount of the 9-mesityl-10-methylacridinium ions inserted to the AlMCM-41 zeolite was calculated ($10^{-5}$ mol/g). The results are shown in the graph of FIG. 2. In FIG. 2, the horizontal axis indicates the concentration (mM) of the 9-mesityl-10-methylacridinium perchlorate in the acetonitrile solution, and the vertical axis indicates the amount of the 9-mesityl-10-methylacridinium ions inserted to the AlMCM-41 zeolite ($10^{-5}$ mol/g). As can be seen from FIG. 2, it was confirmed that up to $1.1 \times 10^{-4}$ mol (saturating amount) of the 9-mesityl-10-methylacridinium ions could be inserted into the AlMCM-41 zeolite. Also, Y zeolite (GL Sciences Inc., trade name "SK40") was used instead of the Na substituted AlMCM-41 zeolite, and the amount of the 9-mesityl-10-methylacridinium ions inserted in this case was compared with that when the Na substituted AlMCM-41 zeolite was used. As a result, as shown in FIG. 2, the 9-mesityl-10-methylacridinium ions were not at all inserted into the pores of the Y zeolite. The reason for this is considered to be that the Y zeolite has a small pore size of about 0.7 nm.

Furthermore, the inorganic-organic composite material was removed from the acetonitrile suspension by filtration, and was isolated by drying at 25°C for 5 hours using a vacuum line. Then, it was confirmed that the inorganic-organic composite material was not denatured by, for example, suspending the inorganic-organic composite material again in acetonitrile and then measuring a diffuse reflectance spectrum. That is, this demonstrates that, in the inorganic-organic composite material, the 9-mesityl-10-methylacridinium ions are immobilized on the AlMCM-41 zeolite, so that it cannot be separated (detached) easily.

[Evaluation of charge-separated state]

Regarding the inorganic-organic composite material ($Acr^{+}$-Mes@AlMCM-41) obtained in Reference Example 1, it was checked whether or not the charge-separated state (electron-transfer state) thereof has a long lifetime and is stable. As the inorganic-organic composite material ($Acr^{+}$-Mes@AlMCM-41), among those obtained in Reference Example 1, the one in which an maximum amount (saturating amount), i.e., $1.1 \times 10^{-4}$ mol of the 9-mesityl-10-methylacridinium ions ($Acr^{+}$-Mes) were inserted into the AlMCM-41 zeolite was used after isolation.

Specifically, first, the inorganic-organic composite material ($Acr^{+-}$Mes@AlMCM-41) was irradiated with visible light for 5 seconds under high vacuum at 25°C (298 K) using a high-pressure mercury lamp (wavelength: $\lambda$ > 390 nm). Thereafter, the ESR spectrum was measured. As a result, a signal indicating the presence of a radical was observed. It is considered that this ESR signal is derived from the charge-separated state ($Acr^{\bullet}$-$Mes^{\bullet +}$) generated by intramolecular electron transfer from the Mes site to the $Acr^{+}$ site that is in the singlet excited state. That is, it was confirmed that, in the AlMCM-41 zeolite, the charge-separated state ($Acr^{\bullet}$-$Mes^{\bullet +}$) of the 9-mesityl-10-methylacridinium ions ($Acr^{+}$-Mes) was generated as shown below, thus providing the charge-separated state ($Acr^{\bullet}$-$Mes^{\bullet +}$@AlMCM-41) of the inorganic-organic composite material ($Acr^{+}$-Mes@AlMCM-41). FIG. 6 shows the ESR spectrum. The mark "*" in the spectrum of FIG. 6 indicates an $Mn^{2+}$ESR marker. From the decay in this ESR signal, it was confirmed that the charge-separated state ($Acr^{\bullet}$-$Mes^{\bullet +}$@AlMCM-41) of the inorganic-organic composite material ($Acr^{+}$-Mes@AlMCM-41) had a long lifetime of about 500 seconds and was stable under high vacuum at 25°C (298 K).

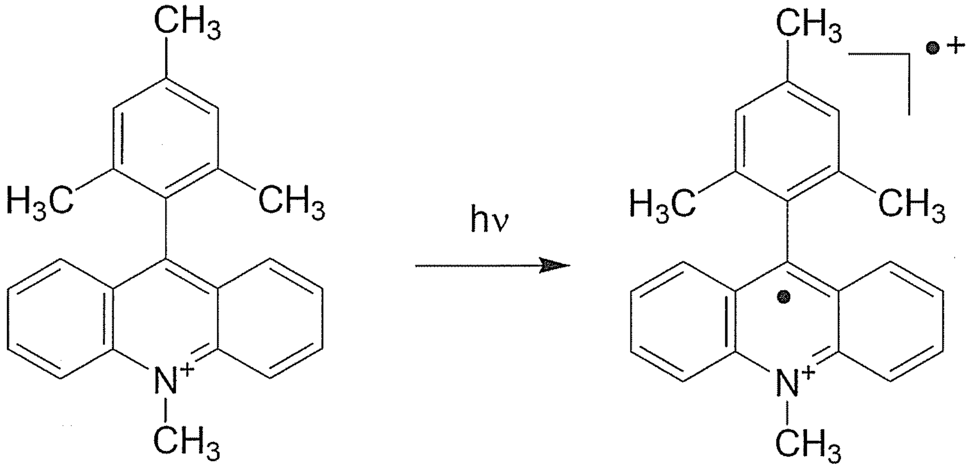


$Acr^{+}$-Mes　　　$Acr^{\cdot}$-$Mes^{\cdot +}$

(A－10)　　　(A－13)

Next, the inorganic-organic composite material ($Acr^{+}$-Mes@AlMCM-41) was irradiated with visible light for 30 seconds under high vacuum at 100°C (373 K) using a high-pressure mercury lamp (wavelength: $\lambda$ > 390 nm). Thereafter, the ESR spectrum was measured in the same manner. As a result, a signal indicating the presence of a radical was observed. It is considered that this ESR signal is derived from the charge-separated state ($Acr^{\cdot}$-$Mes^{\cdot +}$) generated by intramolecular electron transfer from the Mes site to the $Acr^{+}$ site that is in the singlet excited state. That is, similarly to the case where the light irradiation was performed at 25°C (298 K), it was confirmed that, in the AlMCM-41 zeolite, the charge-separated state ($Acr^{\cdot}$-$Mes^{\cdot +}$) of the 9-mesityl-10-methylacridinium ions ($Acr^{+}$-Mes) was generated as shown below, thus providing the charge-separated state ($Acr^{\cdot}$-$Mes^{\cdot +}$@AlMCM-41) of the inorganic-organic composite material ($Acr^{+}$-Mes@AlMCM-41). FIG. 7 shows the ESR spectrum. The mark "*" in the spectrum of FIG. 7 indicates an $Mn^{2+}$ESR marker. Furthermore, the graph of FIG. 8 shows the decay of the ESR signal shown in FIG. 7. In FIG. 8, the horizontal axis indicates an elapsed time (second) after the completion of the light irradiation, and the vertical axis indicates an ESR signal intensity (I). Furthermore, the inset in FIG. 8 is a graph in which the horizontal axis indicates an elapsed time (second) after the completion of the light irradiation, and the vertical axis indicates In (I/Io) (Io means an ESR intensity at a time where the elapsed time after the completion of the light irradiation was zero (i.e., immediately after the light irradiation)). As can be seen from FIG. 8, the decay of this ESR signal followed the first-order reaction rate equation (observed rate constant: $k_{obs}$ = 1.1 x $10^{-2}S^{-1}$). This decay of the ESR signals is considered to be derived from the fact that $Acr^{\cdot}$-$Mes^{\cdot +}$ returned to $Acr^{+}$-Mes again by the intramolecular reverse electron transfer reaction. From the rate of this decay, it was confirmed that, in the inorganic-organic composite material ($Acr^{+}$-Mes@AlMCM-41), the charge-separated state ($Acr^{\cdot}$-$Mes^{\cdot +}$) of the electron donor-acceptor linked molecules incorporated in the AlMCM-41 zeolite had a very long charge separation lifetime of about 10 seconds even at a very high temperature of 100°C.

As described above, the inorganic-organic composite material of Reference Example 1 ($Acr^{+}$-Mes@AlMCM-41) exhibited a long-lived and stable charge-separated state both at a room temperature of 25°C (298 K) and a high temperature of 100°C (373 K). This demonstrates that the inorganic-organic composite material of Reference Example 1 ($Acr^{+}$-Mes@AlMCM-41) has high redox ability, and thus is suitable for use as an oxidizing agent and a reducing agent. Moreover, the fact that visible light irradiation allows the inorganic-organic composite material to exhibit such a long-lived and stable charge-separated state indicates that the inorganic-organic composite material is suitable as a photocatalyst. Conventionally, there has been no case where the charge-separated state of an electron donor-acceptor linked molecules was generated "at temperatures higher than room temperature" and by "visible light irradiation", and the reaction dynamics thereof could be observed by spectroscopic observation as described above. In particular, the generation of a long-lived charge-separated state even at a temperature as high as 100°C is a highly advantageous effect.

Furthermore, the following operations were repeated: after the decay of the ESR signal, irradiation using the high-pressure mercury lamp was carried out again in the same manner as in the above; the irradiation was stopped when the ESR signal reached its maximum; and after the decay of the ESR signal, irradiation using the high-pressure mercury lamp was carried out again under the same conditions. The graph of FIG. 9 shows the ESR signal intensities at these times. In FIG. 9, the horizontal axis indicates an elapsed time, and the vertical axis indicates the ESR intensity

(I). As can be seen from FIG. 9, regardless of the number of times the light irradiation (ON) and the interruption of the light irradiation (OFF) was repeated, the maximum value of the ESR signal did not decrease substantially. This demonstrates that the inorganic-organic composite material of Reference Example 1 ($Acr^+$-Mes@AlMCM-41) is not deteriorated by repeated photoexcitation, and can take repeated use as an oxidizing agent, a reducing agent, a photocatalyst, or the like.

Furthermore, the inorganic-organic composite material of Reference Example 1 ($Acr^+$-Mes@AlMCM-41) was irradiated with light for 300 seconds under high vacuum at 25°C (298 K) using a high-pressure mercury lamp (wavelength: $\lambda$>390nm), and an ultraviolet-visible absorption spectrum (diffuse reflectance spectrum) of the inorganic-organic composite material itself as a solid was measured before and after the light irradiation. The spectral diagram is shown in the graph of FIG. 10. In FIG. 10, the horizontal axis indicates a wavelength (nm), and the vertical axis indicates an absorbance. As can be seen from FIG. 10, an absorption band in the vicinity of 500 nm increased after the light irradiation. It is considered that this is derived from an $Acr^{\cdot}$ radical in the charge-separated state ($Acr^{\cdot}$-$Mes^{\cdot +}$) generated by the light irradiation with respect to the $Acr^+$-Mes inserted in the AlMCM-41 zeolite. According to the checking through visual observation, the inorganic-organic composite material ($Acr^+$-Mes@AlMCM-41) that had been yellow-green before the light irradiation turned to orange after the light irradiation. When at least either one of the electron donor-acceptor linked molecules and the same in the charge-separated state in the inorganic-organic composite material have a unique absorption band in the visible region as described above, it is possible to check the generate of the charge-separated state based on the change in color. This is advantageous in that, for example, when the inorganic-organic composite material is used as an oxidizing agent, a reducing agent, a photocatalyst, or the like, the proceeding of the reaction can be checked easily.

Furthermore, the inorganic-organic composite material of Reference Example 1 ($Acr^+$-Mes@AlMCM-41) was irradiated with light for 10 seconds at 100°C (373 K) using a xenon lamp. As a result, in an ultraviolet-visible absorption spectrum (diffuse reflectance spectrum) of the inorganic-organic composite material itself as a solid, the absorption band in the vicinity of 500 nm increased, as in the above-described case. FIG. 11 shows graphs, which show a decay curve of the diffuse reflectance spectrum and a decay curve of an ESR signal, respectively. FIG. 11A shows the decay curve of the diffuse reflectance spectrum. The horizontal axis indicates an elapsed time (second) after light irradiation, and the vertical axis indicates an absorbance ($\Delta$Abs) at a wavelength of 500 nm. Furthermore, the inset in FIG. 11A is a graph in which the horizontal axis indicates an elapsed time (second) after the completion of the light irradiation, and the vertical axis indicates In $\Delta$Abs. As can be seen from FIG. 11A, the decay of this diffuse reflectance spectrum followed the first-order reaction rate equation (observed rate constant: $k_{obs}$ = 1.1 x $10^{-2}S^{-1}$). Furthermore, FIG. 11B is a graph showing the decay curve of an ESR signal after the irradiation using a high-pressure mercury lamp at 100°C (373 K), and is identical to FIG. 8. As can be seen from FIGs. 11A and 11B, the rate of decay of the ESR signal intensity agreed with the rate of decay of the absorption band derived from $Acr^{\cdot}$ at 500 nm. This result further supports the fact that the inorganic-organic composite material of Reference Example 1 ($Acr^+$-Mes@AlMCM-41) exhibits a long-lived and stable charge-separated state at a temperature as high as 100°C.

As described above, in the inorganic-organic composite material of Reference Example 1 ($Acr^+$-Mes@AlMCM-4l), the electron donor-acceptor linked molecules inserted thereto could be quantified by the respective kinds of spectroscopic observation. When this inorganic-organic composite material was irradiated with light, the generated charge-separated state not only had a long life and was stable at room temperature, but also had a very long life of about 10 seconds and was stable even at a high temperature of 100°C. This charge-separated state could be observed by spectroscopic observations, namely, ESR and diffuse reflectance spectroscopy.

[Reference Example 2]

According to the scheme shown in FIG. 12, an inorganic-organic composite material formed of AlSBA-15 aluminum-substituted mesoporous silica (zeolite) and 9-mesityl-10-methylacridinium ions was produced. As can be seen from FIG. 12, the production scheme is the same as that shown in FIG. 1, except that AlSBA-15 aluminum-substituted mesoporous silica (zeolite) was used instead of AlMCM-41 aluminum-substituted mesoporous silica (zeolite). Similarly to FIG. 1, FIG. 12 merely is a schematic view, and numerical values in FIG. 12 merely are illustrative examples and by no means limit the present invention.

Specifically, to 0.25 g of AlSBA-15 zeolite synthesized in water at 25°C (298 K) and pretreatment by calcination in the above-described manner, 25 ml of 1 $\times$ $10^{-4}$ mol/l acetonitrile solution of 9-mesityl-10-methylacridinium perchlorate (Tokyo Chemical Industry Co., Ltd.) was added, and the resultant mixture was stirred at room temperature for 12 hours to cause a reaction. Thus, a yellow-green suspension was obtained. This suspension is acetonitrile in which an inorganic-organic composite material formed of the AlSBA-15 zeolite and the 9-mesityl-10-methylacridinium ions is suspended. FIG. 14 shows ultraviolet-visible absorption spectra obtained both before the stirring (before the reaction) and after the stirring (after the reaction) in the reaction. The dashed line in FIG. 14 indicates an ultraviolet-visible absorption spectrum of the acetonitrile solution of the 9-mesityl-10-methylacridinium perchlorate (9-mesityl-10-methylacridinium ions,

$Acr^+$-Mes) before the reaction. The solid line in FIG. 14 indicates an ultraviolet-visible absorption spectrum of a supernatant solution obtained by centrifuging the suspension of the inorganic-organic composite material ($Acr^+$-Mes@AlSBA-15) after the reaction. In FIG. 14, the vertical axis indicates an absorbance, and the horizontal axis indicates a wavelength (nm). As can be seen from FIG. 14, both before and after the reaction, the maximum absorption wavelength $\lambda_{max}$ was about 360 nm, and no change was seen before and after the reaction. From the difference between the absorbance indicated with the solid line and the absorbance indicated with the dashed line in FIG. 14, the amount of 9-mesityl-10-methylacridinium ions remaining in the acetonitrile solution could be calculated, based on which the amount of 9-mesityl-10-methylacridinium ions (10-5 mol/g) inserted to the AlSBA-15 zeolite was calculated.

FIG. 15 shows a diffuse reflectance spectrum of the suspension of the inorganic-organic composite material ($Acr^+$-Mes@AlSBA-15) after the reaction. As can be seen from FIG. 15, the diffuse reflectance spectrum of a solid precipitate (yellow-green) obtained by centrifuging the suspension after the reaction shows a different pattern from the acetonitrile solution of the 9-mesityl-10-methylacridinium perchlorate before the reaction and from the AlSBA-15 zeolite (colorless, showing no absorption in the visible region). This confirms the generation of the inorganic-organic composite material formed of the AlSBA-15 zeolite and the 9-mesityl-10-methylacridinium ions. It is speculated that, since the pores (mesopores) of AlSBA-15 zeolite have a relatively large pore size of about 6 to 12 nm, the 9-mesityl-10-methylacridinium ions had been inserted in the pores of the AlSBA-15 zeolite by exchange with protons.

Furthermore, a diffuse reflectance transient absorption spectrum of the inorganic-organic composite material ($Acr^+$-Mes@AlSBA-15) produced (synthesized) in the present reference example was measured by a nanosecond time-resolved laser flash photolysis technique. More specifically, the diffuse reflectance transient absorption spectrum was measured by exciting the inorganic-organic composite material through irradiation with a 10 Hz pulse laser beam (wavelength: $\lambda$ = 430 nm) under high vacuum at 25°C (298 K). FIG. 16 shows a diffuse reflectance transient absorption spectral diagram. In FIG. 16, the horizontal axis indicates a wavelength, and the vertical axis indicates $\Delta J$. $\Delta J$ is a physical amount defined by the following mathematical expression. In the following mathematical expression, Jo is a diffuse reflectance signal intensity before the pulse laser beam irradiation, and $J_t$ is a diffuse reflectance signal intensity after a lapse of time t (second) from immediately after the pulse laser irradiation.

$$\Delta J = (J_0 - J_t)/J_0$$

An open circle (O) in FIG. 16 indicates a spectrum after 0.8 milliseconds, a filled triangle (▲) indicates a spectrum after 4 milliseconds, a filled square (■) indicates a spectrum after 16 milliseconds, a filled circle (●) indicates a spectrum after 50 milliseconds, and an open triangle (△) indicates a spectrum after 150 milliseconds. As can be seen from FIG. 16, in the diffuse reflectance transient absorption spectra of $Acr^+$-Mes@AlSBA-15, an absorption band ($\lambda_{max}$ = 500 nm) considered to be derived from $Acr^\cdot$-$Mes^{\cdot+}$ was observed in the visible region. This demonstrates that $Acr^+$-Mes@AlSBA-15 has a very long charge separation lifetime.

In FIG. 16, an absorption band in a near-infrared region is considered to be derived from a dimer of $Acr^\cdot$-$Mes^{\cdot+}$ and $Acr^+$-Mes, for example. It is to be noted, however, that this merely is an example of possible consideration, and does not limit the present invention by any means.

In Reference Example 3 to be described below, Na-Y zeolite pretreated by calcination was stirred in a solution of electron donor-acceptor linked molecules (compound (I)). Further, the Na-Y zeolite was collected by filtration, washed, and dried. Thus, a composite material of the electron donor-acceptor linked molecules and the zeolite, i.e., an inorganic-organic composite material, was obtained.

In the following, a nuclear magnetic resonance (NMR) spectrum was measured using a device named JNM-AL300 NMR spectrometer (trade name) (300 MHz for $^1H$ measurement) manufactured by JEOL. Chemical shift is indicated in parts per million (ppm). As an internal standard (0 ppm), tetramethylsilane (TMS) was used. The coupling constant (J) is indicated in hertz. Abbreviations s, d, t, q, m, and br indicate a singlet, a doublet, a triplet, a quartet, a multiplet, and a broad, respectively. The mass spectrometry (MS) was measured by the MALDI-TOF-MS method using a device named Kratos Compact MALDI I (trade name) manufactured by Shimadzu Corporation. Elementary analysis values were measured using a Model 240C (trade name) manufactured by Perkin-Elmer. The absorbance (an ultraviolet-visible absorption spectrum) of a solution was measured using a device named 8453 photodiode array spectrophotometer (trade name) manufactured by Hewlett-Packard. For laser irradiation, a device named Nd:YAG laser (SLII-10, 4-6 ns fwhm) (trade name) manufactured by Continuum was used. An ultraviolet-visible absorption spectrum by diffuse reflectance spectroscopy was measured using a Shimadzu UV-3300PC (trade name) manufactured by Shimadzu Corporation and an ISR-3100 (trade name) as an accessory device thereof manufactured by the same All the chemicals were of reagent grade and were purchased from Tokyo Chemical Industry Co., Ltd., Wako Pure Chemical Industries, Ltd., and Aldrich. As zeolite, Y zeolite (GL Sciences Inc., trade name "SK40") was used.

<Synthesis of quinolinium ion derivative salts>

Salts of the quinolinium ion derivatives represented by the above formulae 1 to 5 were synthesized in the following manner.

[1] Synthesis of quinolinium ion derivatives 1 to 5

Salts of the quinolinium derivatives represented by the formulae 1 to 5 were synthesized. The formulae 1 to 5 will be shown below again.

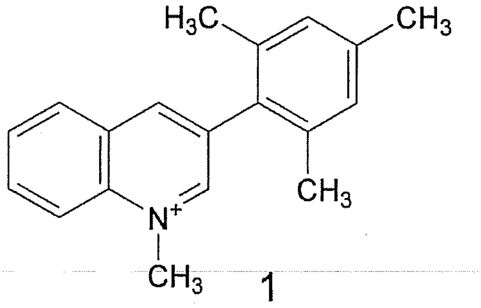
1

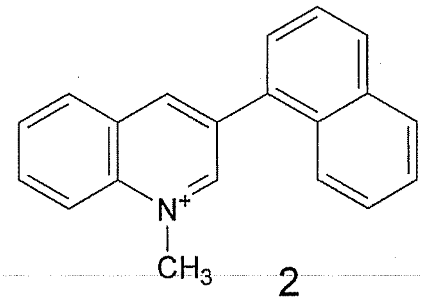
2

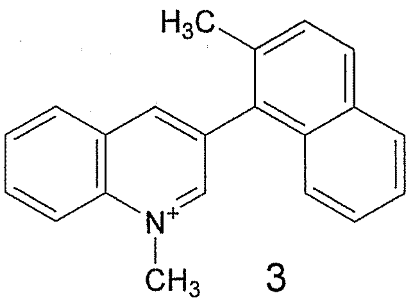
3

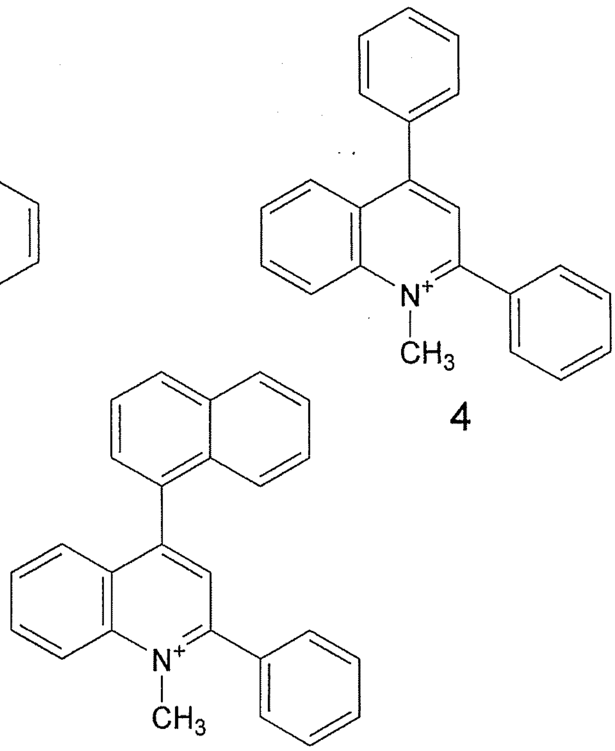
4

5

[1-1] Synthesis of quinolinium ion derivative 1 to 3

According to Scheme 3 below, perchlorate of the quinolinium ion derivative 2 (3-(1-naphthyl)quinolinium ion) was synthesized.

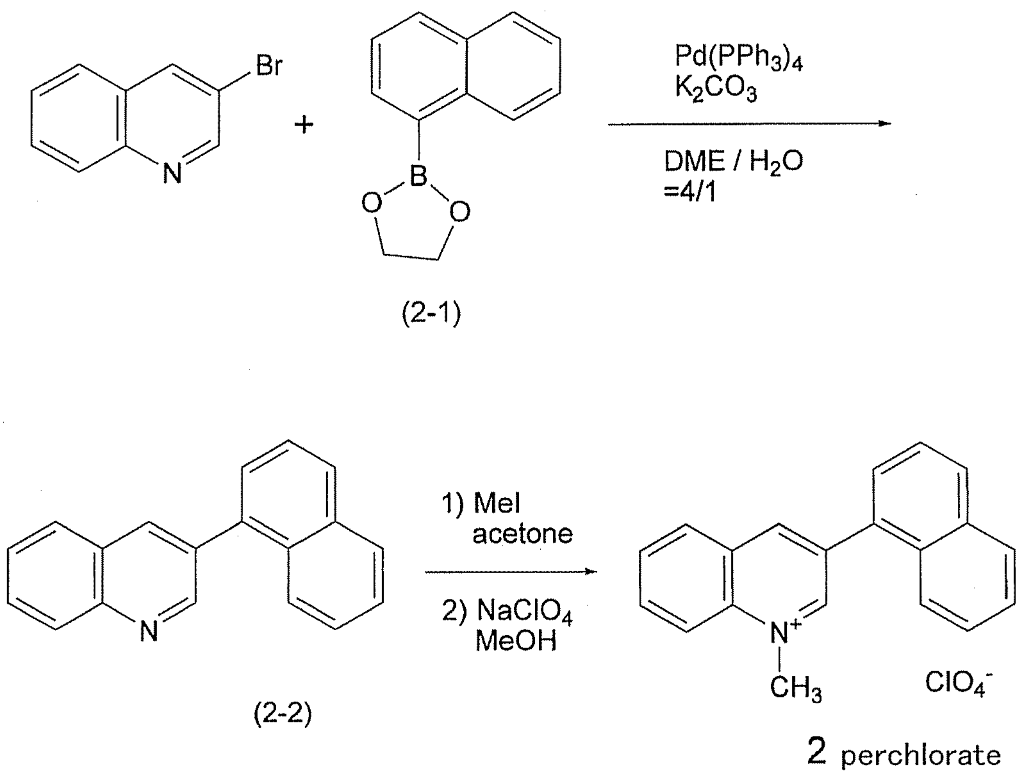


Scheme 3

In the following, Scheme 3 will be described in further detail.

Before causing a reaction of Scheme 3, first, 1-naphthylboronic acid ester (2-1) was synthesized. Specifically, first, in 10 ml of dehydrated THF, a reaction between 1-naphthyl bromide (2.07 g, 10.0 mmol) and magnesium (0.27 g, 11.0 mmol) was caused, thereby generating a Grignard reagent. Next, this Grignard reagent was added to 10 ml of a dehydrated THF solution of trimethoxyborane (2.08 g, 20.0 mmol) at -78°C, and the resultant mixture was stirred for 1 hour. After the completion of the reaction, the solvent was removed. While stirring the thus-obtained white solid in 50 ml of toluene, 5 ml of ethylene glycol was added thereto. Thereafter, the mixture was refluxed at 115°C for 12 hours to cause a reaction. After the completion of the reaction, the mixture was cooled to room temperature. Then, only the toluene phase was extracted, and the solvent was removed. As a result, 1-naphthylboronic acid ester (2-1) was obtained (1.60 g, 81%). Instrumental analysis data regarding this 1-naphthylboronic acid ester (2-1) are shown below.

1-naphthylboronic acid ester (2-1):

$^1H$ NMR (300 MHz, $CDCl_3$) δ 8.73 (d, J = 7.5 Hz, 1H), 8.11 (s, J = 7.5 Hz, 1H), 7.95 (s, J = 7.5 Hz, 1H), 7.84 (s, J = 7.5 Hz, 1H), 7.56-7.45 (m, 3H), 4.52 (s, 4H).

Next, to 4 ml of a dimethoxyethane (DME) solution of the 1-naphthylboronic acid ester (2-1) (1.00 g, 5.00 mmol) and 3-bromoquinoline (0.62 g, 3.00 mmol), 1.0 ml of 2.0 M potassium carbonate aqueous solution and tetrakis(triphenylphosphine)palladium [$Pd(PPh_3)_4$] (30 mg, 0.026 mmol) were added. The resultant mixture was refluxed at 90°C for 12 hours. After the completion of the reaction, the mixture was cooled to room temperature, and 100 ml of chloroform was added thereto. The mixture was washed with 100 ml of water twice, and subsequently washed with 50 ml of saturated saline. The solvent was then removed therefrom, and this was purified by column chromatography using chloroform as a developing solvent. Thus, 3-(1-naphthyl)quinoline (2-2) was obtained (84 mg, 11%). The instrumental analysis data regarding this 3-(1-naphthyl)quinoline (2-2) are shown below.

3-(1-naphthyl)quinoline (2-2):

$^1H$ NMR (300 MHz, $CDCl_3$) δ 9.06 (s, 1H), 8.28 (s, 1H), 8.21 (d, J = 8.4 Hz, 1H), 7.97-7.75 (m, 5H), 7.65-7.46 (m, 5H).

Furthermore, the 3-(1-naphthyl)quinoline (2-2) (70 mg, 0.27 mmol) was dissolved in 10 ml of acetone, and methyl iodide (130 μl, 2 mmol) was further added thereto. The resultant mixture was stirred for 10 hours. The solvent was then removed therefrom, and 20 ml of methanol was added thereto. Subsequently, sodium perchlorate (0.12 g, 1.0 mmol) was added thereto to perform salt exchange (ion exchange). Thus, perchlorate of 3-(1-naphthyl)quinolinium ion

(quinolinium ion derivative 2) was obtained. The yield amount of the thus-obtained perchlorate of the quinolinium ion derivative 2 was 93 mg, and the yield from the 3-(1-naphthyl)quinoline (2-2) was 93%. The instrumental analysis data of this perchlorate of the quinolinium ion derivative 2 are shown below.

perchlorate of quinolinium ion derivative 2:

$^1H$ NMR (300 MHz, $CD_3CN$) δ 9.25 (s, 1H), 9.20 (s, 1H), 8.42 (t, J = 8.4 Hz, 2H), 8.30 (t, J = 8.4 Hz, 1H), 8.15-8.08 (m, 3H), 7.86 (d, J = 8.4 Hz, 1H), 7.74-7.56 (m, 4H), 4.63 (s, 3H), MALDI-TOF-MS m/z 270 ($M^+$ Calcd for $C_{20}H_{16}N$ 270.1). Anal. Calcd for $C_{20}H_{16}ClNO_4$: C, 64.96; H, 4.36; N, 3.79.
Found: C, 64.80; H, 4.24; N, 3.82.

Further, according to Scheme 3' shown below, perchlorate of quinolinium ion derivative 1 was obtained.

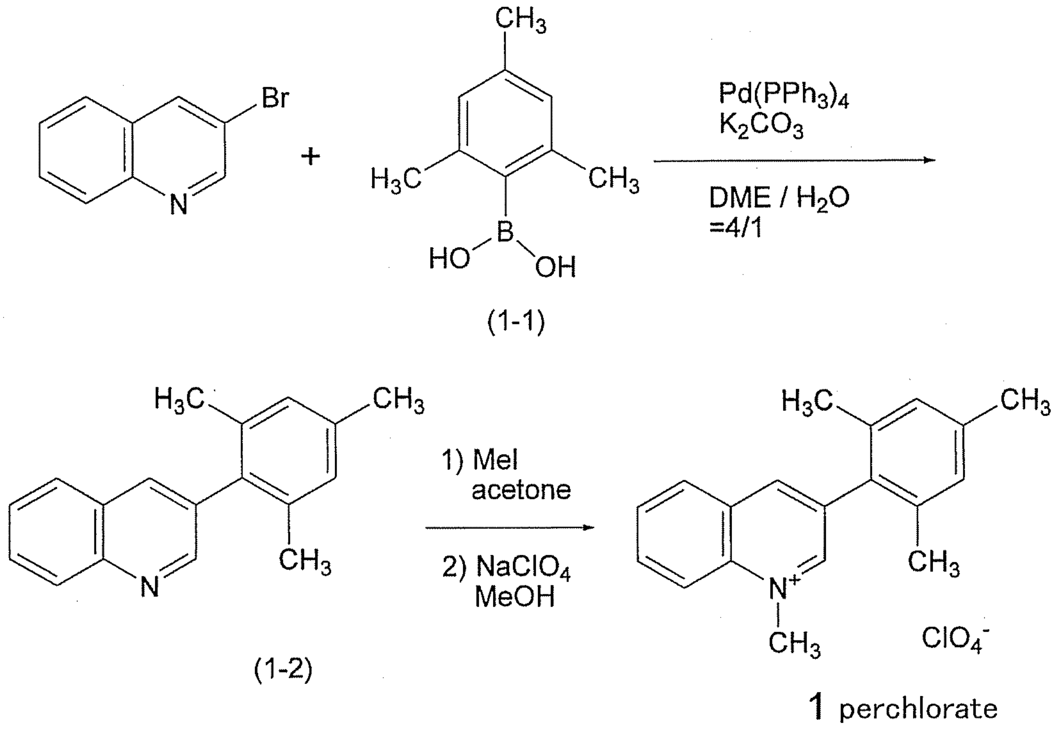


(1-1)

(1-2)

1 perchlorate

Scheme 3'

Specifically, to 8 ml of dimethoxyethane (DME) solution of 2,4,6-trimethylphenylboronic acid (1.64 g, 10.0 mmol) and 3-bromoquinolin (1.24 g, 6.00 mmol), 2.0 ml of 2.0 M potassium carbonate aqueous solution and tetrakis(triphenylphosphine)palladium [$Pd(PPh_3)_4$] (60 mg, 0.052 mmol) were added, and the resultant mixture was refluxed at 90°C for 12 hours. After the completion of the reaction, 100 ml of chloroform was added thereto. The mixture was washed with 100 ml of water twice, and subsequently washed with 50 ml of saturated saline. The solvent was then removed therefrom, and this was purified by column chromatography using dichloromethane as a developing solvent. Thus, 3-(1-mesityl)quinoline (compound 1-2) was obtained (500 mg, 34%).

Methyl iodide (8 mmol) was added to the thus-obtained 3-(1-mesityl)quinoline (500 mg, 2.02 mmol) in 80 ml of acetone, and the resultant mixture was stirred for 10 hours. The solvent was removed therefrom, and 30 ml of methanol was added thereto. Sodium perchlorate (0.12 g, 1.0 mmol) then was added thereto so as to obtain perchlorate through salt exchange. The thus-obtained 3-(1-mesityl)quinolinium ion; $Qu^+$-Mes was 105 mg, and the yield was 14%.

Furthermore, perchlorate of the quinolinium ion derivative 3 was obtained in the same manner as in Scheme 3, except that 2-methyl-1-naphthyl bromide was used instead of 1-naphthyl bromide. The instrumental analysis data regarding the perchlorate of the quinolinium ion derivative 1, the perchlorate of the quinolinium ion derivative 3, and intermediates thereof are shown below.

3-(1-mesityl)quinoline (an intermediate of the perchlorate of the quinolinium ion derivative 1, compound 1-2):

$^1H$ NMR (300 MHz, $CDCl_3$) δ 8.67 (s, 1H), 8.14 (d, J = 8.4 Hz, 1H), 7.96 (s, 1H), 7.76-7.55 (m, 3H), 7.00 (s,

2H), 2.34 (s, 3H), 2.03 (s, 6H).

3-[1-(2-methyl)naphthyl)]quinoline (an intermediate of the perchlorate of the quinolinium ion derivative 3):

$^1$H NMR (300 MHz, $CDCl_3$) δ 8.85 (s, 1H), 8.22 (d, J = 8.4 Hz, 1H), 8.10 (s, 1H), 7.89-7.60 (m, 6H), 7.49-7.32 (m, 3H), 2.29 (s, 3H).

perchlorate of quinolinium ion derivative 1:

$^1$H NMR (300 MHz, $CD_3CN$) δ 8.93 (s, 1H), 8.90 (s, 1H), 8.39 (d, J = 7.8 Hz, 1H), 8.33 (d, J = 7.8 Hz, 1H), 8.26 (t, J = 7.8 Hz, 1H), 8.04 (t, J = 7.8 Hz, 1H), 7.08 (s, 2H), 2.04 (s, 6H), 4.57 (s, 3H), 2.35 (s, 3H), MALDI-TOF-MS m/z 262 ($M^+$ Calcd for $C_{19}H_{20}N$ 261.8). Anal. Calcd for $C_{19}H_{20}ClNO_4$: C, 63.07; H, 5.57; N, 3.87. Found: C, 62.91; H, 5.49; N, 3.89.

perchlorate of quinolinium ion derivative 3:

$^1$H NMR (300 MHz, $CD_3CN$) δ 9.09 (s, 1H), 9.05 (s, 1H), 8.49-8.25 (m, 3H), 7.96-8.12 (m, 3H), 7.62-7.32 (m, 4H), 4.61 (s, 3H), 2.38 (s, 3H), MALDI-TOF-MS m/z 284 ($M^+$ Calcd for $C_{21}H_{18}N$ 284.1). Anal. Calcd for $C_{21}H_{18}ClNO_4$: C, 65.71; H, 4.73; N, 3.65. Found: C, 65.58; H, 4.73; N, 3.65.

[1-2] Synthesis of quinolinium ion derivatives 4 and 5

According to Scheme 4 below, perchlorate of the quinolinium ion derivative 5 (2-phenyl-4-(1-naphthyl)quinolinium ion) was synthesized.

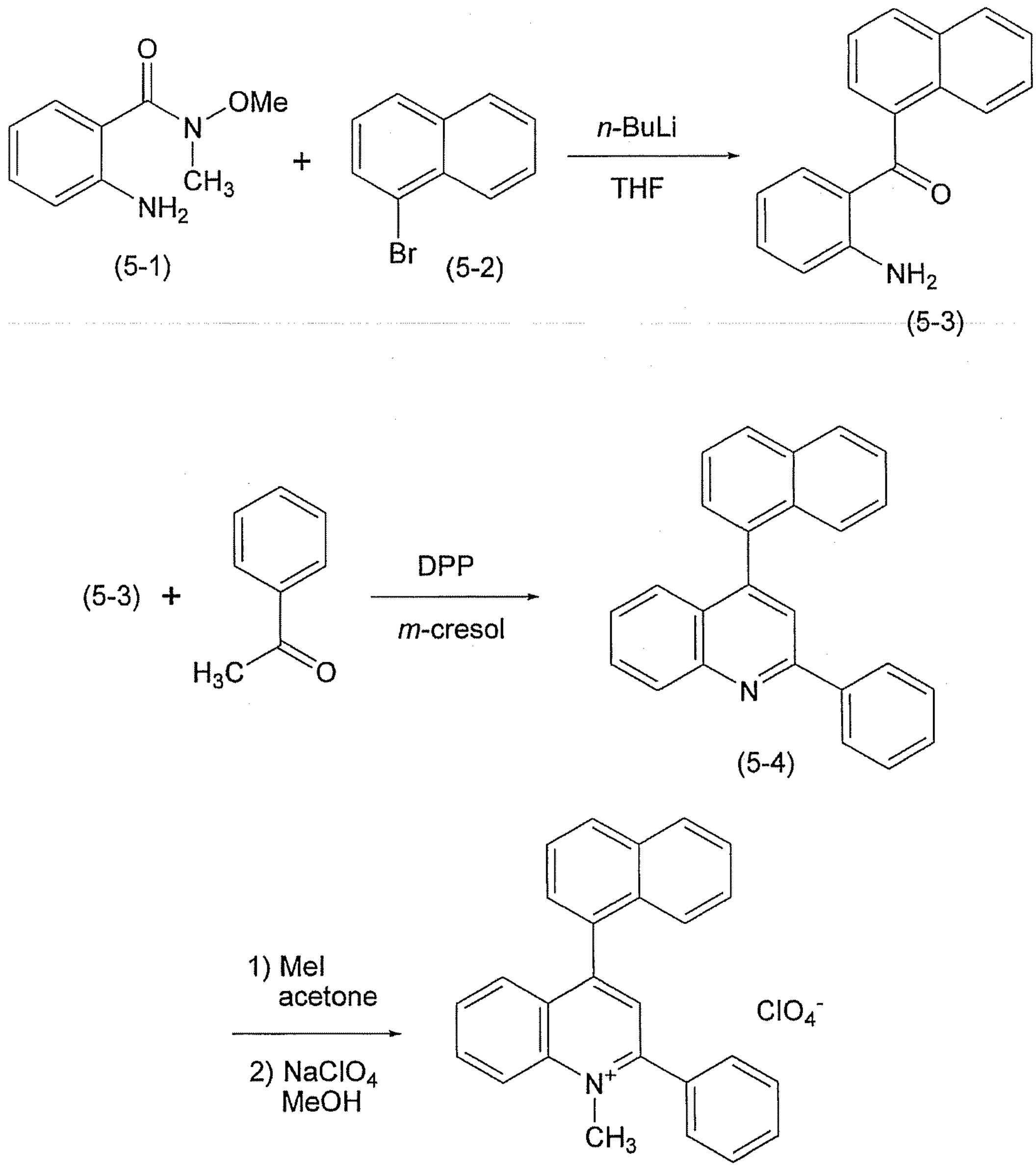


5 perchlorate

Scheme 4

The reaction of Scheme 4 was carried out specifically in the following manner. First, anthranilic acid N-methoxy-N-methylamide (5-1) (2.00 g, 11.1 mmol) and 1-naphthyl bromide (5-2) (2.29 g, 11.1 mmol) were dissolved in 60 ml of dehydrated THF. Then, this solution was cooled to -78°C. To the solution kept at -78°C, n-butyllithium hexane solution (13.8 ml, 1.6M, 22.2 mmol) was added dropwise over 20 minutes while stirring the solution. After the dropwise addition of the n-butyllithium hexane solution, 20 ml of IN hydrochloric acid was added thereto. The resultant mixture was subjected to extraction with 150 ml of ethyl acetate, and then was washed with 100 ml of water twice and subsequently washed with 50 ml of saturated saline. The organic solvent was then removed therefrom, and this was purified by column chromatography using chloroform as a developing solvent. Thus, 1'-naphthyl-2-aminobenzophenone (5-3) was obtained. The yield amount was 500 mg, and the yield was 18%. The instrumental analysis data regarding this 1'-naphthyl-2-aminobenzophenone (5-3) are shown below.

1'-naphthyl-2-aminobenzophenone (5-3):

$^1$H NMR (300 MHz, $CDCl_3$) δ 7.97-7.93 (m, 3H), 7.49-7.42 (m, 4H), 7.28-7.20 (m, 2H), 6.73 (d, J = 7.5Hz, 1H), 6.52 (bs, 1H), 6.43 (t, J = 7.5Hz, 3H).

Next, diphenylphosphite (DPP) (2.5 g, 10.0 mmol) and m-cresol (1.6 g, 14.8 mmol) were added to the 1'-naphthyl-2-aminobenzophenone (5-3) (400 mg, 1.6 mmol) and acetophenone (400 mg, 4.4 mmol), and the resultant mixture was stirred at 140°C for 5 hours. After the completion of the reaction, the mixture was cooled to room temperature, and 100 ml of 10% sodium hydroxide solution and 100 ml of methylene chloride were added thereto. The methylene chloride was separated and collected from the mixture. The mixture was then washed with 100 ml of water three times and subsequently washed with 50 ml of saturated saline. The solvent was removed therefrom, and this then was purified by column chromatography using chloroform as a developing solvent. Thus, 2-phenyl-4-(1-naphthyl)quinoline (5-4) was obtained. The yield amount was 150 mg, and the yield from I'-naphthyl-2-aminobenzophenone (5-3) was 28%. The instrumental analysis data regarding this 2-phenyl-4-(1-naphthyl) quinoline (5-4) are shown below.

2-phenyl-4-(1-naphthyl)quinoline (5-4):

$^1$H NMR (300 MHz, $CDCl_3$) δ 8.27 (d, J = 8.5 Hz, 1H), 8.21 (d, J = 8.5 Hz, 2H), 7.97 (t, J = 8.5 Hz, 2H), 7.91s, 1H), 7.71 (t, J = 8.5Hz, 1H), 7.61-7.32 (m, 11H).

Further, methyl triflate (methyl trifluoromethanesulfonate) (82 mg, 0.50 mmol) was added to 10 ml of methylene chloride solution of 4-naphthyl-2-phenylquinoline (5-4) (150 mg, 0.45 mmol), and the resultant mixture was stirred at room temperature for 2 hours. The solvent was removed therefrom, and 20 ml of methanol was added thereto. Sodium perchlorate (0.12 g, 1.0 mmol) then was added thereto so as to obtain perchlorate through salt exchange. Recrystallization was performed using hot methanol. Thus, 190 mg of perchlorate of 2-phenyl-4-(1-naphthyl)quinolinium ion (quinolinium ion derivative 5) was obtained. The yield from the 4-naphthyl-2-phenylquinoline (5-4) was 95%. The instrumental analysis data regarding the perchlorate of the quinolinium ion derivative 5 are shown below.

perchlorate of quinolinium ion derivative 5:

$^1$H NMR (300 MHz, $CD_3CN$) δ 8.52 (d, J = 9.0 Hz, 1H), 8.25 (t, J = 9.0 Hz, 1H), 8.18 (d, J = 9.0 Hz, 1H), 8.08 (d, J = 9.0 Hz, 1H), 8.05 (s, 1H), 7.82-7.69 (m, 8H), 7.61 (t, J = 9.0 Hz, 2H), 7.45 (t, J = 9.0 Hz, 1H), 7.41 (d, J = 9.0 Hz, 1H), 4.44 (s, 3H), MALDI-TOF-MS m/z 346 ($M^+$ Calcd for $C_{20}H_{16}N$ 346.2). Anal. Calcd for $C_{26}H_{20}ClNO_4$: C, 70.03; H, 4.52; N, 3.14. Found: C, 69.78; H, 4.39; N, 3.19.

Furthermore, perchlorate of the quinolinium ion derivative 4 was obtained in the same manner as in Scheme 4, except that bromobenzene was used instead of 1-naphthyl bromide. The instrumental analysis data of the perchlorate of the quinolinium ion derivative 4 and an intermediate thereof are shown below.

2,4-diphenylquinoline (an intermediate of the perchlorate of the quinolinium ion derivative 4) :

$^1$H NMR (300 MHz, $CDCl_3$) δ 8.26-8.18 (m, 2H), 7.90 (d, J = 8.4Hz, 1H), 7.82 (s, 1H), 7.73 (t, J = 8.4 Hz, 1H), 7.57-7.43 (m, 10H).

perchlorate of quinolinium ion derivative 4:

$^1$H NMR (300 MHz, $CD_3CN$) δ 8.48 (d, J = 8.4 Hz, 1H), 8.31-8.25 (m, 2H), 7.98 (t, J = 8.4 Hz, 1H), 7.95 (s, 1H), 7.75-7.67 (m, 10H), 4.36 (s, 3H), MALDI-TOF-MS m/z 270 ($M^+$ Calcd for $C_{20}H_{16}N$ 270.1). Anal. Calcd for $C_{22}H_{18}ClNO_4$: C, 66.75; H, 4.58; N, 3.54.

[Reference Example 3]

200 mg of Y zeolite was calcinated in an electric furnace at 200°C for 8 hours. This was immersed in 200 ml of acetonitrile solution ($1.0 \times 10^{-4}$ M) of 7.2 mg ($2.0 \times 10^{-2}$ mmol) of the quinolinium ion derivative 1, i.e., 3-(1-mesityl)quinolinium ion ($Qu^+$-Mes), and stirred at room temperature for 12 hours. The Y zeolite was collected by filtration. Thereafter, it was washed three times with acetonitrile, and dried under vacuum (reduced pressure). Thus, an inorganic-organic composite material could be obtained (this is set to Reference Example 3-1).

FIG. 17 shows the results of measuring ultraviolet-visible absorption spectra of the acetonitrile solution before and after the addition of the zeolite in Reference Example 3-1 ($Qu^+$-Mes). In FIG. 17, the horizontal axis indicates a wavelength, and the vertical axis indicates an absorbance. As can be seen from FIG. 17, in Reference Example 1, the absorption band changed considerably after the addition of the zeolite. This confirms the generation of the inorganic-organic composite material. Although the structure of this inorganic-organic composite material is not necessarily clear, it is considered that, for example, $Qu^+$-Mes molecules were inserted in supercages of the Y zeolite. Furthermore, by using the quinolinium ion derivatives 2 to 5, respectively, instead of the quinolinium ion derivative 1 ($Qu^+$-Mes), inorganic-organic composite materials were similarly obtained (they are set to Reference Examples 3-2 to 3-5, respectively).

Furthermore, immersion, collection by filtration, washing, and drying were carried out in the same manner as in the above, except that the amount of the zeolite used in Reference Example 3-1 ($Qu^+$-Mes) was set to 0.50 mg with respect to 1 ml of acetonitrile, and that the concentration of the electron donor-acceptor linked molecule solution was varied to several different values. Then, from the decrease in absorption band on the ultraviolet-visible absorption spectrum of the solution from before to after the addition of the zeolite, the number of $Qu^+$-Mes molecules with respect to ten supercages of the zeolite was determined. The results are shown in the graph of FIG. 18. In FIG. 18, the horizontal axis indicates a concentration of the electron donor-acceptor linked molecule solution, and the vertical axis indicates the number of the electron donor-acceptor linked molecules (D-A dyads) with respect to ten supercages of the zeolite. As can be seen from FIG. 18, $Qu^+$-Mes (mesityl quinolinium ion) formed an inorganic-organic composite material. The number of $Qu^+$-Mes molecules with respect to ten supercages of the zeolite increased in proportion to the $Qu^+$-Mes concentration, and became saturated with 2.2 molecules at a maximum. Furthermore, an ultraviolet-visible absorption spectrum of the solid inorganic-organic composite material that had undergone the immersion, the collection by filtration, the washing, and the drying was measured by diffuse reflectance spectroscopy. As a result, it was confirmed that the absorbance also increases in response to the increase in the number of $Qu^+$-Mes molecules with respect to ten supercages of the zeolite.

Furthermore, 1.0 mg of the inorganic-organic composite material (having undergone the collection by filtration, washing, and drying) of Reference Example 3-1 ($Qu^+$-Mes) was added to 3. 8 ml of acetonitrile and suspended therein, and an ultraviolet-visible absorption spectrum was measured by diffuse reflectance spectroscopy. FIG. 19 shows the result thereof, together with an ultraviolet-visible absorption spectrum of an acetonitrile solution of $Qu^+$-Mes. In FIG. 19, the horizontal axis indicates a wavelength, and the vertical axis indicates an absorbance. In FIG. 19, "zeolite suspension in acetonitrile" indicated with the solid line is an ultraviolet-visible absorption spectrum of the inorganic-organic composite material measured by the diffuse reflectance spectroscopy. Further, "in acetonitrile" indicated with the dotted line is an ultraviolet-visible absorption spectrum of $5.00 \times 10^{-5}$ mol/l acetonitrile solution of $Qu^+$-Mes. As can be seen from FIG. 19, the absorption band in the spectrum of the inorganic-organic composite material obtained by diffuse reflectance spectroscopy was in excellent agreement with the absorption band of the $Qu^+$-Mes solution. This demonstrates that the inorganic-organic composite material of Reference Example 3-1 stably incorporates $Qu^+$-Mes molecules even in an acetonitrile solution.

The inorganic-organic composite material used for the measurement in FIG. 19 was produced by carrying out immersion, collection by filtration, washing, and drying in the same manner as in the above, except that $1.25 \times 10^{-4}$ mol/l acetonitrile solution of the quinolinium ion derivative 1, i.e., 3-(1-mesityl)quinolinium ion ($Qu^+$-Mes), was used and the amount of the zeolite to be used was set to 0.50 mg per 1 ml of acetonitrile. The number of $Qu^+$-Mes molecules with respect to ten supercages of the zeolite was determined, as in the above, from the decrease in absorption band on the ultraviolet-visible absorption spectrum of the solution from before to after the addition of the zeolite in the production process. As a result, the number of $Qu^+$-Mes molecules was found to be 2.2.

Furthermore, regarding the same inorganic-organic composite material (having undergone the collection by filtration, washing, and drying) as that used in the measurement in FIG. 19, an ultraviolet-visible absorption spectrum thereof as a solid was measured by diffuse reflectance spectroscopy without immersing it in a solvent. The result is shown in FIG. 20. In FIG. 20, the horizontal axis indicates a wavelength, and the vertical axis indicates an absorbance. As can be seen from FIG. 20, the maximum absorption wavelength of the inorganic-organic composite material of Reference Example 3-1 itself as a solid measured by diffuse reflectance spectroscopy substantially agreed with the same measured in the state where the inorganic-organic composite material was in acetonitrile (FIG. 19). This measurement result further supports the fact that the inorganic-organic composite material of Reference Example 3-1 is stable in an acetonitrile solution.

When the inorganic-organic composite materials of Reference Examples 3-1 to 3-5 were photoexcited in a solvent such as acetonitrile, the generation of charge-separated state (electron-transfer state) having a still longer lifetime was observed as compared with the cases of the solutions of the quinolinium ion derivatives 1 to 5 alone. Furthermore, since the quinolinium ion derivatives 1 to 5 each have an absorption band in the visible region, the inorganic-organic composite materials containing the same according to Reference Examples 3-1 to 3-5 could be excited with visible light.

Industrial Applicability

As specifically described above, according to the present invention configured as above, it is possible to provide a process for producing an oxidation reaction product of an aromatic compound, having excellent environmental load reduction performance, cost reduction performance, etc. According to the present invention, it is possible to synthesize

aromatic halides, which are used as, e.g., various materials, raw materials of drugs, and the like, with high yield and high selectively. Furthermore, the present invention is applicable not only to such an oxidative substitution reaction of hydrogen on an aromatic ring but also to an oxidation reaction of a substituent on an aromatic ring, and can be used in a broad range of applications. Furthermore, by using an electron donor-acceptor linked molecule as a substance playing a photocatalytic role, it is possible to produce an oxidation reaction product of an aromatic compound easily under mild conditions without the need for heating or the like. Moreover, according to the present invention, by using a reactant, a solvent, and the like with low toxicity, environmental load and cost can be reduced drastically. The present invention is applicable to a variety of areas such as organic synthesis, precise synthesis, etc. at a level appropriate for industrial plants and experimental laboratories. Thus, the present invention is of great use in terms of industrial application.

## Claims

**1.** A process for producing an oxidation reaction product of a raw material aromatic compound comprising a benzene, naphthalene or thiophene aromatic ring having at least one substituent selected from the group consisting of $-OR^{100}$, $-NR^{200}_2$, and $-AR^{100}$ which is covalently bound to the benzene, naphthalene or thiophene ring,
$R^{100}$ is a hydrogen atom or an arbitrary substituent, and when a plurality of $R^{100}$s are present, they may be identical to or different from each other,
$R^{200}$s are each a hydrogen atom or an arbitrary substituent, and they may be identical to or different from each other, and
$AR^{100}$ is an aryl group, and when a plurality of $AR^{100}$s are present, they may be identical to or different from each other,
which process comprises reacting the raw material aromatic compound with an oxidizing agent which is oxygen as an elementary substance, wherein
the process further uses an electron donor-acceptor linked molecule, and a Brønsted acid selected from hydrogen halides, sulfuric acid, nitric acid, sulfonic acid, carboxylic acids and phosphoric acid and anions thereof,
and the process comprises the steps of:

generating an electron-transfer state of the electron donor-acceptor linked molecule by photoexcitation;
reacting the electron donor-acceptor linked molecule in the electron-transfer state, the oxidizing agent, the raw material aromatic compound, and the Brønsted acid or anion thereof thereby generating an oxidation reaction product, wherein;
the oxidation reaction product comprises a substitution product obtained by substitution of at least one hydrogen atom on an aromatic ring of the raw material aromatic compound with an atom or atomic group of the Brønsted acid anion, and
the electron donor-acceptor linked molecule is at least one selected from the group consisting of: nitrogen-containing aromatic cation derivatives represented by the following formulae (A-1) to (A-8); 9-substituted acridinium ions represented by the following formula (A-9); quinolinium ion derivatives represented by the following formula (I); stereoisomers and tautomers thereof; and salts thereof:

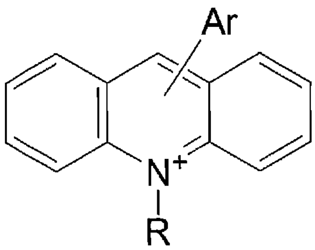
(A-1)

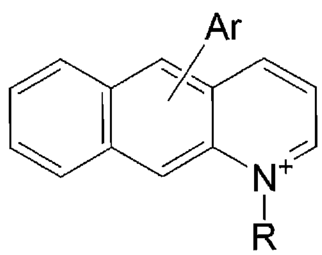
(A-2)

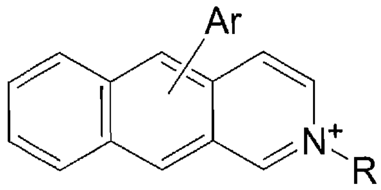

(A－3)

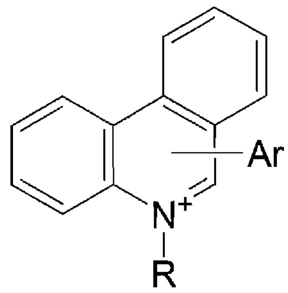

(A－4)

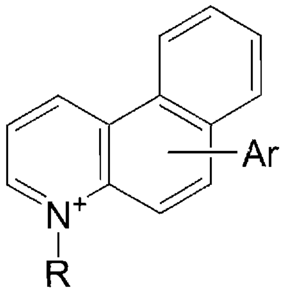

(A－5)

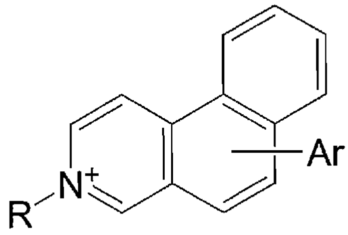

(A－6)

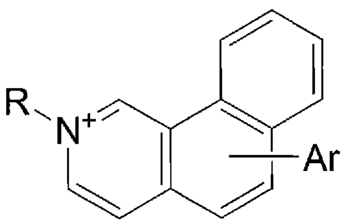

(A－7)

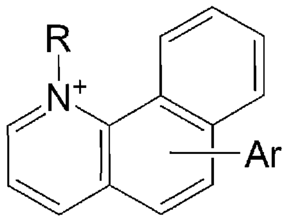

(A－8)

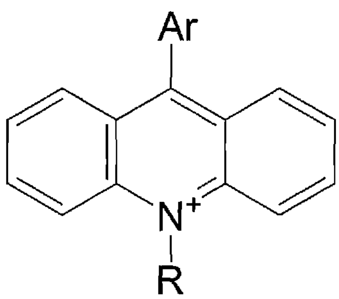

(A－9)

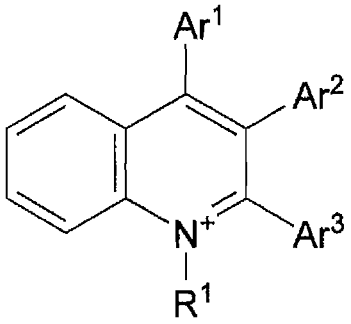

(I)

where in the formulae (A-1) to (A-8) and (A-9),
R is a hydrogen atom or an arbitrary substituent,
Ar is the electron donor group, and the number of Ars may be one or more, and when a plurality of Ars are present, they may be identical to or different from each other,
a nitrogen-containing aromatic ring that forms a nitrogen-containing aromatic cation may or may not contain at least one arbitrary substituent other than R and Ar, and
in the formula (I),
$R^1$ is a hydrogen atom or an arbitrary substituent,
$Ar^1$ to $Ar^3$ are each a hydrogen atom or the electron donor group and may be identical to or different from each other, and at least one of $Ar^1$ to $Ar^3$ is the electron donor group.

**2.** The process according to claim 1, wherein
the electron donor group of the electron donor-acceptor linked molecule, is at least one selected from the group consisting of alkyl groups, and aromatic rings, and
the aromatic ring further may have one or more substituents on the ring, and when the plurality of substituents are present, they may be identical to or different from each other, and when a plurality of electron donor groups are present, they may be identical to or different from each other.

**3.** The process according to claim 2, wherein
in the electron donor group of the electron donor-accepting linked molecule, the aromatic ring is at least one selected from the group consisting of a benzene ring, a naphthalene ring, an anthracene ring, a phenanthrene ring, a pyridine ring, a thiophene ring, and a pyrene ring.

**4.** The process according to any one of claims 1 to 3, wherein
the Brønsted acid is a hydrogen halide, and the substitution product is an aromatic halide obtained by substitution of at least one hydrogen atom on the aromatic ring of the raw material aromatic compound with a halogen.

**5.** The process according to any one of claims 1 to 4, wherein
the oxidizing agent comprises an oxygen atom, and the raw material aromatic compound comprises a methylene group ($-CH_2-$) that is covalently bound to an aromatic ring, and
in the step of generating the oxidation reaction product, the oxidation reaction product is generated by converting the methylene group ($-CH_2-$) to a carbonyl group (-CO-) through oxidation.

**6.** The process according to any one of claims 1 to 5, wherein
the oxidizing agent is oxygen or ozone.

**7.** The process according to any one of claims 1 to 6, wherein
$R^{100}$ is at least one selected from the group consisting of a hydrogen atom, alkyl groups, aryl groups, and acyl groups, and
$R^{200}$ is at least one selected from the group consisting of a hydrogen atom, alkyl groups, aryl groups, and acyl groups.

**8.** The process according to any one of claims 1 to 6, wherein $AR^{100}$ is an aryl group comprising a benzene, naphthylene, anthracene, phenanthrene, pyridine, thiophene or pyrene ring which may have one or more substituents and when

a plurality of substituents are present they may be identical or different from each other.

**9.** The process according to any one of claims 1 to 8 wherein the electron-transfer state of the electron donor-acceptor linked molecule is generated by irradiation at a wavelength from 300 to 450 nm and at a temperature from 10 to 30°C.

**10.** The process according to any one of claims 1 to 9, wherein the electron donor-acceptor linked molecule is 9-mesityl-10-methylacridinium ion represented by the following formula (A-10)

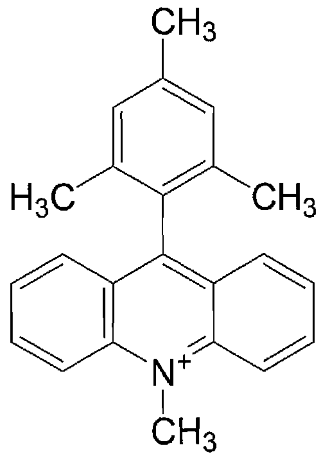

(A－１０)

**11.** The process according to any one of claims 1 to 9, wherein the quinolinium ion derivative represented by the formula (I) is a quinolinium ion derivative represented by any one of the following formulae 1 to 5

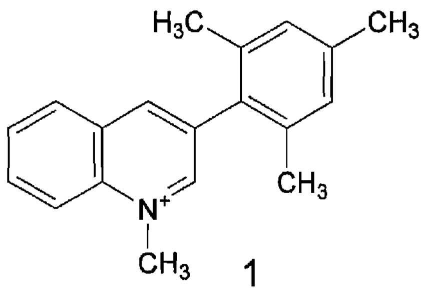

1

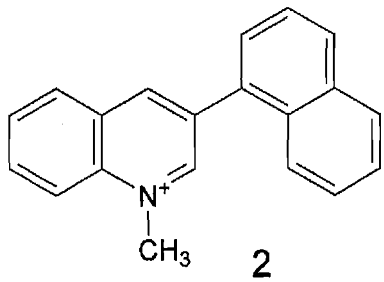

2

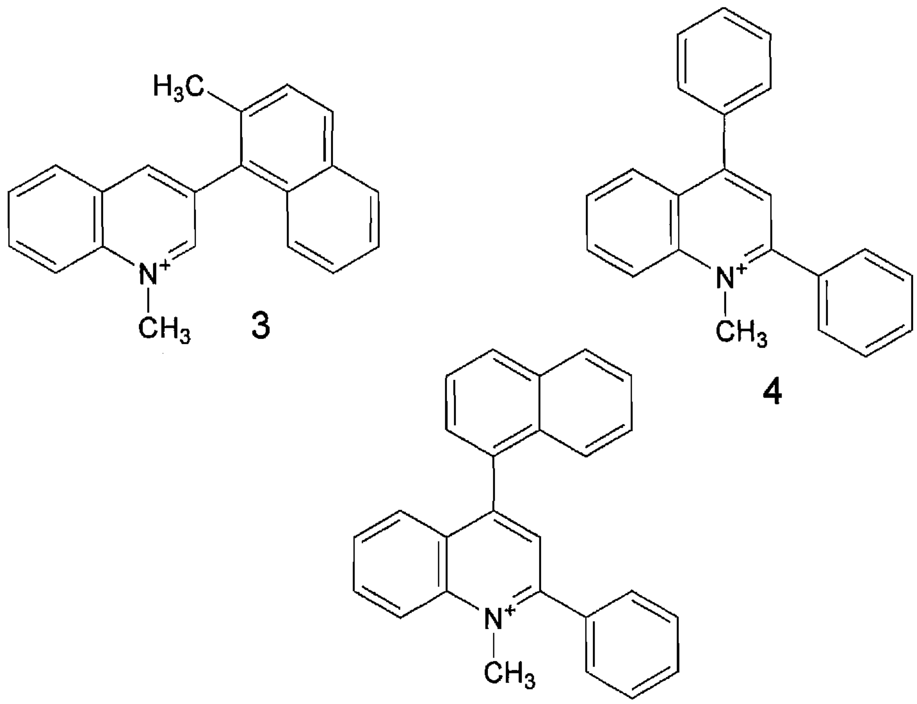

3

4

5

12. The process according to any one of claims 1 to 11, wherein a porous material further is used, and the electron donor-acceptor linked molecule forms a composite material with the porous material.

13. The process according to claim 12, wherein the porous material is at least one of aluminum-substituted mesoporous silica obtained by substitution of at least one of silicon (Si) atoms in mesoporous silica with an aluminum (Al) atom, and zeolite.

14. A process for producing hydrogen peroxide, the process comprising the step of producing an oxidation reaction product of a raw material aromatic compound by the process according to any one of claims 1 to 13,
wherein the oxidizing agent comprises oxygen ($O_2$),
a supply source of a hydrogen atom further is used, and
in the step of generating the oxidation reaction product, the oxygen binds to the hydrogen atom, thereby generating hydrogen peroxide.

**Patentansprüche**

1. Verfahren zur Herstellung eines Oxidationsreaktionsprodukts einer aromatischen Rohstoffverbindung, die einen aromatischen Benzol-, Naphthalin- oder Thiophenring umfasst, der wenigstens einen Substituenten aufweist, der aus der Gruppe ausgewählt ist, die aus $-OR^{100}$, $-NR^{200}_2$ und $-AR^{100}$ besteht, das kovalent an den Benzol-, Naphthalin- oder Thiophenring gebunden ist;
wobei $R^{100}$ ein Wasserstoffatom oder ein beliebiger Substituent ist, und wenn mehrere $R^{100}$ vorhanden sind, können sie gleich oder verschieden sein;
$R^{200}$ jeweils ein Wasserstoffatom oder ein beliebiger Substituent sind und sie gleich oder verschieden sein können; und
$AR^{100}$ eine Arylgruppe ist, und wenn mehrere $R^{100}$ vorhanden sind, können sie gleich oder verschieden sein;
wobei das Verfahren das Umsetzen der aromatischen Rohstoffverbindung mit einem Oxidationsmittel umfasst, bei dem es sich um elementaren Sauerstoff handelt; wobei
das Verfahren weiterhin ein Elektronen-Donor-Akzeptor-verknüpftes Molekül und eine Brønsted-Säure, die aus

Halogenwasserstoffen, Schwefelsäure, Salpetersäure, Sulfonsäure, Carbonsäuren und Phosphorsäure sowie Anionen davon ausgewählt ist, verwendet; und
das Verfahren die folgenden Schritte umfasst:

Erzeugen eines Elektronenübertragungszustands bei dem Elektronen-Donor-Akzeptor-verknüpften Molekül durch optische Anregung;
Umsetzen des Elektronen-Donor-Akzeptor-verknüpften Moleküls im Elektronenübertragungszustand, des Oxidationsmittels, der aromatischen Rohstoffverbindung und der Brønsted-Säure oder deren Anion, wodurch ein Oxidationsreaktionsprodukt entsteht, wobei:

das Oxidationsreaktionsprodukt ein Substitutionsprodukt umfasst, das durch Substitution von wenigstens einem Wasserstoffatom an einem aromatischen Ring der aromatischen Rohstoffverbindung durch ein Atom oder eine Atomgruppe des Anions der Brønsted-Säure erhalten wird, und
das Elektronen-Donor-Akzeptor-verknüpfte Molekül wenigstens eines ist, das aus der Gruppe ausgewählt ist, die aus stickstoffhaltigen aromatischen Kationderivaten, die durch die folgenden Formeln (A-1) bis (A-8) dargestellt werden; 9-substituierten Acridinium-Ionen, die durch die folgende Formel (A-9) dargestellt wird; Chinolinium-Ion-Derivate, die durch die folgende Formel (I) dargestellt werden, Stereoisomeren und Tautomeren davon und Salzen davon besteht:

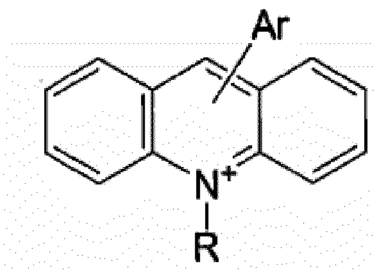

(A-1)

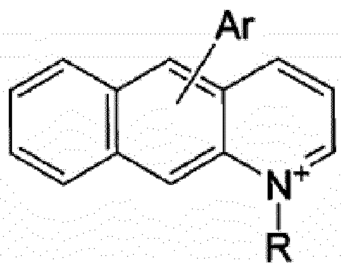

(A-2)

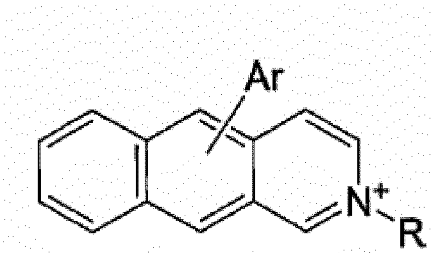

(A-3)

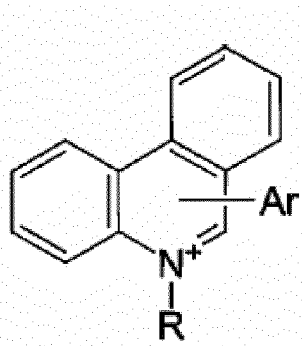

(A-4)

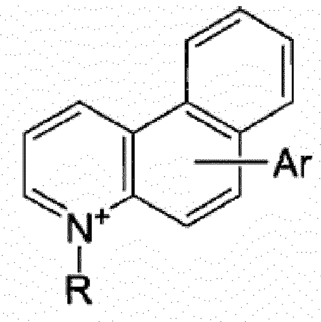

(A-5)

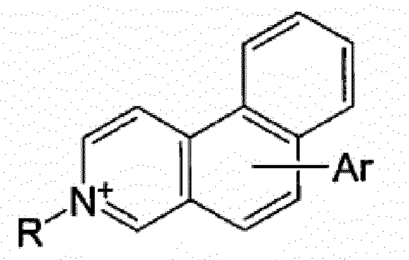

(A-6)

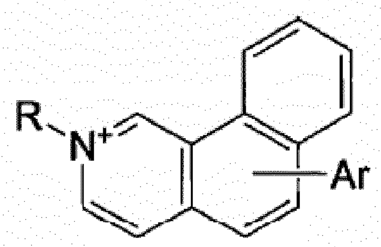

(A-7)

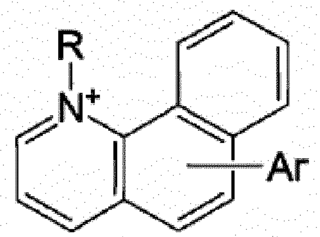

(A-8)

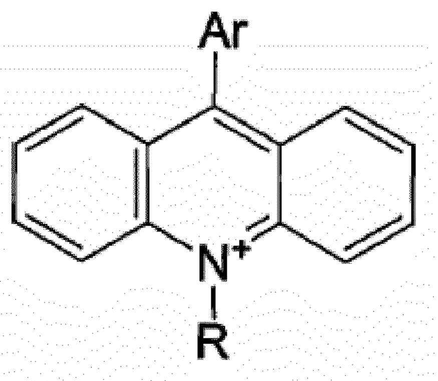

(A-9)

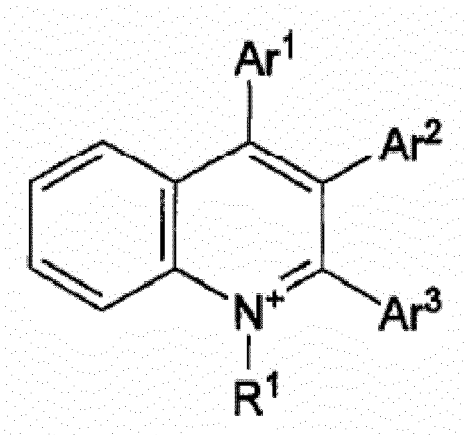

(I)

wobei in den Formeln (A-1) bis (A-8) und (A-9)
R ein Wasserstoffatom oder ein beliebiger Substituent ist;
Ar die Elektronendonorgruppe ist und die Zahl der Ar eins oder mehr betragen kann, und wenn mehrere Ar vorhanden sind, sind sie gleich oder verschieden;
ein stickstoffhaltiger aromatischer Ring, der ein stickstoffhaltiges aromatisches Kation bildet, wenigstens einen willkürlichen Substituenten, der von R und Ar verschieden ist, enthalten kann oder auch nicht; und in Formel (I)
$R^1$ ein Wasserstoffatom oder ein beliebiger Substituent ist;
$Ar^1$ bis $Ar^3$ jeweils ein Wasserstoffatom oder die Elektronendonorgruppe sind und gleich oder verschieden sein können, wobei wenigstens eines aus $Ar^1$ bis $Ar^3$ die Elektronendonorgruppe ist.

**2.** Verfahren gemäß Anspruch 1, wobei
die Elektronendonorgruppe des Elektronen-Donor-Akzeptor-verknüpften Moleküls wenigstens eine ist, die aus der Gruppe ausgewählt ist, welche aus Alkylgruppen und aromatischen Ringen besteht; und
der aromatische Ring weiterhin einen oder mehrere Substituenten am Ring aufweisen kann, und wenn die mehreren Substituenten vorhanden sind, können sie gleich oder verschieden sein, und wenn mehrere Elektronendonorgruppen vorhanden sind, können sie gleich oder verschieden sein.

**3.** Verfahren gemäß Anspruch 2, wobei
in der Elektronendonorgruppe des Elektronen-Donor-Akzeptor-verknüpften Moleküls der aromatische Ring wenigstens einer ist, der aus der Gruppe ausgewählt ist, die aus einem Benzolring, einem Naphthalinring, einem Anthracenring, einem Phenanthrenring, einem Pyridinring, einem Thiophenring und einem Pyrenring besteht.

**4.** Verfahren gemäß einem der Ansprüche 1 bis 3, wobei
die Brønsted-Säure ein Halogenwasserstoff ist und das Substitutionsprodukt ein aromatisches Halogenid ist, das durch Substitution wenigstens eines Wasserstoffatoms an dem aromatischen Ring der aromatischen Rohstoffverbindung durch ein Halogen erhalten wird.

**5.** Verfahren gemäß einem der Ansprüche 1 bis 4, wobei
das Oxidationsmittel ein Sauerstoffatom umfasst und die aromatische Rohstoffverbindung eine Methylengruppe ($-CH_2-$) umfasst, die kovalent an den aromatischen Ring gebunden ist; und
in dem Schritt des Erzeugens des Oxidationsreaktionsprodukts das Oxidationsreaktionsprodukt dadurch erzeugt wird, dass man die Methylengruppe ($-CH_2-$) durch Oxidation in eine Carbonylgruppe (-CO-) umwandelt.

**6.** Verfahren gemäß einem der Ansprüche 1 bis 5, wobei es sich bei dem Oxidationsmittel um Sauerstoff oder Ozon handelt.

**7.** Verfahren gemäß einem der Ansprüche 1 bis 6, wobei
$R^{100}$ wenigstens eines ist, das aus der Gruppe ausgewählt ist, die aus einem Wasserstoffatom, Alkylgruppen, Arylgruppen und Acylgruppen besteht; und

$R^{200}$ wenigstens eines ist, das aus der Gruppe ausgewählt ist, die aus einem Wasserstoffatom, Alkylgruppen, Arylgruppen und Acylgruppen besteht.

**8.** Verfahren gemäß einem der Ansprüche 1 bis 6, wobei $AR^{100}$ eine Arylgruppe ist, die einen Benzol-, Naphthalin-, Anthracen-, Phenanthren-, Pyridin-, Thiophen- oder Pyrenring, der einen oder mehrere Substituenten aufweisen kann, umfasst, und wenn mehrere Substituenten vorhanden sind, können sie gleich oder verschieden sein.

**9.** Verfahren gemäß einem der Ansprüche 1 bis 8, wobei der Elektronenübertragungszustand des Elektronen-Donor-Akzeptorverknüpften Moleküls durch Bestrahlung mit einer Wellenlänge von 300 bis 450 nm und bei einer Temperatur von 10 bis 30 °C erzeugt wird.

**10.** Verfahren gemäß einem der Ansprüche 1 bis 9, wobei
es sich bei dem Elektronen-Donor-Akzeptor-verknüpften Molekül um ein 9-Mesityl-10-methylacridinium-Ion handelt, das durch die folgende Formel (A-10) dargestellt wird:

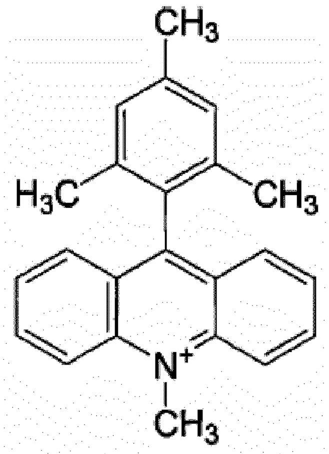

(A−10) .

**11.** Verfahren gemäß einem der Ansprüche 1 bis 9, wobei das durch die Formel (I) dargestellte Chinolinium-Ion-Derivat ein Chinolinium-Ion-Derivat ist, das durch eine der folgenden Formeln 1 bis 5 dargestellt wird:

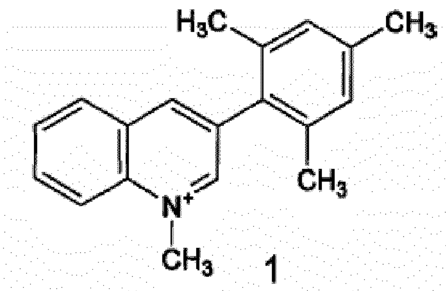

1

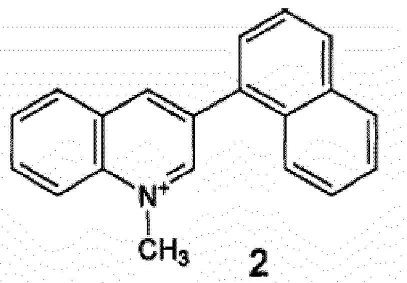

2

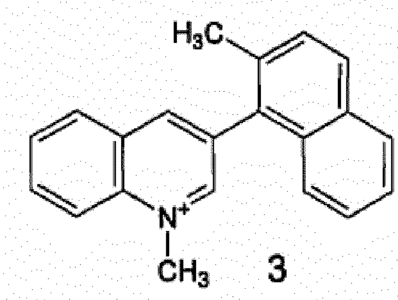

3

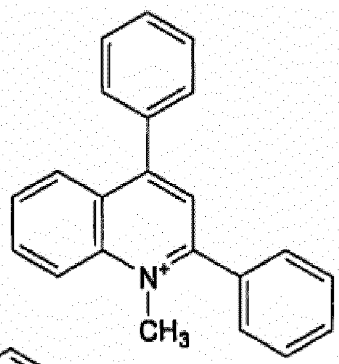

4

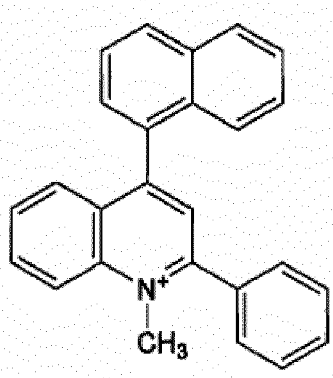

5

**12.** Verfahren gemäß einem der Ansprüche 1 bis 11, wobei weiterhin ein poröses Material verwendet wird und das Elektronen-Donor-Akzeptor-verknüpfte Molekül mit dem porösen Material einen Verbundstoff bildet.

**13.** Verfahren gemäß Anspruch 12, wobei das poröse Material wenigstens eines aus Aluminium-substituiertem mesoporösem Siliciumdioxid, das durch Substitution wenigstens eines Siliciumatoms (Si) in mesoporösem Siliciumdioxid durch ein Aluminiumatom (Al) erhalten wird, und Zeolith ist.

**14.** Verfahren zur Herstellung von Wasserstoffperoxid, wobei das Verfahren den folgenden Schritt umfasst:

Herstellen eines Oxidationsreaktionsprodukts einer aromatischen Rohstoffverbindung nach dem Verfahren gemäß einem der Ansprüche 1 bis 13;
wobei das Oxidationsmittel Sauerstoff ($O_2$) umfasst;
weiterhin eine Quelle für Wasserstoffatome verwendet wird; und
in dem Schritt des Erzeugens des Oxidationsreaktionsprodukts der Sauerstoff an das Wasserstoffatom bindet, wodurch Wasserstoffperoxid entsteht.

## Revendications

**1.** Procédé de production d'un produit de réaction d'oxydation d'un composé aromatique de départ comprenant un noyau aromatique de type benzène, naphtalène ou thiophène ayant au moins un substituant choisi dans le groupe constitué de $-OR^{100}$, $-NR^{200}_2$ et $-AR^{100}$ qui est lié par liaison covalente au noyau benzénique, naphtalénique ou thiophénique,
$R^{100}$ est un atome d'hydrogène ou un substituant arbitraire, et quand plusieurs $R^{100}$ sont présents, ils peuvent être identiques les uns aux autres ou différents les uns des autres,
les $R^{200}$ sont chacun un atome d'hydrogène ou un substituant arbitraire, et ils peuvent être identiques les uns aux autres ou différents les uns des autres, et
$AR^{100}$ est un groupe aryle, et quand plusieurs $AR^{100}$ sont présents, ils peuvent être identiques les uns aux autres ou différents les uns des autres,
ledit procédé comprenant la réaction du composé aromatique de départ avec un agent oxydant qui est l'oxygène sous forme de substance élémentaire, dans lequel

le procédé utilise en outre une molécule liée à un donneur-accepteur d'électrons, et un acide de Brønsted choisi parmi les halogénures d'hydrogène, l'acide sulfurique, l'acide nitrique, l'acide sulfonique, les acides carboxyliques et l'acide phosphorique et leurs anions,
et le procédé comprend les étapes suivantes :

la génération d'un état de transfert d'électrons de la molécule liée à un donneur-accepteur d'électrons par photoexcitation ;
la réaction de la molécule liée à un donneur-accepteur d'électrons dans l'état de transfert d'électrons, de l'agent d'oxydation, du composé aromatique de départ et de l'acide de Brønsted ou de son anion pour ainsi générer un produit de réaction d'oxydation, dans laquelle :

le produit de réaction d'oxydation comprend un produit de substitution obtenu par la substitution d'au moins un atome d'hydrogène sur un noyau aromatique du composé aromatique de départ par un atome ou un groupe atomique de l'anion de l'acide de Brønsted, et
la molécule liée à un donneur-accepteur d'électrons est au moins une molécule choisie dans le groupe constitué : des dérivés aromatiques cationiques azotés représentés par les formules (A-1) à (A-8) suivantes ; des ions acridinium substitués en position 9 représentés par la formule (A-9) suivante ; des dérivés d'ion quinolinium représentés par la formule (I) suivante ; de leurs stéréoisomères et tautomères ; et de leurs sels :

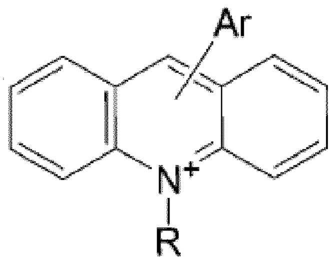
(A−1)

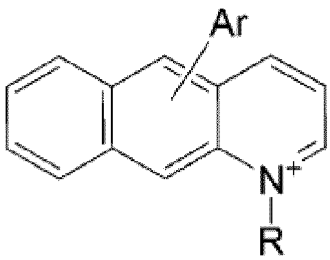
(A−2)

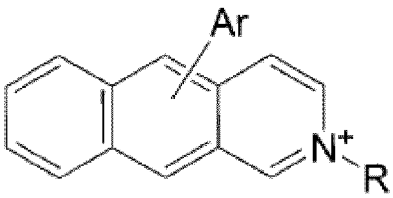
(A−3)

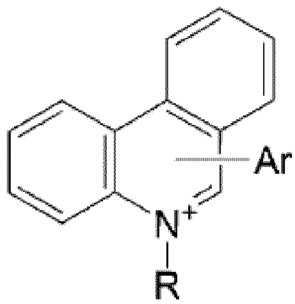
(A−4)

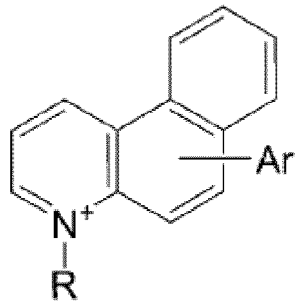

(A－5)

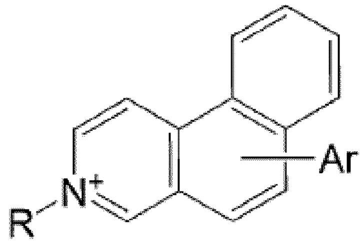

(A－6)

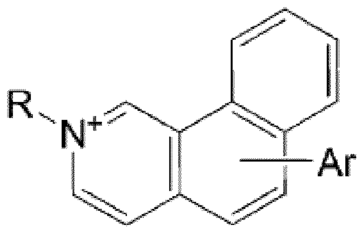

(A－7)

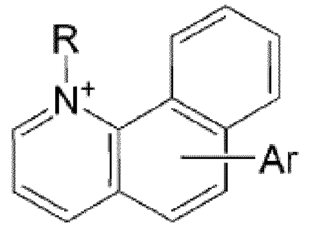

(A－8)

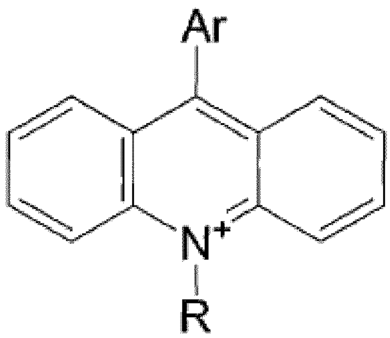

(A－9)

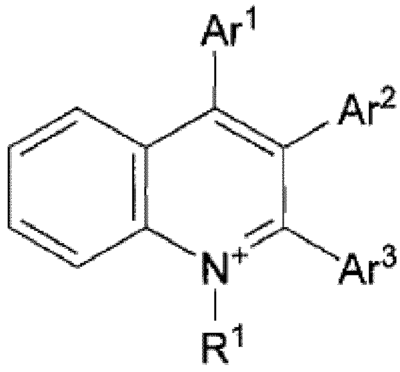

(I)

où dans les formules (A-1) à (A-8) et (A-9),
R est un atome d'hydrogène ou un substituant arbitraire,
Ar est le groupe donneur d'électrons, et le nombre d'Ar peut être un ou plus, et quand plusieurs Ar sont présents, ils peuvent être identiques les uns aux autres ou différents les uns des autres,
un noyau aromatique azoté qui forme un cation aromatique azoté peut ou non contenir au moins un substituant arbitraire différent de R et d'Ar, et
dans la formule (I)
$R^1$ est un atome d'hydrogène ou un substituant arbitraire,
$Ar^1$ à $Ar^3$ sont chacun un atome d'hydrogène ou le groupe donneur d'électrons et peuvent être identiques les uns aux autres ou différents les uns des autres, et au moins un parmi $Ar^1$ à $Ar^3$ est le groupe donneur d'électrons.

**2.** Procédé selon la revendication 1, dans lequel le groupe donneur d'électrons de la molécule liée à un donneur-accepteur d'électrons est au moins un choisi dans le groupe constitué des groupes alkyle et des noyaux aromatiques, et
le noyau aromatique peut avoir en outre un ou plusieurs substituants sur le noyau, et quand plusieurs substituants sont présents, ils peuvent être identiques les uns aux autres ou différents les uns des autres, et quand plusieurs groupes donneurs d'électrons sont présents, ils peuvent être identiques les uns aux autres ou différents les uns des autres.

**3.** Procédé selon la revendication 2, dans lequel
dans le groupe donneur d'électrons de la molécule liée à un donneur-accepteur d'électrons, le noyau aromatique est au moins un choisi dans le groupe constitué d'un noyau benzénique, d'un noyau naphtalénique, d'un noyau anthracénique, d'un noyau phénanthrénique, d'un noyau pyridinique, d'un noyau thiophénique et d'un noyau pyrénique.

**4.** Procédé selon l'une quelconque des revendications 1 à 3, dans lequel
l'acide de Brønsted est un halogénure d'hydrogène, et le produit de substitution est un halogénure aromatique obtenu par la substitution d'au moins un atome d'hydrogène sur le noyau aromatique du composé aromatique de départ par un halogène.

**5.** Procédé selon l'une quelconque des revendications 1 à 4, dans lequel
l'agent d'oxydation comprend un atome d'oxygène et le composé aromatique de départ comprend un groupe méthylène ($-CH_2-$) qui est lié par liaison covalente à un noyau aromatique, et
dans l'étape de génération du produit de réaction d'oxydation, le produit de réaction d'oxydation est généré par la conversion du groupe méthylène ($-CH_2-$) en groupe carbonyle (-CO-) par oxydation.

**6.** Procédé selon l'une quelconque des revendications 1 à 5, dans lequel
l'agent d'oxydation est l'oxygène ou l'ozone.

**7.** Procédé selon l'une quelconque des revendications 1 à 6, dans lequel
$R^{100}$ est au moins un choisi dans le groupe constitué d'un atome d'hydrogène, des groupes alkyle, des groupes

aryle et des groupes acyle, et
$R^{200}$ est au moins un choisi dans le groupe constitué d'un atome d'hydrogène, des groupes alkyle, des groupes aryle et des groupes acyle.

**8.** Procédé selon l'une quelconque des revendications 1 à 6, dans lequel $AR^{100}$ est un groupe aryle comprenant un noyau benzénique, naphtalénique, anthracénique, phénanthrénique, pyridinique, thiophénique et pyrénique qui peut avoir un ou plusieurs substituants et, quand plusieurs substituants sont présents, ils peuvent être identiques les uns aux autres ou différents les uns des autres.

**9.** Procédé selon l'une quelconque des revendications 1 à 8, dans lequel l'état de transfert d'électrons de la molécule liée à un donneur-accepteur d'électrons est généré par irradiation à une longueur d'onde de 300 nm à 450 nm et à une température de 10 °C à 30 °C.

**10.** Procédé selon l'une quelconque des revendications 1 à 9, dans lequel
la molécule liée à un donneur-accepteur d'électrons est l'ion 9-mésityl-10-méthylacridinium représenté par la formule (A-10) suivante

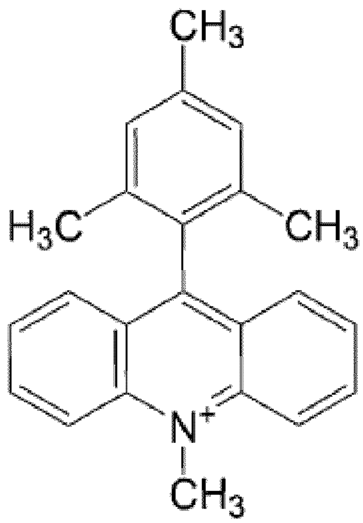

(A-10)

**11.** Procédé selon l'une quelconque des revendications 1 à 9, dans lequel le dérivé d'ion quinolinium représenté par la formule (I) est un dérivé d'ions quinolinium représenté par l'une quelconque des formules 1 à 5 suivantes

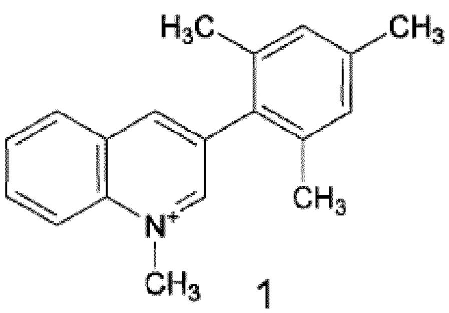

1

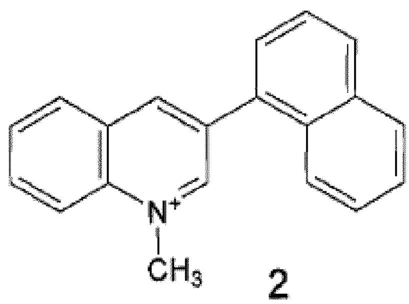

2

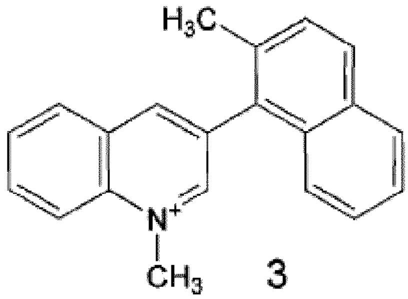

3

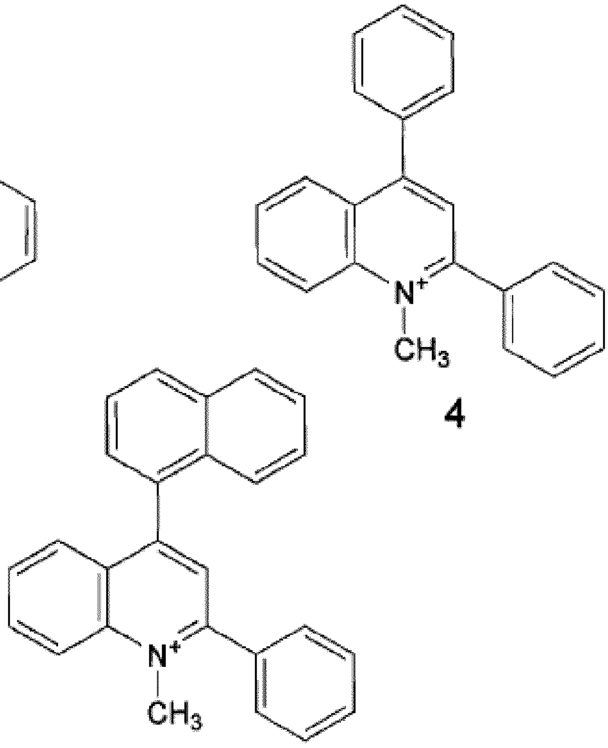

4

5

**12.** Procédé selon l'une quelconque des revendications 1 à 11, dans lequel un matériau poreux est également utilisé, et la molécule liée à un donneur-accepteur d'électrons forme un matériau composite avec le matériau poreux.

**13.** Procédé selon la revendication 12, dans lequel le matériau poreux est au moins un parmi une silice mésoporeuse substituée avec de l'aluminium obtenue par la substitution d'au moins un atome de silicium (Si) dans la silice mésoporeuse par un atome d'aluminium (Al), et une zéolithe.

**14.** Procédé de production de peroxyde d'hydrogène, le procédé comprenant l'étape suivante :

la production d'un produit de réaction d'oxydation d'un composé aromatique de départ par le procédé selon l'une quelconque des revendications 1 à 13,
dans lequel l'agent d'oxydation comprend de l'oxygène ($O_2$),
une source d'alimentation en atome d'hydrogène est également utilisée, et
dans l'étape de génération du produit de réaction d'oxydation, l'oxygène se lie à l'atome d'hydrogène pour ainsi générer du peroxyde d'hydrogène.

Scheme 1

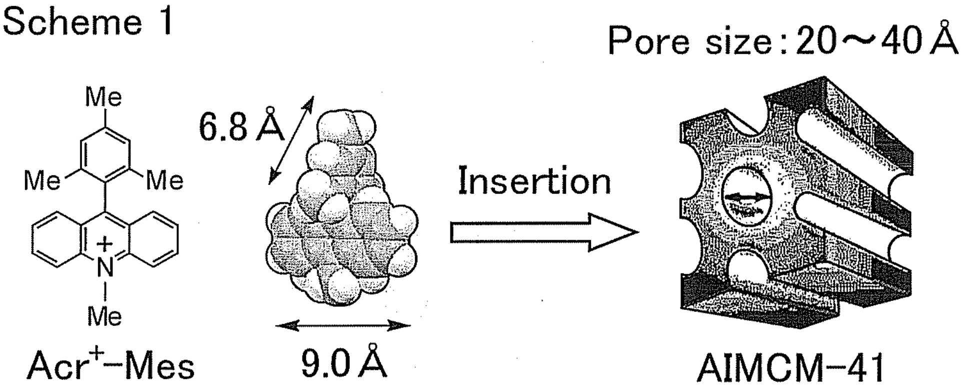


$Acr^+$-Mes

FIG. 1

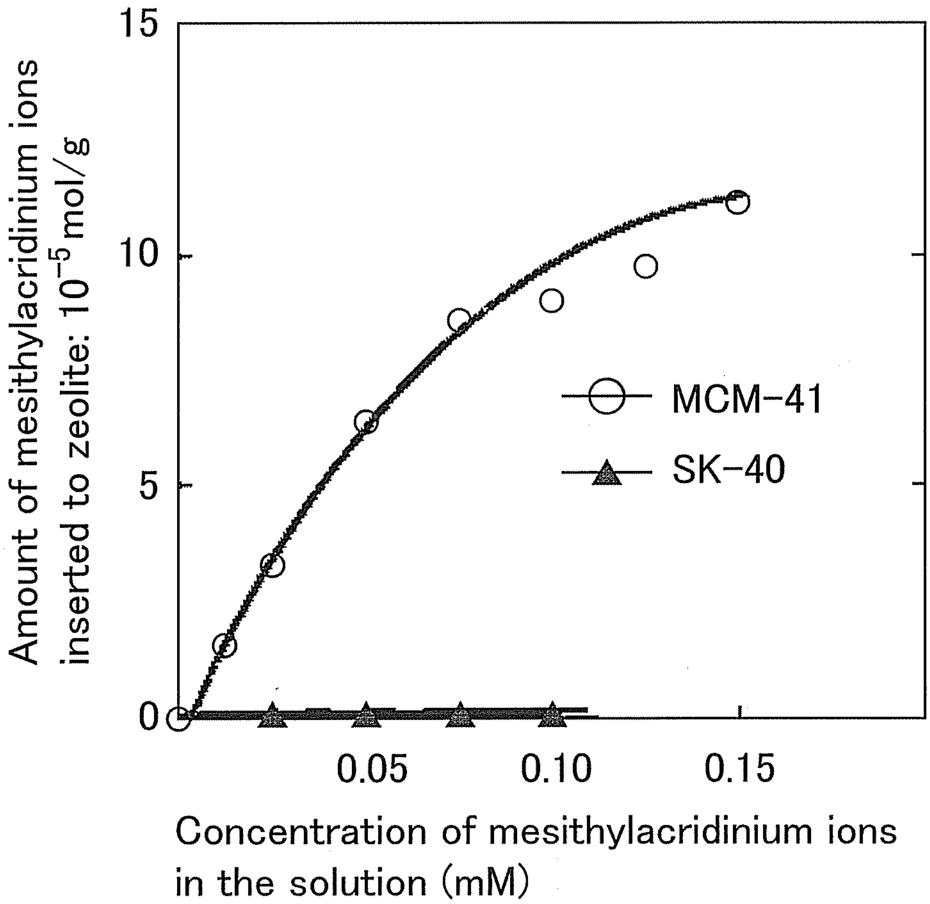


FIG. 2

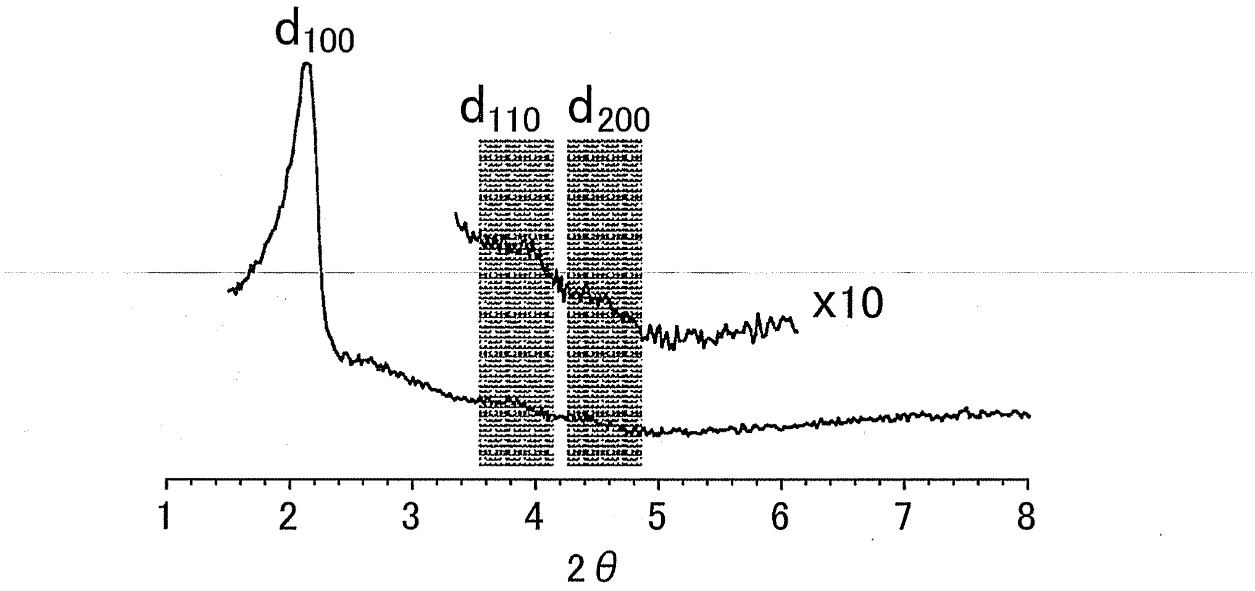

d100
d110
d200
x10
1
2
3
4
5
6
7
8
2θ


FIG. 3

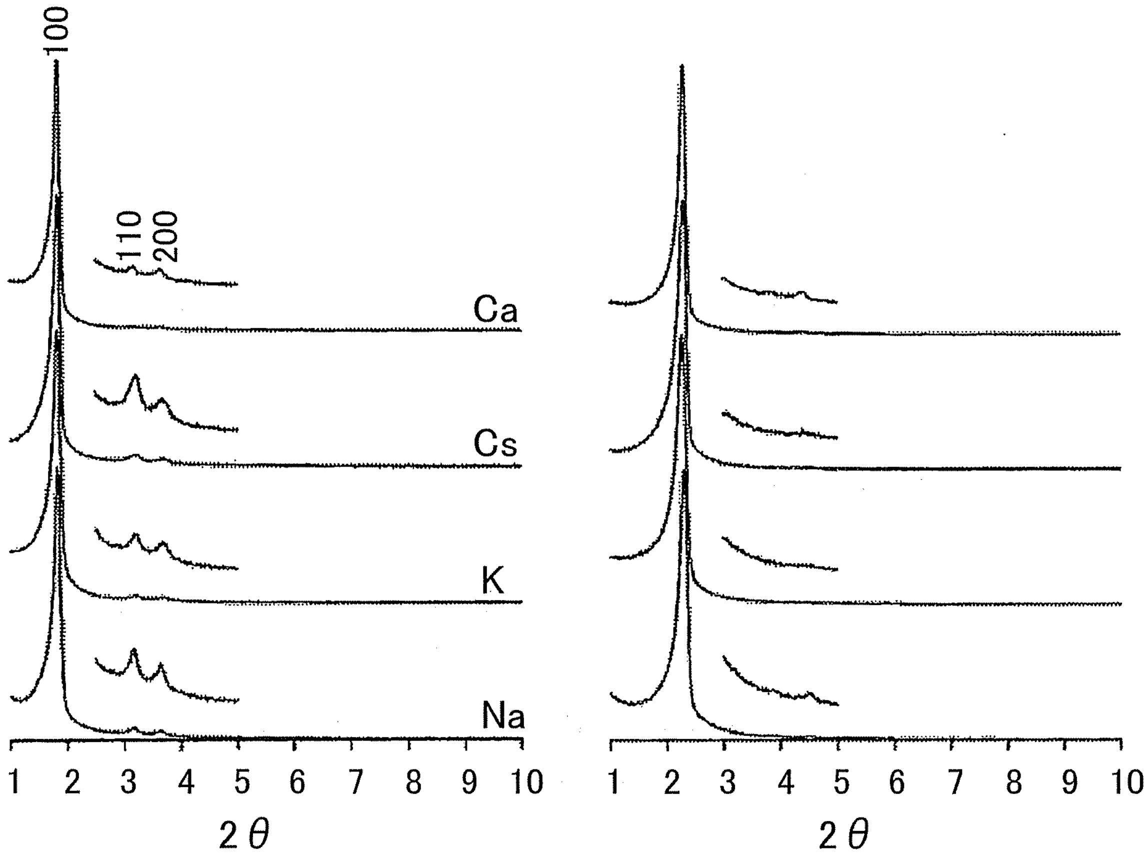

100
110
200
Ca
Cs
K
Na
1
2
3
4
5
6
7
8
9
10
2θ
2θ


FIG. 4

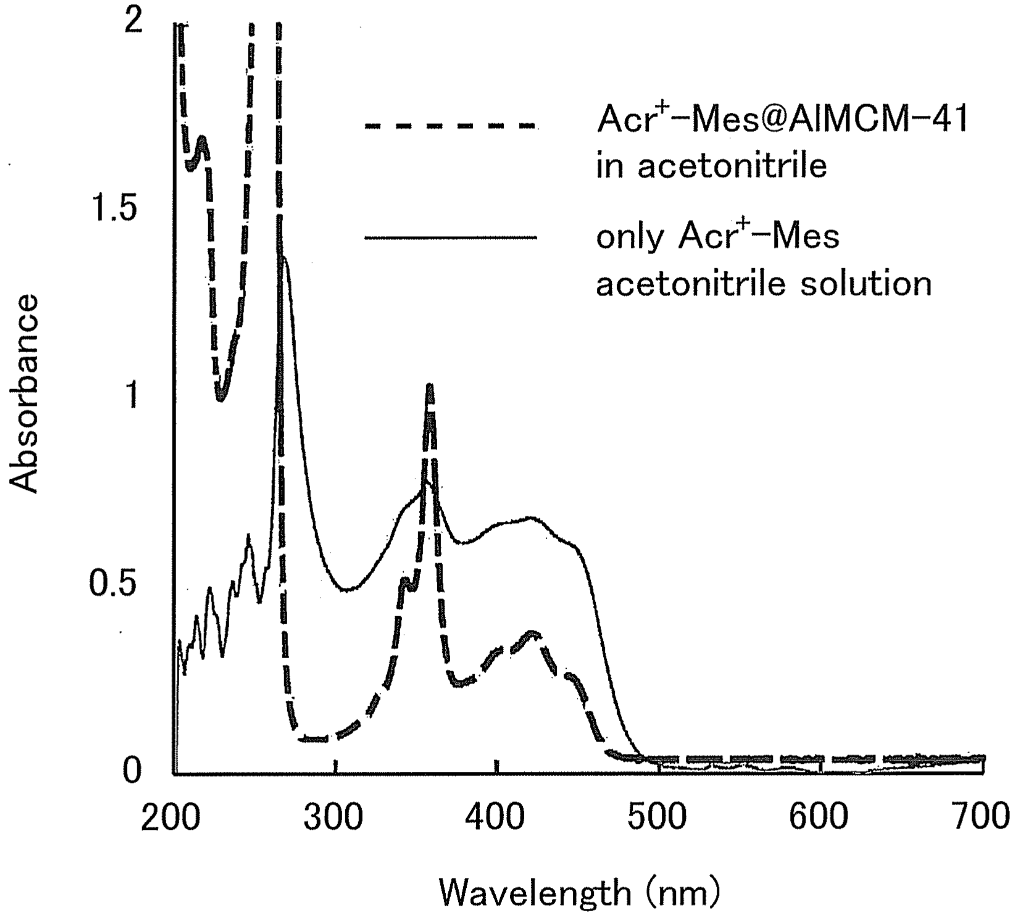
2
1.5
1
0.5
0
Absorbance
200
300
400
500
600
700
Wavelength (nm)
Acr+−Mes@AlMCM−41
in acetonitrile
only Acr+−Mes
acetonitrile solution

FIG. 5

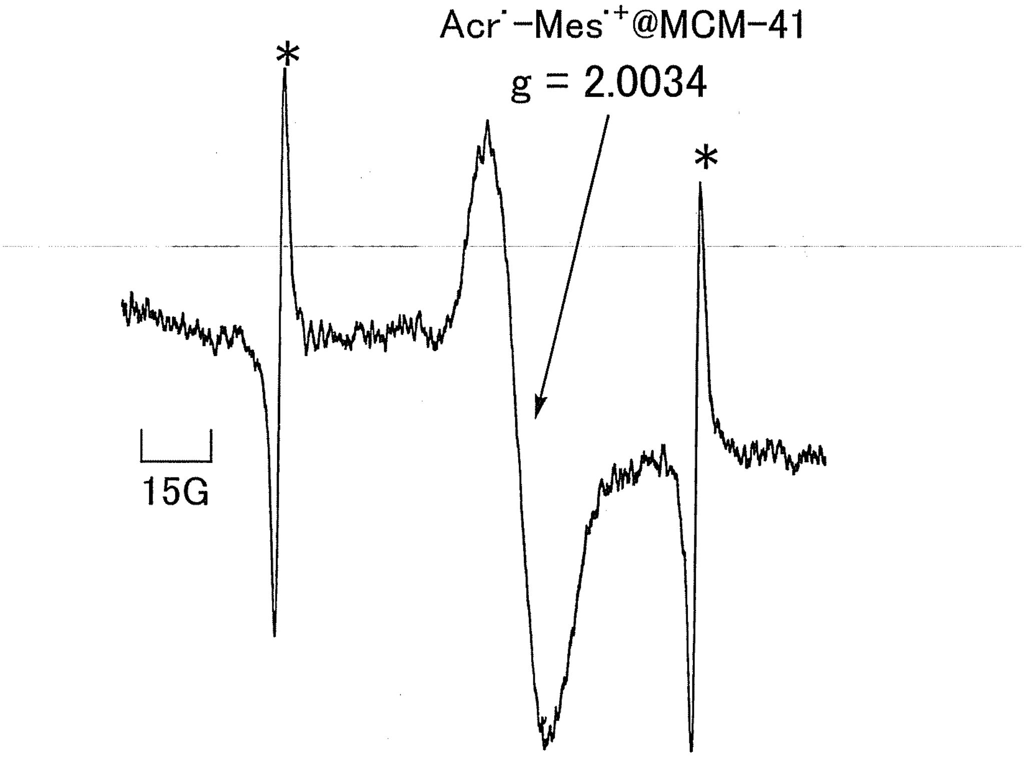


FIG. 6

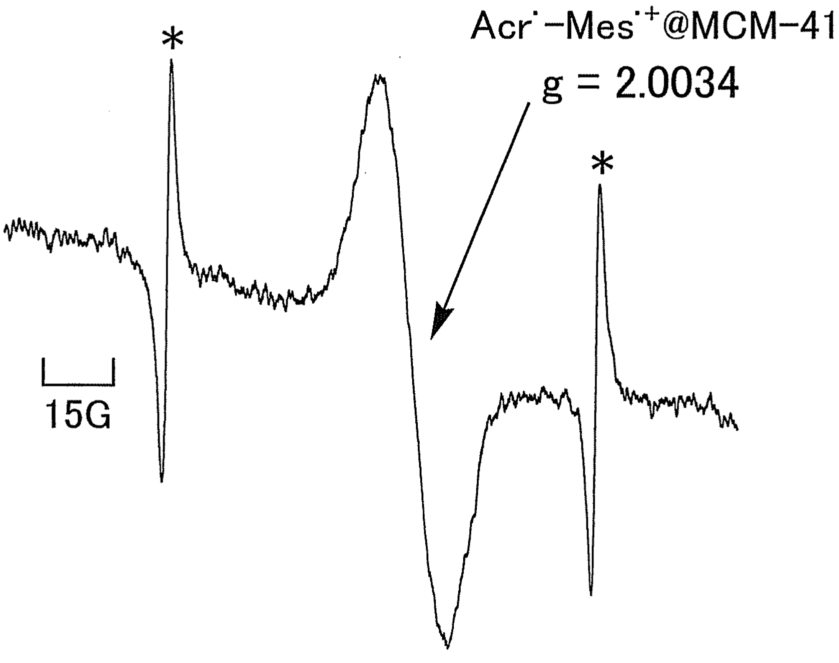


FIG. 7

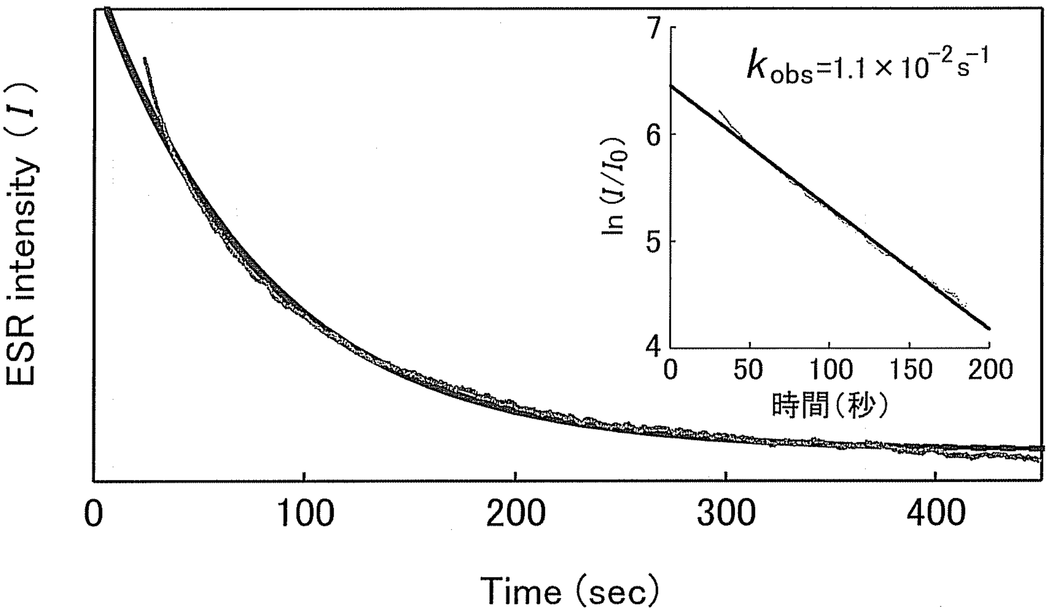
ESR intensity (I)
Time (sec)
0
100
200
300
400
$k_{obs}$=1.1×10$^{-2}$s$^{-1}$
ln (I/I0)
7
6
5
4
0
50
100
150
200
時間(秒)

FIG. 8

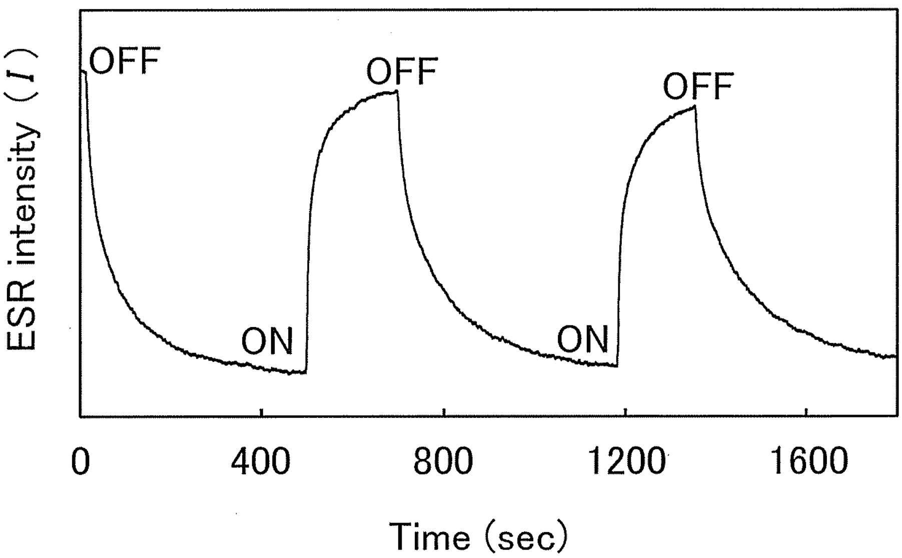
ESR intensity (I)
OFF
ON
OFF
ON
OFF
0
400
800
1200
1600
Time (sec)

FIG. 9

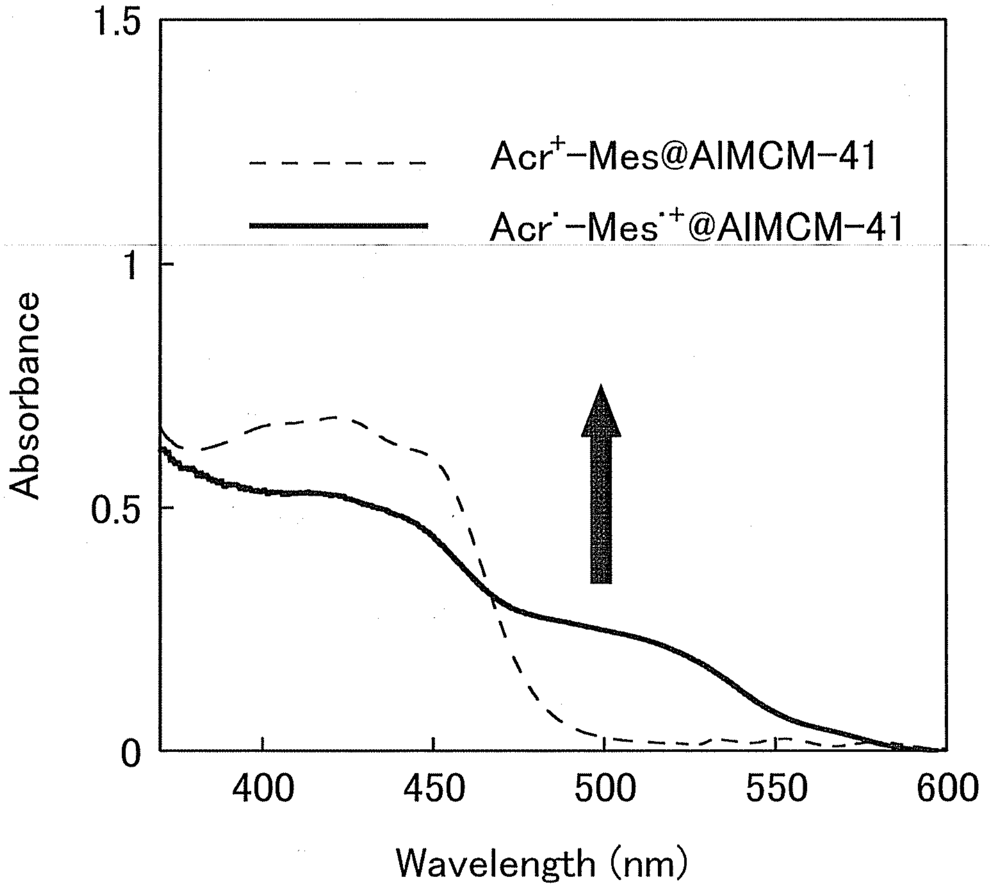
1.5
1
0.5
0
Absorbance
Acr$^+$–Mes@AlMCM–41
Acr$^\cdot$–Mes$^{\cdot+}$@AlMCM–41
400
450
500
550
600
Wavelength (nm)

FIG. 10

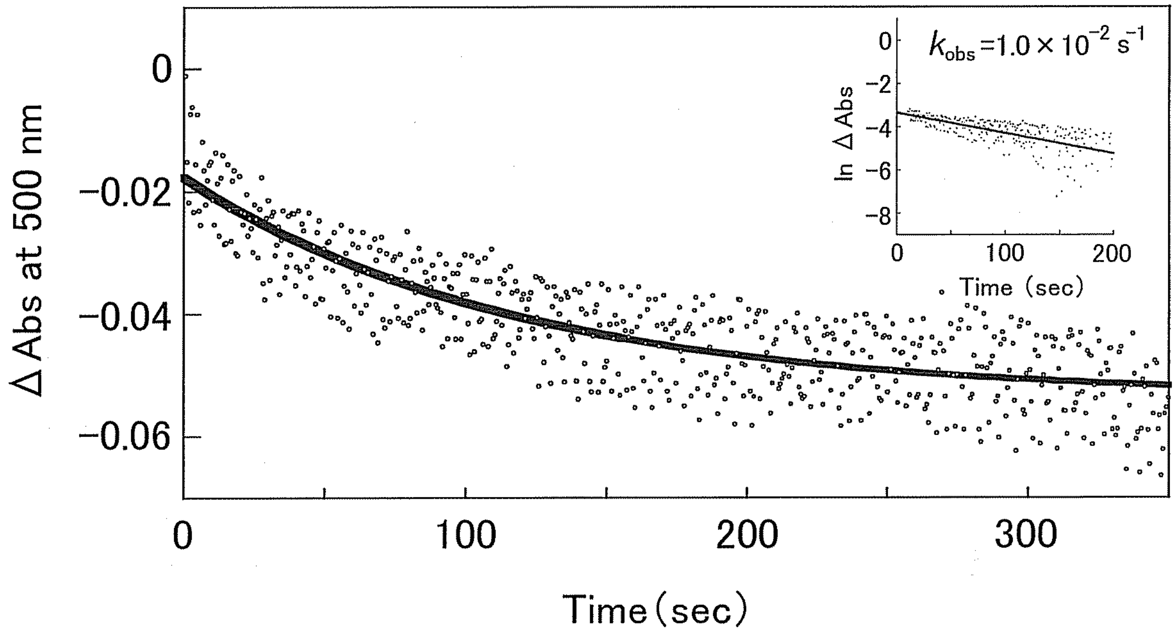


FIG. 11A

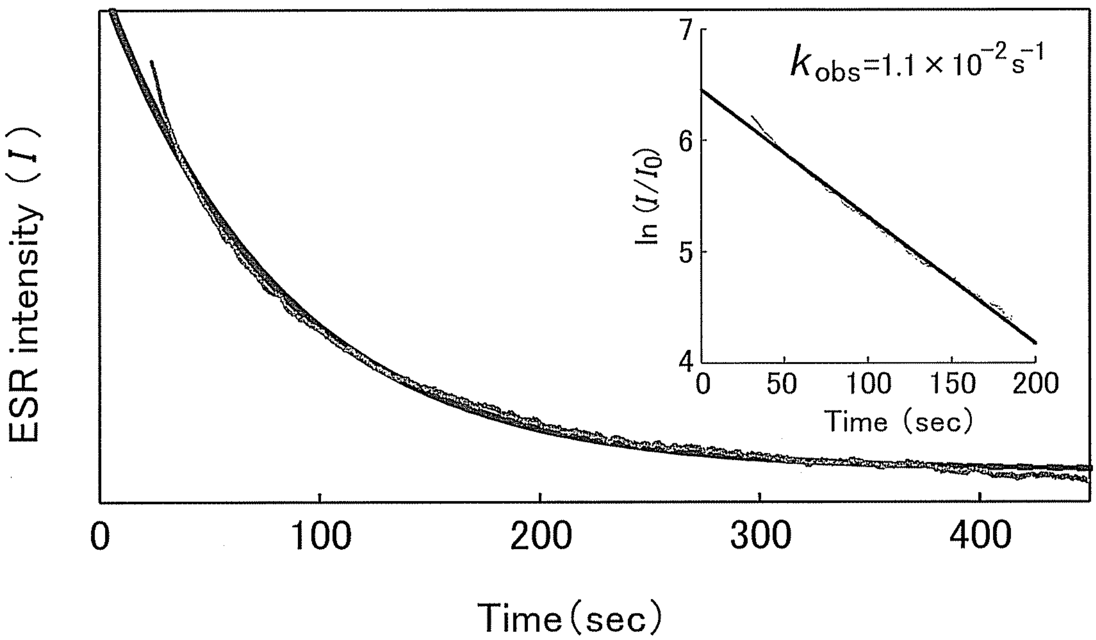


FIG. 11B

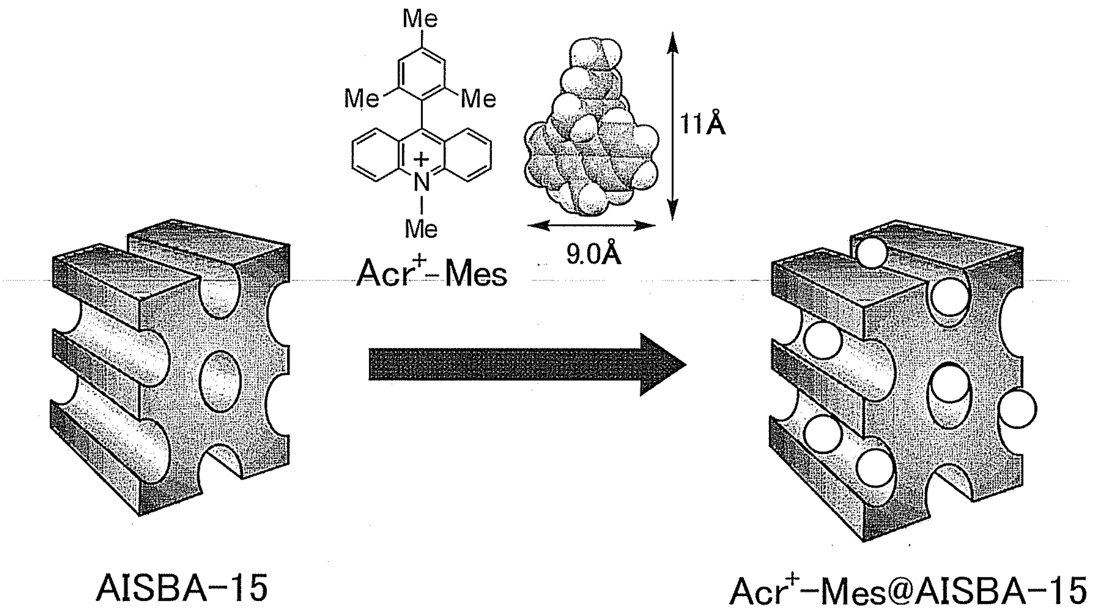


FIG. 12

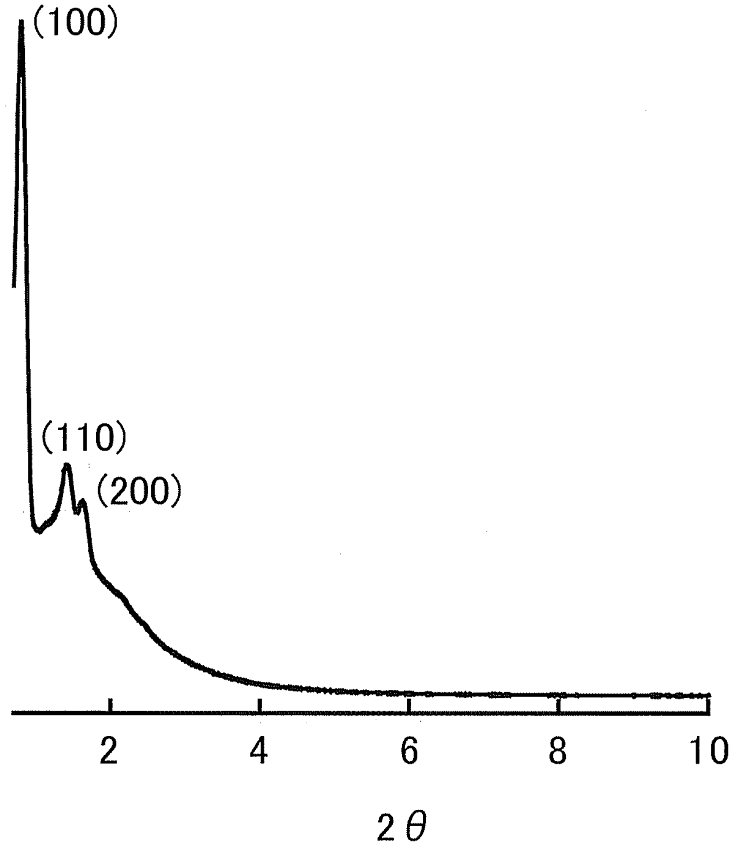


FIG. 13

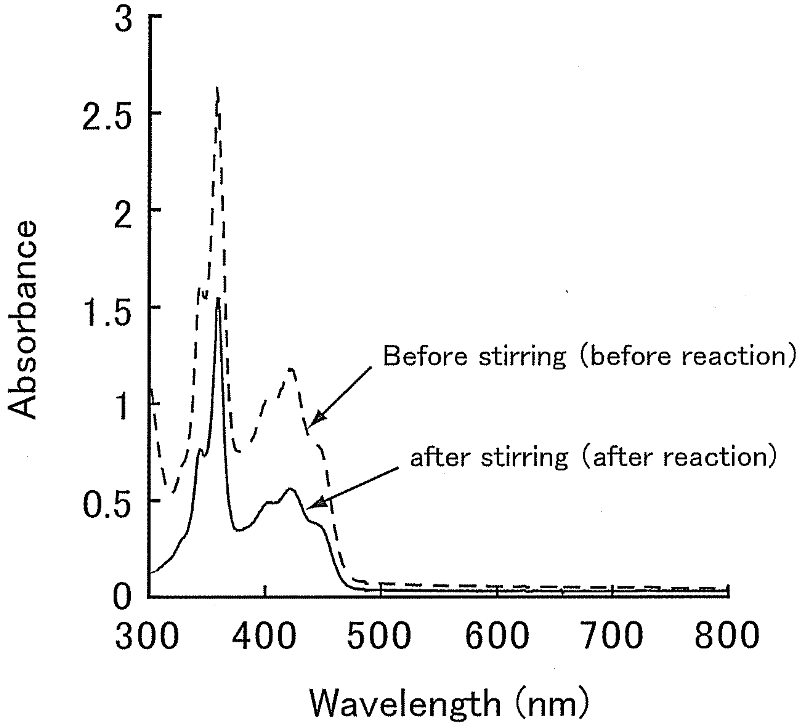
Absorbance
3
2.5
2
1.5
1
0.5
0
300
400
500
600
700
800
Wavelength (nm)
Before stirring (before reaction)
after stirring (after reaction)

FIG. 14

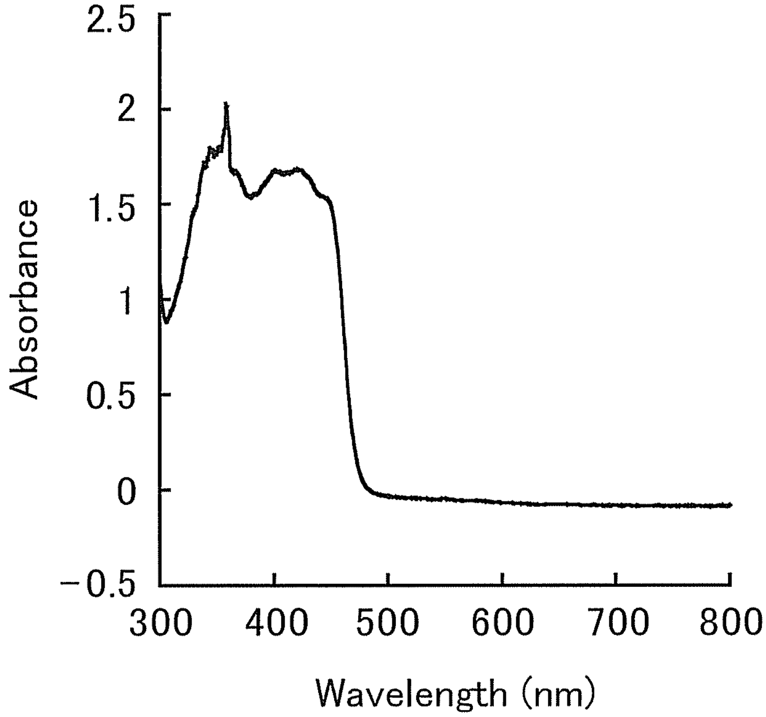
Absorbance
2.5
2
1.5
1
0.5
0
−0.5
300
400
500
600
700
800
Wavelength (nm)

FIG. 15

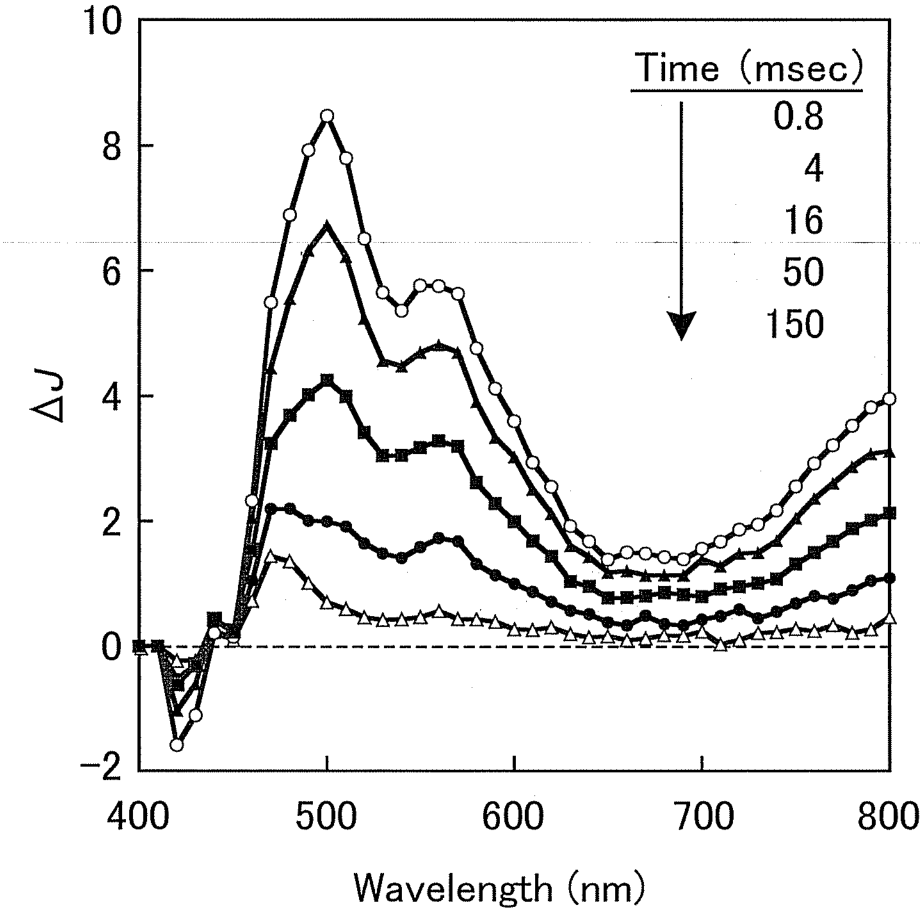
10
8
6
4
2
0
−2
ΔJ
400
500
600
700
800
Wavelength (nm)
Time (msec)
0.8
4
16
50
150

FIG. 16

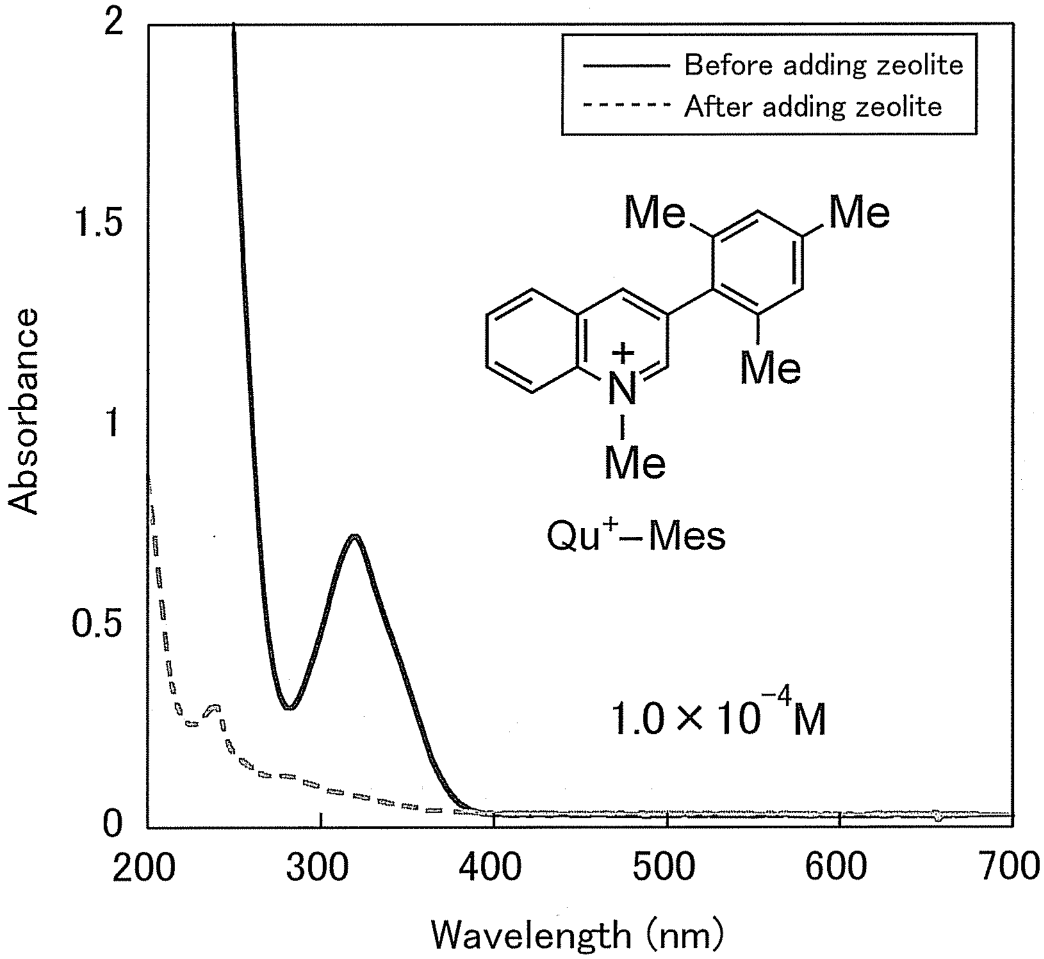
Before adding zeolite
After adding zeolite
Qu+–Mes
1.0 × 10−4 M
Absorbance
Wavelength (nm)
0
0.5
1
1.5
2
200
300
400
500
600
700

FIG. 17

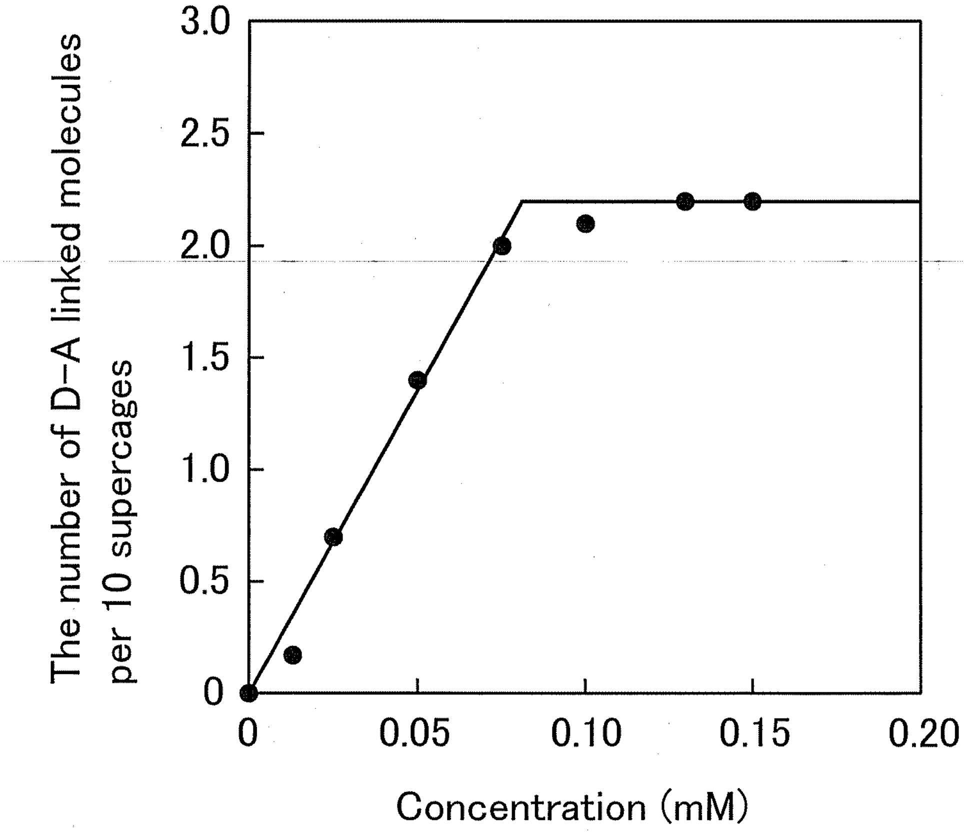

3.0
2.5
2.0
1.5
1.0
0.5
0
The number of D−A linked molecules per 10 supercages
0
0.05
0.10
0.15
0.20
Concentration (mM)


FIG. 18

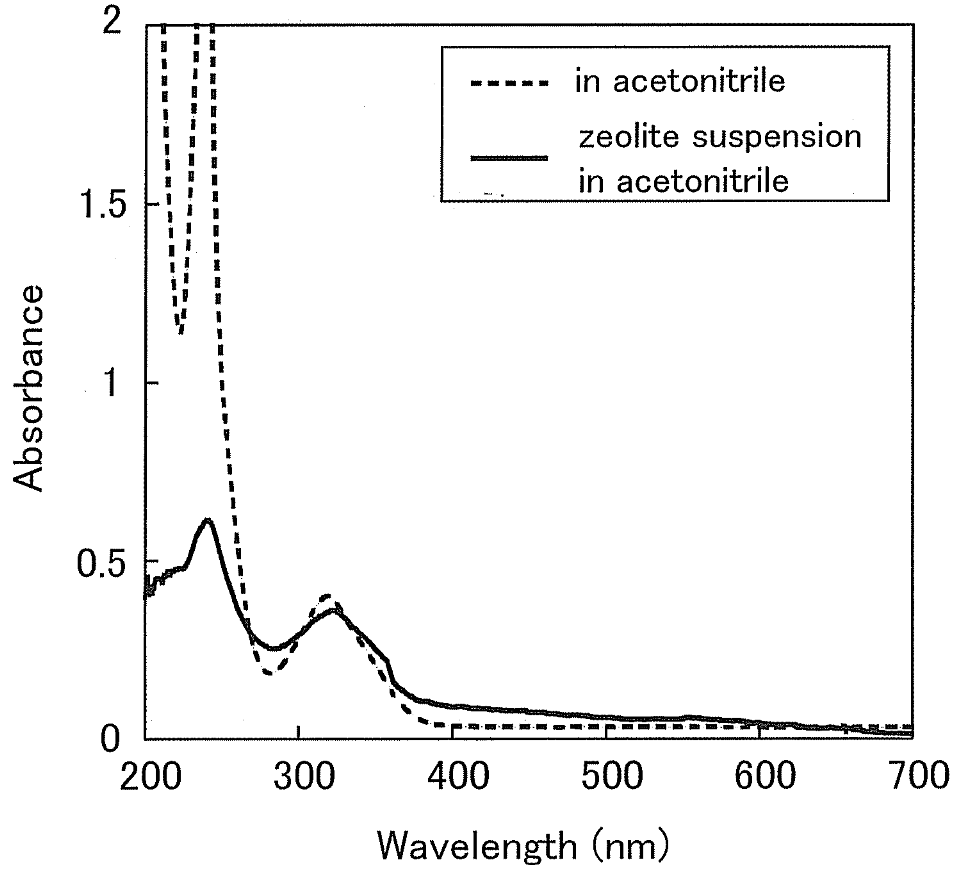
2
1.5
1
0.5
0
Absorbance
200
300
400
500
600
700
Wavelength (nm)
in acetonitrile
zeolite suspension
in acetonitrile

FIG. 19

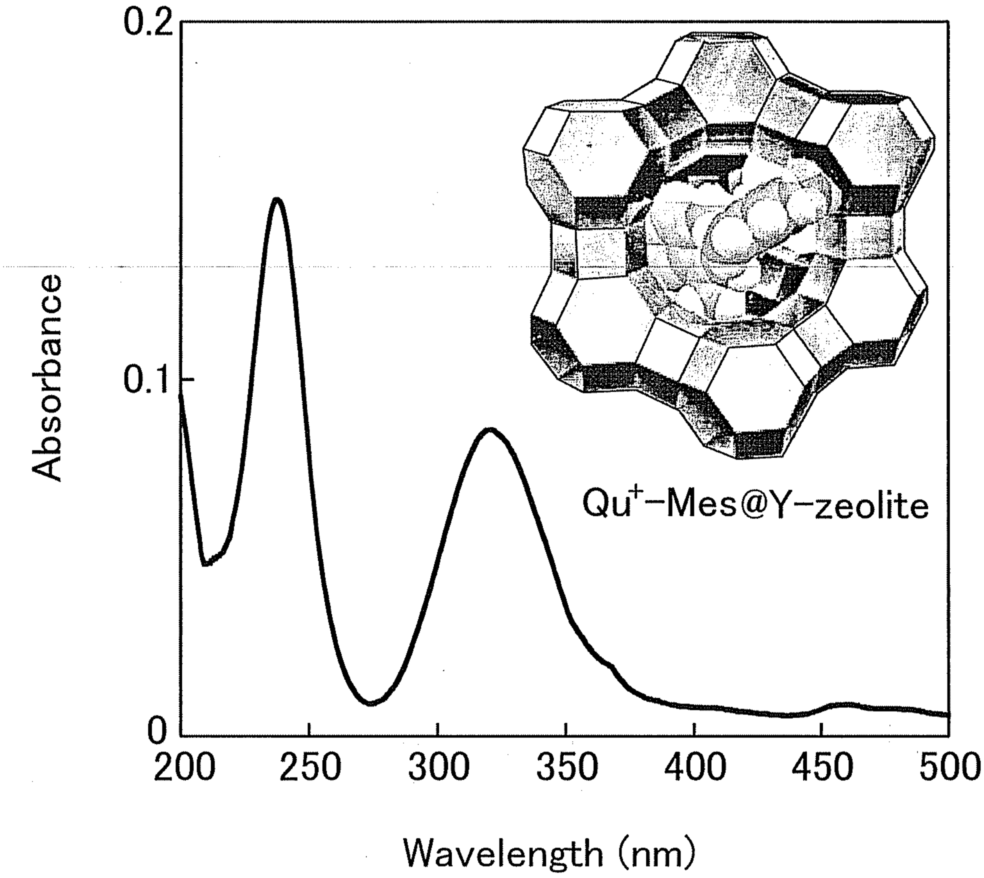
0.2
0.1
0
Absorbance
200
250
300
350
400
450
500
Wavelength (nm)
Qu+-Mes@Y-zeolite

FIG. 20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2005145853 A

### Non-patent literature cited in the description


- Kagaku Daijiten (chemical encyclopedia). Kyoritsu Shuppan Co., Ltd, 30 December 1960, vol. 4, 656
- the 39th impression of the compact edition. 15 September 2006
- **MOLINARI et al.** *Journal of Molecular Catalysis A,* 2007, vol. 262, 156-163
- **OHKUBO, K et al.** *Bulletin of the Chemical Society of Japan,* 2009, vol. 82, 303-315
- **KRESGE, C. T. ; LEONOWICZ, M. E. ; ROTH, W. J. ; VARTULI, J. C. ; BECK, J. S.** *Nature,* 1992, vol. 359, 710-712
- **BECK, J. S. et al.** *J. Am. Chem. Soc.,* 1992, vol. 114, 10834
- **CORMA, A. ; KUMAR, D.** *Stud. Surf. Sci. Catal.,* 1998, vol. 117, 201
- **JANICKE, M. T. et al.** *J. Am. Chem. Soc.,* 1998, vol. 120, 6940-6951
- **TUEL, A. et al.** *Chem. Mater.,* 2004, vol. 16, 2969-2974
- **MARTIN, H. et al.** *J. Phys. Chem. B,* 2004, vol. 108, 11496-11505